(19) **Europäisches Patentamt / European Patent Office / Office européen des brevets**

(11) **EP 2 062 981 B1**

(12) **EUROPEAN PATENT SPECIFICATION**

(45) Date of publication and mention of the grant of the patent:
**25.09.2013  Bulletin 2013/39**

(51) Int Cl.:
***C12Q 1/68*** (2006.01)

(21) Application number: **09154300.9**

(22) Date of filing: **16.06.2006**

(54) **Gene expression profiling for identification and monitoring of multiple sclerosis**

Genexpressionsprofil zur Identifizierung und Verlaufskontrolle von multipler Sklerose

Profilage d'expression génétique pour l'identification et surveillance de la sclérose en plaques

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.06.2005  US 155930**
**07.11.2005  US 734681 P**

(43) Date of publication of application:
**27.05.2009  Bulletin 2009/22**

(62) Document number(s) of the earlier application(s) in accordance with Art. 76 EPC:
**06784998.4 / 1 910 571**

(73) Proprietor: **Life Technologies Corporation**
**Carlsbad, CA 92008 (US)**

(72) Inventors:
• **Bevilacqua, Michael**
**Boulder, CO 80301 (US)**
• **Tryon, Victor**
**Woodinville, WA 98077 (US)**
• **Bankaitis-Davis, Danute**
**Longmont, CO 80503 (US)**
• **Siconolfi, Lisa**
**Westminster, CO 80234 (US)**
• **Trollinger, David B.**
**Boulder, CO 80301 (US)**
• **Wassmann, Karl**
**Dover, MA 02030 (US)**

(74) Representative: **Weber, Birgit**
**Life Technologies GmbH**
**Frankfurter Strasse 129 B**
**64293 Darmstadt (DE)**

(56) References cited:
WO-A-97/30150    WO-A-02/079218
WO-A2-03/023056

• **BOMPREZZI R ET AL: "Gene expression profile in multiple sclerosis patients and healthy controls: identifying pathways relevant to disease" HUMAN MOLECULAR GENETICS, OXFORD UNIVERSITY PRESS, SURREY, GB, vol. 12, no. 17, 2003, pages 2191-2199, XP002349005 ISSN: 0964-6906**
• **MANDEL M ET AL: "Autoimmunity gene expression portrait: specific signature that intersects or differentiates between multiple sclerosis and systemic lupus erythematosus." CLINICAL AND EXPERIMENTAL IMMUNOLOGY. OCT 2004, vol. 138, no. 1, October 2004 (2004-10), pages 164-170, XP002405223 ISSN: 0009-9104**
• **MYCKO MARCIN P ET AL: "cDNA microarray analysis in multiple sclerosis lesions: detection of genes associated with disease activity." BRAIN : A JOURNAL OF NEUROLOGY. MAY 2003, vol. 126, no. Pt 5, May 2003 (2003-05), pages 1048-1057, XP002405222 ISSN: 0006-8950**
• **SATOH J-I ET AL: "Microarray analysis identifies an aberrant expression of apoptosis and DNA damage-regulatory genes in multiple sclerosis" NEUROBIOLOGY OF DISEASE, BLACKWELL SCIENCE, OXFORD, GB, vol. 18, no. 3, April 2005 (2005-04), pages 537-550, XP004779664 ISSN: 0969-9961**

**(Cont. next page)**

- RAMANATHAN M ET AL: "In vivo gene expression revealed by cDNA arrays: the pattern in relapsing-remitting multiple sclerosis patients compared with normal subjects" JOURNAL OF NEUROIMMUNOLOGY, ELSEVIER SCIENCE PUBLISHERS BV, XX, vol. 116, 1 January 2001 (2001-01-01), pages 213-219, XP002984384 ISSN: 0165-5728
- BENNETTS BRUCE H ET AL: "HLA-DMB gene and HLA-DRA promoter region polymorphisms in Australian multiple sclerosis patients" HUMAN IMMUNOLOGY, vol. 60, no. 9, September 1999 (1999-09), pages 886-893, XP002523934 ISSN: 0198-8859
- LOCK CHRISTOPHER B ET AL: "Gene microarray analysis of multiple sclerosis lesions." TRENDS IN MOLECULAR MEDICINE. DEC 2003, vol. 9, no. 12, December 2003 (2003-12), pages 535-541, XP002405224 ISSN: 1471-4914
- RANSOHOFF ET AL: "Regulation of class II major histocompatibility complex genes: Relation to multiple sclerosis" RESEARCH IN IMMUNOLOGY, EDITIONS SCIENTIFIQUES ET MEDICALES ELSEVIER, vol. 140, no. 2, 1 January 1989 (1989-01-01), pages 202-207, XP023924285 ISSN: 0923-2494 [retrieved on 1989-01-01]
- STEWART G J ET AL: "HLA family studies and multiple sclerosis: A common gene, dominantly expressed" HUMAN IMMUNOLOGY, NEW YORK, NY, US, vol. 3, no. 1, 1 August 1981 (1981-08-01), pages 13-29, XP023715057 ISSN: 0198-8859 [retrieved on 1981-08-01]

## Description

### Field of the Invention

The present invention relates generally to the identification of biological markers associated with the identification of multiple sclerosis. More specifically, the invention relates to the use of gene expression data in the identification, monitoring and treatment of multiple sclerosis and in the characterization and evaluation of inflammatory conditions induced or related to multiple sclerosis.

### Background of the Invention

Multiple sclerosis (MS) is an autoimmune disease that affects the central nervous system (CNS). The CNS consists of the brain, spinal cord, and the optic nerves. Surrounding and protecting the nerve fibers of the CNS is a fatty tissue called myelin, which helps nerve fibers conduct electrical impulses. In MS, myelin is lost in multiple areas, leaving scar tissue called sclerosis. These damaged areas are also known as plaques or lesions. Sometimes the nerve fiber itself is damaged or broken. Myelin not only protects nerve fibers, but makes their job possible. When myelin or the nerve fiber is destroyed or damaged, the ability of the nerves to conduct electrical impulses to and from the brain is disrupted, and this produces the various symptoms of MS. People with MS can expect one of four clinical courses of disease, each of which might be mild, moderate, or severe. These include Relapsing-Remitting, Primary-Progressive, Secondary-Progressive, and Progressive-Relapsing

Individuals Progressive-Relapsing MS experience clearly defined flare-ups (also called relapses, attacks, or exacerbations). These are episodes of acute worsening of neurologic function. They are followed by partial or complete recovery periods (remissions) free of disease progression.

Individuals with Primary-Progressive MS experience a slow but nearly continuous worsening of their disease from the onset, with no distinct relapses or remissions. However, there are variations in rates of progression over time, occasional plateaus, and temporary minor improvements.

Individuals with Secondary- Progressive MS experience an initial period of relapsing- remitting disease, followed by a steadily worsening disease course with or without occasional flare- ups, minor recoveries (remissions), or plateaus.

Individuals with Progressive-Relapsing MS experience a steadily worsening disease from the onset but also have clear acute relapses (attacks or exacerbations), with or without recovery. In contrast to relapsing-remitting MS, the periods between relapses are characterized by continuing disease progression.

Information on any condition of a particular patient and a patient's response to types and dosages of therapeutic or nutritional agents has become an important issue in clinical medicine today not only from the aspect of efficiency of medical practice for the health care industry but for improved outcomes and benefits for the patients. Thus a need exists for better ways to diagnose and monitor the progression of multiple sclerosis.

Currently, the characterization of disease condition related to MS (including diagnosis, staging, monitoring disease progression, monitoring treatment effects on disease activity) is imprecise. Imaging that detects what appears to be plaques in CNS tissue is typically insufficient, by itself, to give a definitive diagnosis of MS. Often, diagnosis of MS is made only after both detection of plaques and of clinically evident neuropathy. It is clear that diagnosis of MS is usually made well after initiation of the disease process; i.e., only after detection of a sufficient number of plaques and of clinically evident neurological symptoms. Additionally, staging of MS is typically done by subjective measurements of exacerbation of symptoms, as well of other clinical manifestations. There are difficulties in diagnosis and staging because symptoms vary widely among individuals and change frequently within the individual. Thus, there is the need for tests which can aid in the diagnosis, monitor the progression and staging of MS.

WO03023056 relates to an in vitro method for the characterisation of multiple sclerosis and in a subject based on a sample from the subject that provides RNA. The method comprises the assessment of a profile data set of a plurality of members wherein each member is a quantitative measure of at least two constituents as distinct RNA constituents in the subject sample. The quantitative measure is obtained by amplification under measurement conditions that are substantially repeatable and the constituents are selected so that the measurement of the constituents distinguishes between a normal subject and an MS patient with an accuracy of at least 75%. However, the WO03023056 discloses different marker genes than those used in the current invention.

### Summary of the Invention

The invention is based in part upon the identification of gene expression profiles associated with multiple sclerosis (MS). Theses genes are referred to herein as MS-associated genes. More specifically, the invention is based upon the surprising discovery that detection of as few as two MS-associated genes is capable of identifying individuals with or without MS with at least 75% accuracy.

In various aspects the invention provides a method for determining a profile data set for characterizing a subject with multiple sclerosis based on a sample from the subject, the sample providing a source of RNAs, by using amplification for measuring the amount of RNA in a panel of constituents including HLADRA, and a second constituent selected from ITGAL, CASP9, NFKBIB, STAT3, NFKB1, ITGAM, BCL2, CD4, IL1B, HSPA1A, ICAM1, IL18R1, CD14, IFI16, PI3, TGFBR2, or CD8A.

Also provided by the invention is a method of characterizing multiple sclerosis in a subject, based on a sample from the subject, the sample providing a source of RNAs, by assessing a profile data set of a plurality of members, each member being a quantitative measure of the amount of a distinct RNA constituent in a panel of constituents selected as above.

The panel of constituents are selected so as to distinguish from a normal and a MS-diagnosed subject. The MS-diagnosed subject is washed out from therapy for three or more months. Preferably, the panel of constituents are selected so as to distinguish from a normal and a MS-diagnosed subject with at least 75%, 80%, 85%, 90%, 95%, 97%, 98%, 99% or greater accuracy. By "accuracy " is meant that the method has the ability to distinguish between subjects having multiple sclerosis or an inflammatory condition associated with multiple sclerosis and those that do not. Accuracy is determined for example by comparing the results of the Gene Expression Profiling to standard accepted clinical methods of diagnosing MS, e.g. MRI, sign and symptoms such as blurred vision, fatigue, loss or balance.

Alternatively, the panel of constituents is selected as to permit characterizing severity of MS in relation to normal over time so as to track movement toward normal as a result of successful therapy and away from normal in response to symptomatic flare.

The panel contains 10, 8, 5, 4, 3 or fewer constituents. Optimally, the panel of constituents includes ITGAM, HLADRA, CASP9, ITGAL or STAT3. Alternatively, the panel includes ITGAM and i) CD4 and NFKB1B, v). The panel includes two or more constituents from Table 6. Preferably, the panel includes any 2, 3, 4, or 5 genes in the combination shown in Tables 6. For example the panel contains i) HLADRA and one or more or the following: ITGAL, CASP9, NFKBIB, STAT2, NFKB1, ITGAM, ITGAL, CD4, IL1B, HSPA1A, ICAM1, IFI16, or TGFBR2.

Optionally, assessing may further include comparing the profile data set to a baseline profile data set for the panel. The baseline profile data set is related to the multiple sclerosis or an inflammatory condition related to multiple sclerosis to be characterized. The baseline profile data set is derived from one or more other samples from the same subject, taken when the subject is in a biological condition different from that in which the subject was at the time the first sample was taken, with respect to at least one of age, nutritional history, medical condition, clinical indicator, medication, physical activity, body mass, and environmental exposure, and the baseline profile data set may be derived from one or more other samples from one or more different subjects. In addition, the one or more different subjects may have in common with the subject at least one of age group, gender, ethnicity, geographic location, nutritional history, medical condition, clinical indicator, medication, physical activity, body mass, and environmental exposure. A clinical indicator may be used to assess multiple sclerosis or am inflammatory condition related to multiple sclerosis of the one or more different subjects, and may also include interpreting the calibrated profile data set in the context of at least one other clinical indicator, wherein the at least one other clinical indicator such as blood chemistry, urinalysis, X- ray or other radiological or metabolic imaging technique, other chemical assays, and physical findings.

The baseline profile data set may be derived from one or more other samples from the same subject taken under circumstances different from those of the first sample, and the circumstances may be selected from the group consisting of (i) the time at which the first sample is taken, (ii) the site from which the first sample is taken, (iii) the biological condition of the subject when the first sample is taken.

The subject has one or more presumptive signs of a multiple sclerosis. Presumptive signs of multiple sclerosis includes for example, altered sensory, motor, visual or proprioceptive system with at least one of numbness or weakness in one or more limbs, often occurring on one side of the body at a time or the lower half of the body, partial or complete loss of vision, frequently in one eye at a time and often with pain during eye movement, double vision or blurring of vision, tingling or pain in numb areas of the body, electric-shock sensations that occur with certain head movements, tremor, lack of coordination or unsteady gait, fatigue, dizziness, muscle stiffness or spasticity, slurred speech, paralysis, problems with bladder, bowel or sexual function, and mental changes such as forgetfulness or difficulties with concentration, relative to medical standards.

By multiple sclerosis or an inflammatory condition related to multiple sclerosis is meant that the condition is an autoimmune condition, an environmental condition, a viral infection, a bacterial infection, a eukaryotic parasitic infection, or a fungal infection.

The sample is any sample derived from a subject which contains RNA. For example the sample is blood, a blood fraction, body fluid, and a population of cells or tissue from the subj ect.

Optionally one or more other samples can be taken over an interval of time that is at least one month between the first sample and the one or more other samples, or taken over an interval of time that is at least twelve months between the first sample and the one or more samples, or they may be taken pre-therapy intervention or post-therapy intervention. In such embodiments, the first sample may be derived from blood and the baseline profile data set may be derived from tissue or body fluid of the subject other than blood. Alternatively, the first sample is derived from tissue or body fluid of the subject and the baseline profile data set is derived from blood.

All of the forgoing embodiments are carried out wherein the measurement conditions are substantially repeatable, particularly within a degree of repeatability of better than five percent or more particularly within a degree of repeatability of better than three percent, and/or wherein efficiencies of amplification for all constituents are substantially similar, more particularly wherein the efficiency of amplification is within two percent, and still more particularly wherein the efficiency of amplification for all constituents is less than one percent.

Additionally the invention includes storing the profile data set in a digital storage medium. Optionally, storing the profile data set includes storing it as a record in a database.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description and claims.

## Brief Description of the Drawings

The foregoing features of the invention will be more readily understood by reference to the following detailed description, taken with reference to the accompanying drawings, in which:

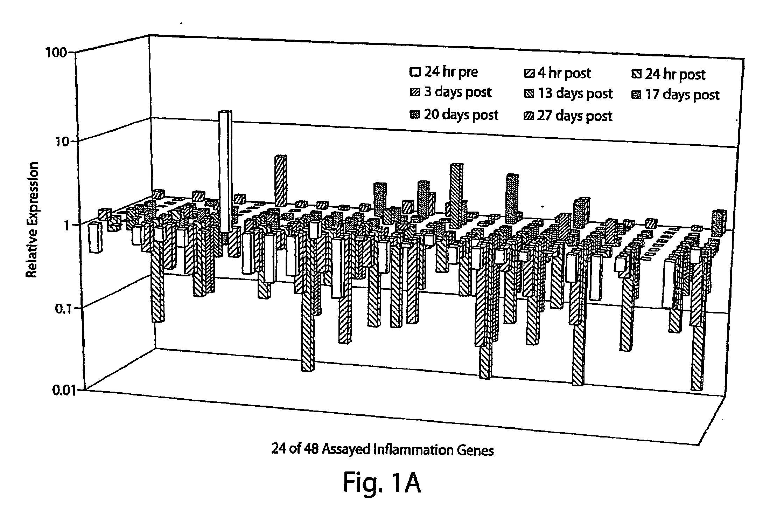

Fig. 1A shows the results of assaying 24 genes from the Source Inflammation Gene Panel (shown in Table 1 of US patent 6,692,916, such Panel is hereafter referred to as the Inflammation Gene Expression Panel) on eight separate days during the course of optic neuritis in a single male subject.

1B illustrates use of an inflammation index in relation to the data of Fig. 1A, in accordance with an embodiment of the present invention.

Fig. 2 is a graphical illustration of the same inflammation index calculated at 9 different, significant clinical milestones.

Fig. 3 shows the effects of single dose treatment with 800 mg of ibuprofen in a single donor as characterized by the index.

Fig. 4 shows the calculated acute inflammation index displayed graphically for five different conditions.

Fig. 5 shows a Viral Response Index for monitoring the progress of an upper respiratory infection (URI).

Figs. 6 and 7 compare two different populations using Gene Expression Profiles (with respect to the 48 loci of the Inflammation Gene Expression Panel).

Fig. 8 compares a normal population with a rheumatoid arthritis population derived from a longitudinal study.

Fig. 9 compares two normal populations, one longitudinal and the other cross sectional.

Fig. 10 shows the shows gene expression values for various individuals of a normal population.

Fig. 11 shows the expression levels for each of four genes (of the Inflammation Gene Expression Panel), of a single subject, assayed monthly over a period of eight months.

Figs. 12 and 13 show the expression levels for each of 48 genes (of the Inflammation Gene Expression Panel), of distinct single subjects (selected in each case on the basis of feeling well and not taking drugs), assayed weekly over a period of four weeks.

Fig. 13 show the expression levels for each of 48 genes (of the Inflammation Gene Expression Panel), of distinct single subjects (selected in each case on the basis of feeling well and not taking drugs), assayed monthly over a period of six months.

Fig. 14 shows the effect over time, on inflammatory gene expression in a single human subject, of the administration of an anti-inflammatory steroid, as assayed using the Inflammation Gene Expression Panel.

Fig. 15, shows the effect over time, via whole blood samples obtained from a human subject, administered a single dose of prednisone, on expression of 5 genes (of the Inflammation Gene Expression Panel).

Fig. 16 shows the effect over time, on inflammatory gene expression in a single human subject suffering from rheumatoid arthritis, of the administration of a TNF-inhibiting compound, but here the expression is shown in comparison to the cognate locus average previously determined (in connection with Figs. 6 and 7) for the normal (i.e., undiagnosed, healthy) population.

Fig. 17A illustrates the consistency of inflammatory gene expression in a population.

Fig. 17B shows the normal distribution of index values obtained from an undiagnosed population.

Fig. 17C illustrates the use of the same index as Fig. 17B, where the inflammation median for a normal population has been set to zero and both normal and diseased subjects are plotted in standard deviation units relative to that median.

Fig. 18 plots, in a fashion similar to that of Fig. 17A, Gene Expression Profiles, for the same 7 loci as in Fig. 17A, two different (responder v. non-responder) 6-subject populations of rheumatoid arthritis patients.

Fig. 19 illustrates use of the inflammation index for assessment of a single subject suffering from rheumatoid arthritis, who has not responded well to traditional therapy with methotrexate.

Fig. 20 illustrates use of the inflammation index for assessment of three subjects suffering from rheumatoid arthritis, who have not responded well to traditional therapy with methotrexate.

Fig. 21 shows the inflammation index for an international group of subjects, suffering from rheumatoid arthritis, undergoing three separate treatment regimens

Fig. 22 shows the inflammation index for an international group of subjects, suffering from rheumatoid arthritis, undergoing three separate treatment regimens

Fig. 23 shows the inflammation index for an international group of subjects, suffering from rheumatoid arthritis, undergoing three separate treatment regimens.

Fig. 24 illustrates use of the inflammation index for assessment of a single subject suffering from inflammatory bowel disease.

Fig. 25 shows Gene Expression Profiles with respect to 24 loci (of the Inflammation Gene Expression Panel of) for whole blood treated with Ibuprofen in vitro in relation to other non-steroidal anti-inflammatory drugs (NSAIDs).

Fig. 26 illustrates how the effects of two competing anti-inflammatory compounds can be compared objectively, quantitatively, precisely, and reproducibly.

Fig. 27 uses a novel bacterial Gene Expression Panel of 24 genes, developed to discriminate various bacterial conditions in a host biological system.

Fig. 28 shows differential expression for a single locus, IFNG, to LTA derived from three distinct sources: S. pyrogenes, B. subtilis, and S. aureus.

Figs. 29 show the response after two hours of the Inflammation 48A and 48B loci respectively (discussed above in connection with Figs. 6 and 7 respectively) in whole blood to administration of a Gram-positive and a Gram-negative organism.

Fig. 30 show the response after two hours of the Inflammation 48A and 48B loci respectively (discussed above in connection with Figs. 6 and 7 respectively) in whole blood to administration of a Gram-positive and a Gram-negative organism.

Figs. 31 show the response after six hours of the Inflammation 48A and 48B loci respectively (discussed above in connection with Figs. 6 and 7 respectively) in whole blood to administration of a Gram-positive and a Gram-negative organism.

Figs. 32 show the response after six hours of the Inflammation 48A and 48B loci respectively (discussed above in connection with Figs. 6 and 7 respectively) in whole blood to administration of a Gram-positive and a Gram-negative organism.

Fig. 33 compares the gene expression response induced by E. coli and by an organism-free E. coli filtrate.

Fig. 34 is similar to Fig. 33, but compared responses are to stimuli from E. coli filtrate alone and from E. coli filtrate to which has been added polymyxin B.

Fig. 35 illustrates the gene expression responses induced by *S. aureus* at 2, 6, and 24 hours after administration.

Figs. 36 illustrate the comparison of the gene expression induced by *E. coli* and *S. aureus* under various concentrations and times.

Figs. 37 illustrate the comparison of the gene expression induced by *E. coli* and *S. aureus* under various concentrations and times.

Figs. 38 illustrate the comparison of the gene expression induced by *E. coli* and *S. aureus* under various concentrations and times.

Figs. 39 illustrate the comparison of the gene expression induced by *E. coli* and *S. aureus* under various concentrations and times.

Figs. 40 illustrate the comparison of the gene expression induced by *E. coli* and *S. aureus* under various concentrations and times.

Figs. 41 illustrate the comparison of the gene expression induced by *E. coli* and *S. aureus* under various concentrations and times.

Fig. 42 illustrates application of a statistical T-test to identify potential members of a signature gene expression panel that is capable of distinguishing between normal subjects and subjects suffering from unstable rheumatoid arthritis.

Fig. 43 illustrates, for a panel of 17 genes, the expression levels for 8 patients presumed to have bacteremia.

Fig. 44 illustrates application of a statistical T-test to identify potential members of a signature gene expression panel that is capable of distinguishing between normal subjects and subjects suffering from bacteremia

Fig. 45 illustrates application of an algorithm (shown in the figure), providing an index pertinent to rheumatoid arthritis (RA) as applied respectively to normal subjects, RA patients, and bacteremia patients.

Fig. 46 illustrates application of an algorithm (shown in the figure), providing an index pertinent to bacteremia as applied respectively to normal subjects, rheumatoid arthritis patients, and bacteremia patients.

Fig. 47 illustrates, for a panel of 47 genes selected genes from Table 1, the expression levels for a patient suffering from multiple sclerosis on dates May 22, 2002 (no treatment), May 28, 2002 (after 5 mg prednisone given on May 22), and July 15, 2002 (after 100 mg prednisone given on May 28, tapering to 5 mg within one week).

Fig. 48 shows a scatter plot of a three-gene model useful for discriminating MS subjects generated by Latent Class Modeling analysis using ITGAM with MMP9 and ITGA4.

Fig. 49 shows a scatter plot of an alternative three-gene model useful for discriminating MS subjects using ITGAM with CD4 and MMP9.

Fig. 50 shows a scatter plot of the same alternative three-gene model of Figure 49 useful for discriminating MS subjects using ITGAM with MMP9 and CD4 but now displaying only washed out subjects relative to normals.

Fig. 51 shows a scatter plot of a four-gene model useful for discriminating MS subjects using ITGAM with ITGA4, MMP9 and CALCA.

Fig. 52 shows a scatter plot of a five-gene model useful for discriminating MS subjects using ITGAM with ITGA4, NFKB1B, MMP9 and CALCA.

Fig. 53 shows another five-gene model useful for discriminating MS subjects using ITGAM with ITGA4, NFKB1B, MMP9 and CXCR3 replacing CALCA.

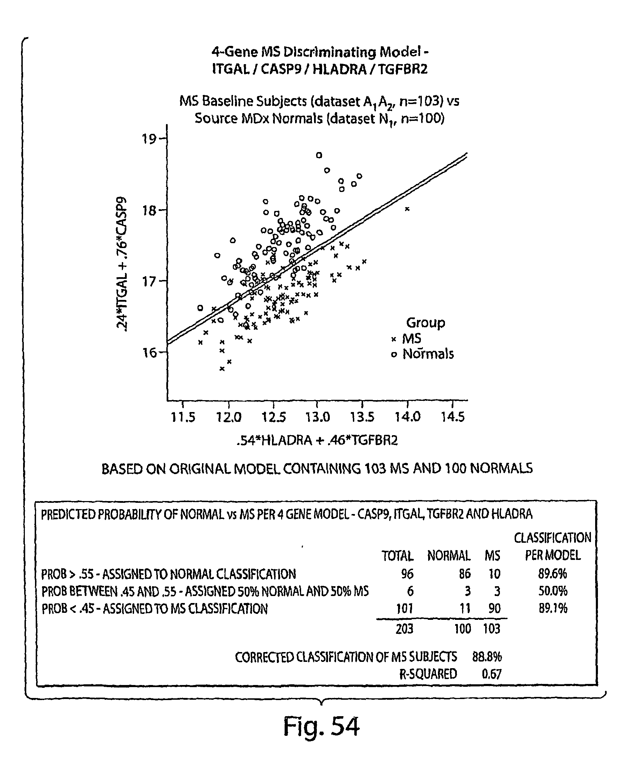

Fig 54 show a shows a four-gene model useful for discriminating MS subjects using ITGAL, CASP9, HLADRA and TGFBR2.

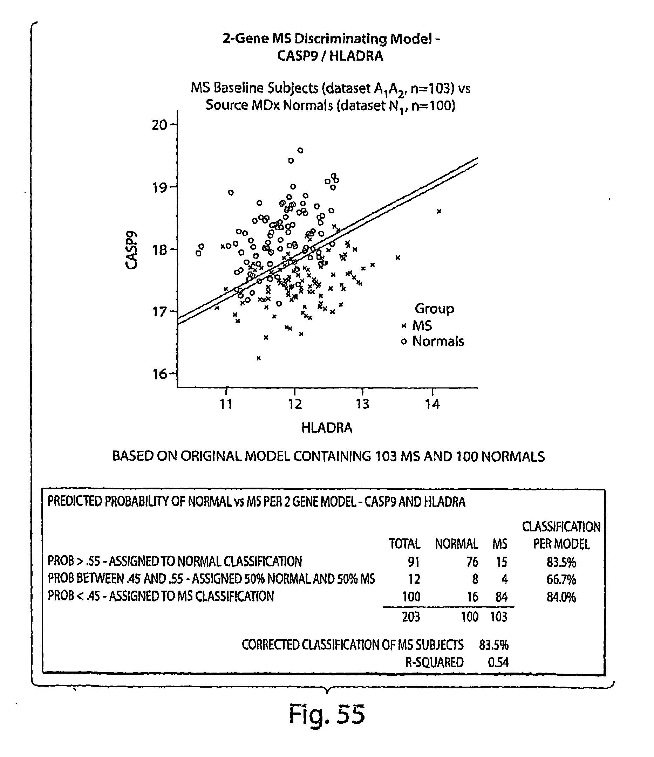

Fig 55 show a shows a two-gene model useful for discriminating MS subjects using CASP9 and HLADRA.

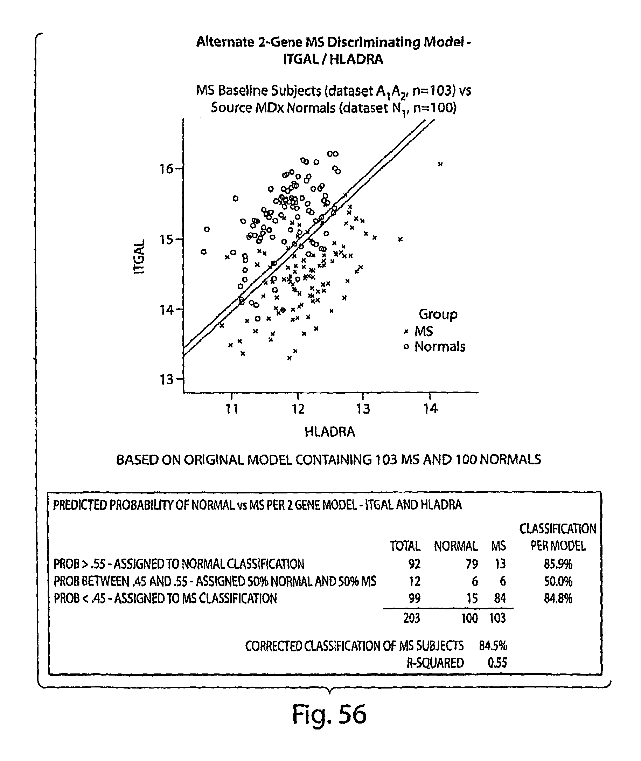

Fig 56 show a shows a two-gene model useful for discriminating MS subjects using ITGAL and HLADRA.

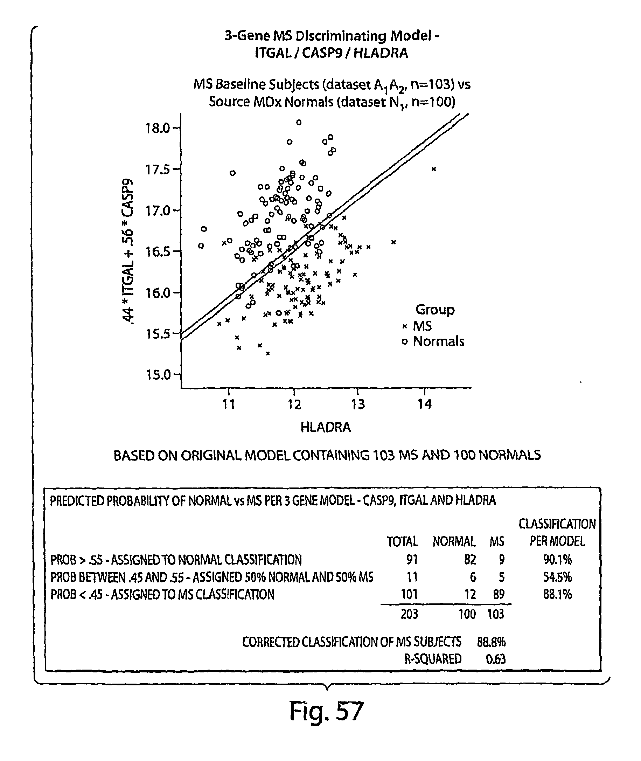

Fig 57 show a shows a three-gene model useful for discriminating MS subjects using ITGAL, CASP9, and HLADRA.

## Detailed Description of Specific Embodiments

*Definitions*

The following terms shall have the meanings indicated unless the context otherwise requires:

"*Algorithm*" is a set of rules for describing a biological condition. The rule set may be defined exclusively algebraically but may also include alternative or multiple decision points requiring domain-specific knowledge, expert interpretation or other clinical indicators.

An "*agent*" is a "*composition*" or a "*stimulus*", as those terms are defined herein, or a combination of a *composition* and a *stimulus*.

"*Amplification*" in the context of a quantitative RT-PCR assay is a function of the number of DNA replications that are tracked to provide a quantitative determination of its concentration. "*Amplification*" here refers to a degree of sensitivity and specificity of a quantitative assay teclmique. Accordingly, amplification provides a measurement of concentrations of constituents that is evaluated under conditions wherein the efficiency of amplification and therefore the degree of sensitivity and reproducibility for measuring all constituents is substantially similar.

"*Accuracy*" is measure of the strength of the relationship between true values and their predictions. Accordingly, accuracy provided a measurement on how close to a true or accepted value a measurement lies

A "*baseline profile data set*" is a set of values associated with constituents of a *Gene Expression Panel* resulting from evaluation of a biological sample (or population or set of samples) under a desired *biological condition* that is used for mathematically normative purposes. The desired biological condition may be, for example, the condition of a subject (or population or set of subjects) before exposure to an agent or in the presence of an untreated disease or in the absence of a disease. Alternatively, or in addition, the desired biological condition may be health of a subj ect or a population or set of subjects. Alternatively, or in addition, the desired biological condition may be that associated with a population or set of subjects selected on the basis of at least one of age group, gender, ethnicity, geographic location, nutritional history, medical condition, clinical indicator, medication, physical activity, body mass, and environmental exposure.

A "set" or "population" of samples or subjects refers to a defined or selected group of samples or subjects wherein there is an underlying commonality or relationship between the members included in the set or population of samples or subjects.

A "population of cells" refers to any group of cells wherein there is an underlying commonality or relationship between the members in the population of cells, including a group of cells taken from an organism or from a culture of cells or from a biopsy, for example,

A "*biological condition*" of a subject is the condition of the subject in a pertinent realm that is under observation, and such realm may include any aspect of the subject capable of being monitored for change in condition, such as health, disease including cancer; autoimmune condition; trauma; aging; infection; tissue degeneration; developmental steps; physical fitness; obesity, and mood. As can be seen, a condition in this context may be chronic or acute or simply transient. Moreover, a targeted biological condition may be manifest throughout the organism or population of cells or may be restricted to a specific organ (such as skin, heart, eye or blood), but in either case, the condition may be monitored directly by a sample of the affected population of cells or indirectly by a sample derived elsewhere from the subject. The term *"biological condition"* includes a *"physiological condition"*.

*"Body fluid"* of a subject includes blood, urine, spinal fluid, lymph, mucosal secretions, prostatic fluid, semen, haemolymph or any other body fluid known in the art for a subj ect.

*"Calibrated profile data set"* is a function of a member of a first *profile data set* and a corresponding member of a *baseline profile data set* for a given constituent in a panel.

A *"clinical indicator"* is any physiological datum used alone or in conjunction with other data in evaluating the *physiological condition* of a collection of cells or of an organism. This term includes pre-clinical indicators.

A *"composition"* includes a chemical compound, a nutraceutical, a phamaceutical, a homeopathic formulation, an allopathic formulation, a naturopathic formulation, a combination of compounds, a toxin, a food, a food supplement, a mineral, and a complex mixture of substances, in any physical state or in a combination of physical states.

To *"derive"* a profile data set from a *sample* includes determining a set of values associated with constituents of a *Gene Expression Panel* either (i) by direct measurement of such constituents in a biological *sample* or (ii) by measurement of such constituents in a second biological *sample* that has been exposed to the original sample or to matter derived from the original sample.

*"Distinct RNA or protein constituent"* in a panel of constituents is a distinct expressed product of a gene, whether RNA or protein. An *"expression"* product of a gene includes the gene product whether RNA or protein resulting from translation of the messenger RNA.

A *"Gene Expression Panel"* is an experimentally verified set of constituents, each constituent being a distinct expressed product of a gene, whether RNA or protein, wherein constituents of the set are selected so that their measurement provides a measurement of a targeted *biological condition*.

A *"Gene Expression Profile"* is a set of values associated with constituents of a *Gene Expression Panel* resulting from evaluation of a biological sample (or population or set of samples).

A *"Gene Expression Profile Inflammatory Index"* is the value of an index function that provides a mapping from an instance of a *Gene Expression Profile* into a single-valued measure of inflammatory condition.

The *"health"* of a subject includes mental, emotional, physical, spiritual, allopathic, naturopathic and homeopathic condition of the subject.

*"Index"* is an arithmetically or mathematically derived numerical characteristic developed for aid in simplifying or disclosing or informing the analysis of more complex quantitative information. A disease or population index may be determined by the application of a specific algorithm to a plurality of subjects or samples with a common biological condition.

*"Inflammation"* is used herein in the general medical sense of the word and may be an acute or chronic; simple or suppurative; localized or disseminated; cellular and tissue response, initiated or sustained by any number of chemical, physical or biological agents or combination of agents.

*"Inflammatory state"* is used to indicate the relative biological condition of a subject resulting from inflammation, or characterizing the degree of inflammation

A *"large number"* of data sets based on a common panel of genes is a number of data sets sufficiently large to permit a statistically significant conclusion to be drawn with respect to an instance of a data set based on the same panel.

*"Multiple sclerosis"* (MS) is a debilitating wasting disease. The disease is associated with degeneration of the myelin sheaths surrounding nerve cells which leads to a loss of motor and sensory function.

A *"normative"* condition of a subject to whom a composition is to be administered means the condition of a subject before administration, even if the subject happens to be suffering from a disease.

A *"panel"* of genes is a set of genes including at least two constituents.

A *"sample"* from a subject may include a single cell or multiple cells or fragments of cells or an aliquot of body fluid, taken from the subject, by means including venipuncture, excretion, ejaculation, massage, biopsy, needle aspirate, lavage sample, scraping, surgical incision or intervention or other means known in the art.

A *"Signature Profile"* is an experimentally verified subset of a *Gene Expression Profile* selected to discriminate a biological condition, agent or physiological mechanism of action.

A *"Signature Panel"* is a subset of a *Gene Expression Panel,* the constituents of which are selected to permit discrimination of a *biological condition*, *agent* or physiological mechanism of action.

A *"subject"* is a cell, tissue, or organism, human or non-human, whether in vivo, ex vivo or in vitro, under observation. When we refer to evaluating the biological condition of a subject based on a sample from the subject, we include using blood or other tissue sample from a human subject to evaluate the human subject's condition; but we also include, for example, using a blood sample itself as the subject to evaluate, for example, the effect of therapy or an agent upon the sample.

A *"stimulus"* includes (i) a monitored physical interaction with a subject, for example ultraviolet A or B, or light therapy for seasonal affective disorder, or treatment of psoriasis with psoralen or treatment of melanoma with embedded radioactive seeds, other radiation exposure, and (ii) any monitored physical, mental, emotional, or spiritual activity or inactivity of a subject.

"*Therapy*" includes all interventions whether biological, chemical, physical, metaphysical, or combination of the foregoing, intended to sustain or alter the monitored biological condition of a subject.

The PCT patent application publication number WO 01/25473, published April 12, 2001, entitled "Systems and Methods for Characterizing a Biological Condition or Agent Using Calibrated Gene Expression Profiles," filed for an invention by inventors herein, and which is herein incorporated by reference, discloses the use of Gene Expression Panels for the evaluation of (i) biological condition (including with respect to health and disease) and (ii) the effect of one or more agents on biological condition (including with respect to health, toxicity, therapeutic treatment and drug interaction).

In particular, Gene Expression Panels may be used for measurement of therapeutic efficacy of natural or synthetic compositions or stimuli that may be formulated individually  or in combinations or mixtures for a range of targeted biological conditions; prediction of toxicological effects and dose effectiveness of a composition or mixture of compositions for an individual or for a population or set of individuals or for a population of cells; determination of how two or more different agents administered in a single treatment might interact so as to detect any of synergistic, additive, negative, neutral or toxic activity; performing pre-clincal and clinical trials by providing new criteria for pre-selecting subjects according to informative profile data sets for revealing disease status; and conducting preliminary dosage studies for these patients prior to conducting phase 1 or 2 trials. These Gene Expression Panels may be employed with respect to samples derived from subjects in order to evaluate their biological condition.

The present invention provides Gene Expression Panels for the evaluation of multiple sclerosis and inflammatory condition related to multiple sclerosis. In addition, the Gene Expression Profiles described herein also provided the evaluation of the affect of one or more agents for the treatment of multiple sclerosis and inflammatory condition related to multiple sclerosis.

A Gene Expression Panel is selected in a manner so that quantitative measurement of RNA or protein constituents in the Panel constitutes a measurement of a biological condition of a subject. In one kind of arrangement, a calibrated profile data set is employed. Each member of the calibrated profile data set is a function of (i) a measure of a distinct constituent of a Gene Expression Panel and (ii) a baseline quantity.

It has been discovered that valuable and unexpected results are achieved when the quantitative measurement of constituents is performed under repeatable conditions (within a degree of repeatability of measurement of better than twenty percent, and preferably five percent or better, and more preferably three percent or better). For the purposes of this description and the following claims, a degree of repeatability of measurement of better than twenty percent as providing measurement conditions that are "substantially repeatable". In particular, it is desirable that, each time a measurement is obtained corresponding to the level of expression of a constituent in a particular sample, substantially the same measurement should result for the substantially the same level of expression. In this manner, expression levels for a constituent in a Gene Expression Panel may be meaningfully compared from sample to sample. Even if the expression level measurements for a particular constituent are  inaccurate (for example, say, 30% too low), the criterion of repeatability means that all measurements for this constituent, if skewed, will nevertheless be skewed systematically, and therefore measurements of expression level of the constituent may be compared meaningfully. In this fashion valuable information may be obtained and compared concerning expression of the constituent under varied circumstances.

In addition to the criterion of repeatability, it is desirable that a second criterion also be satisfied, namely that quantitative measurement of constituents is performed under conditions wherein efficiencies of amplification for all constituents are substantially similar (within one to two percent and typically one percent or less). When both of these criteria are satisfied, then measurement of the expression level of one constituent may be meaningfully compared with measurement of the expression level of another constituent in a given sample and from sample to sample..

Present embodiments relate to the use of an index or algorithm resulting from quantitative measurement of constituents, and optionally in addition, derived from either expert analysis or computational biology (a) in the analysis of complex data sets; (b) to control or normalize the influence of uninformative or otherwise minor variances in gene expression values between samples or subjects; (c) to simplify the characterization of a complex data set for comparison to other complex data sets, databases or indices or algorithms derived from complex data sets; (d) to monitor a biological condition of a subject; (e) for measurement of therapeutic efficacy of natural or synthetic compositions or stimuli that may be formulated individually or in combinations or mixtures for a range of targeted biological conditions; (f) for predictions of toxicological effects and dose effectiveness of a composition or mixture of compositions for an individual or for a population or set of individuals or for a population of cells; (g) for determination of how two or more different agents administered in a single treatment might interact so as to detect any of synergistic, additive, negative, neutral of toxic activity (h) for performing pre-clinical and clinical trials by providing new criteria for pre-selecting subjects according to informative profile data sets for revealing disease status and conducting preliminary dosage studies for these patients prior to conducting phase 1 or 2 trials.

Gene expression profiling and the use of index characterization for a particular condition or agent or both may be used to reduce the cost of phase 3 clinical trials and may be used beyond phase 3 trials; labeling for approved drugs; selection of suitable medication in a class of medications for a particular patient that is directed to their unique physiology; diagnosing or determining a prognosis of a medical condition or an infection which may precede onset of symptoms or alternatively diagnosing adverse side effects associated with administration of a therapeutic agent; managing the health care of a patient; and quality control for different batches of an agent or a mixture of agents.

The subject

The methods disclosed here may be applied to cells of humans, mammals or other organisms without the need for undue experimentation by one of ordinary skill in the art because all cells transcribe RNA and it is known in the art how to extract RNA from all types of cells.

A subject can include those who have not been previously diagnosed as having multiple sclerosis or an inflammatory condition related to multiple sclerosis. Alternatively, a subject can also include those who have already been diagnosed as having multiple sclerosis or an inflammatory condition related to multiple sclerosis. Optionally, the subject has been previously treated with therapeutic agents, or with other therapies and treatment regimens for o multiple sclerosis or an inflammatory condition related to multiple sclerosis. A subject can also include those who are suffering from, or at risk of developing multiple sclerosis or an inflammatory condition related to multiple sclerosis, such as those who exhibit known risk factors for multiple sclerosis or an inflammatory condition related to multiple sclerosis.

Selecting constituents of a Gene Expression Panel

The general approach to selecting constituents of a Gene Expression Panel has been described in PCT application publication number WO 01/ 25473. A wide range of Gene Expression Panels have been designed and experimentally verified, each panel providing a quantitative measure, of biological condition, that is derived from a sample of blood or other tissue. For each panel, experiments have verified that a Gene Expression Profile using the panel's constituents is informative of a biological condition. (It has also been demonstrated hat in being informative of biological condition, the Gene Expression Profile can be used to used, among other things, to measure the effectiveness of therapy, as well as to provide a target for therapeutic intervention.) Tables 1, 2 ,3,4, 5, 6, 7, 8, or 9 listed below, include relevant genes which may be selected for a given Gene Expression Panel, such as the Gene Expression Panels demonstrated herein to be useful in the evaluation of multiple sclerosis and inflammatory condition related to multiple sclerosis.

In general, panels may be constructed and experimentally verified by one of ordinary skill in the art in accordance with the principles articulated in the present application.

Design of assays

Typically, a sample is run through a panel in quadruplicate or triplicate; that is, a sample is divided into aliquots and for each aliquot we measure concentrations of each constituent in a Gene Expression Panel. Over a total of 900 constituent assays, with each assay conducted in quadruplicate, we found an average coefficient of variation, (standard deviation/ average) * 100, of less than 2 percent, typically less than 1 percent, among results for each assay. This figure is a measure called "intra- assay variability". Assays have also been conducted on different occasions using the same sample material. With 72 assays, resulting from concentration measurements of constituents in a panel of 24 members, and such concentration measurements determined on three different occasions over time, we found an average coefficient of variation of less than 5 percent, typically less than 2 percent. This as a measure of 1 "inter- assay variability".

It has been determined that it is valuable to use the duplicate or triplicate test results to identify and eliminate data points that are statistical "outliers"; such data points are those that differ by a percentage greater, for example, than 3% of the average of all four values and that do not result from any systematic skew that is greater, for example, than 1%. Moreover, if more than one data point in a set of four is excluded by this procedure, then all data for the relevant constituent is discarded.

Measurement of Gene Expression for a constituent in the Panel

For measuring the amount of a particular RNA in a sample, methods known to one of ordinary skill in the art to extract and quantify transcribed RNA from a sample with respect to a constituent of a Gene Expression Panel have been used (See detailed protocols below. Also see PCT application publication number WO 98/24935 for RNA analysis protocols). Briefly, RNA is extracted from a sample such as a tissue, body fluid, or culture medium in which a population of cells of a subject might be growing. For example, cells may be lysed and RNA eluted in a suitable solution in which to conduct a DNAse reaction. First strand synthesis may be performed using a reverse transcriptase. Gene amplification, more specifically quantitative PCR assays, can then conducted and the gene of interest size calibrated against a marker such as 18S rRNA (Hirayama et al., Blood 92, 1998: 46-52). Samples are measured in multiple duplicates, for example, 4 replicates. Relative quantitation of the mRNA is determined by the difference in threshold cycles between the internal control and the gene of interest. In an embodiment of the invention, quantitative PCR is performed using amplification, reporting agents and instruments such as those supplied commercially by Applied Biosystems (Foster City, CA). Given a defined efficiency of amplification of target transcripts, the point (e.g., cycle number) that signal from amplified target template is detectable may be directly related to the amount of specific message transcript in the measured sample. Similarly, other quantifiable signals such as fluorescence, enzyme activity, disintegrations per minute, absorbance, etc., when correlated to a known concentration of target templates (e.g., a reference standard curve) or normalized to a standard with limited variability can be used to quantify the number of target templates in an unknown sample.

Although not limited to amplification methods, quantitative gene expression techniques may utilize amplification of the target transcript. Alternatively or in combination with amplification of the target transcript, amplification of the reporter signal may also be used. Amplification of the target template may be accomplished by isothermic gene amplification strategies, or by gene amplification by thermal cycling such as PCR.

It is desirable to obtain a definable and reproducible correlation between the amplified target or reporter and the concentration of starting templates. It has been discovered that this objective can be achieved by careful attention to, for example, consistent primer-template ratios and a strict adherence to a narrow permissible level of experimental amplification efficiencies (for example 99.0 to 100% relative efficiency, typically 99.8 to 100% relative efficiency). For example, in determining gene expression levels with regard to a single Gene Expression Profile, it is necessary that all constituents of the panels maintain a similar and limited range of primer template ratios (for example, within a 10-fold range) and amplification efficiencies (within, for example, less than 1%) to permit accurate and precise relative measurements for each constituent. Amplification efficiencies are regarded as being "substantially similar", for the purposes of this description and the following claims, if they differ by no more than approximately 10%. Preferably they should differ by less than approximately 2% and more preferably by less than approximately 1%. These constraints should be observed over the entire range of concentration levels to be measured associated with the relevant biological condition.

In practice, tests runs are performed to assure that these conditions are satisfied. For example, a number of primer-probe sets are designed and manufactured, and it is determined experimentally which set gives the best performance. Even though primer-probe design and manufacture can be enhanced using computer techniques known in the art, and notwithstanding common practice, we still find that experimental validation is useful. Moreover, in the course of experimental validation, the selected primer-probe combination is associated with a set of features:

The reverse primer should be complementary to the coding DNA strand. In one embodiment, the primer should be located across an intron-exon junction, with not more than three bases of the three-prime end of the reverse primer complementary to the proximal exon. (If more than three bases are complementary, then it would tend to competitively amplify genomic DNA.)

In an embodiment of the invention, the primer probe should amplify cDNA of less than 110 bases in length and should not amplify genomic DNA or transcripts or cDNA from related but biologically irrelevant loci.

A suitable target of the selected primer probe is first strand cDNA, which may be prepared, in one embodiment, is described as follows:

(a) Use of whole blood for *ex vivo* assessment of a biological condition affected by an agent.

Human blood is obtained by venipuncture and prepared for assay by separating samples for baseline, no stimulus, and stimulus with sufficient volume for at least three time points. Typical stimuli include lipopolysaccharide (LPS), phytohemagglutinin (PHA) and heat- killed staphylococci (HKS) or carrageenan and may be used individually (typically) or in combination. The aliquots of heparinized, whole blood are mixed without stimulus and held at 37°C in an atmosphere of 5% $CO_2$ for 30 minutes. Stimulus is added at varying concentrations, mixed and held loosely capped at 37°C for 30 min. Additional test compounds may be added at this point and held for varying times depending on the expected pharmacokinetics of the test compound. At defined times, cells are collected by centrifugation, the plasma removed and RNA extracted by various standard means. Nucleic acids, RNA and or DNA are purified from cells, tissues or fluids of the test population of cells or indicator cell lines. RNA is preferentially obtained from the nucleic acid mix using a variety of standard procedures (or RNA Isolation Strategies, pp. 55- 104, in RNA Methodologies, A laboratory guide for isolation and characterization, 2nd edition, 1998, Robert E. Farrell, Jr., Ed., Academic Press), in the present using a filter- based RNA isolation system from Ambion (RNAqueous ™, Phenol- free Total RNA Isolation Kit, Catalog #1912, version 9908; Austin, Texas) .

In accordance with one procedure, the whole blood assay for Gene Expression Profiles determination was carried out as follows: Human whole blood was drawn into 10 mL Vacutainer tubes with Sodium Heparin. Blood samples were mixed by gently inverting tubes 4-5 times. The blood was used within 10-15 minutes of draw. In the experiments, blood was diluted 2-fold, i.e. per sample per time point, 0.6 mL whole blood + 0.6 mL stimulus. The assay medium was prepared and the stimulus added as appropriate.

A quantity (0.6 mL) of whole blood was then added into each 12 x 75 mm polypropylene tube. 0.6 mL of 2X LPS (from *E. coli* serotype 0127:B8, Sigma#L3880 or serotype 055, Sigma #L4005, 10ng/mL, subject to change in different lots) into LPS tubes was added. Next, 0.6 mL assay medium was added to the "control" tubes with duplicate tubes for each condition. The caps were closed tightly. The tubes were inverted 2-3 times to mix samples. Caps were loosened to first stop and the tubes incubated @ 37°C, 5% $CO_2$ for 6 hours. At 6 hours, samples were gently mixed to resuspend blood cells, and 1 mL was removed from each tube (using a micropipettor with barrier tip), and transferred to a 2 mL "dolphin" microfuge tube (Costar #3213).

The samples were then centrifuged for 5 min at 500 x g, ambient temperature (IEC centrifuge or equivalent, in microfuge tube adapters in swinging bucket), and as much serum from each tube was removed as possible and discarded. Cell pellets were placed on ice; and RNA extracted as soon as possible using an Ambion RNAqueous kit.

(b) Amplification strategies.

Specific RNAs are amplified using message specific primers or random primers. The specific primers are synthesized from data obtained from public databases (e.g., Unigene, National Center for Biotechnology Information, National Library of Medicine, Bethesda, MD), including information from genomic and cDNA libraries obtained from humans and other animals. Primers are chosen to preferentially amplify from specific RNAs obtained from the test or indicator samples, see, for example, RT PCR, Chapter 15 in RNA Methodologies, A laboratory guide for isolation and characterization, 2nd edition, 1998, Robert E. Farrell, Jr., Ed., Academic Press; or Chapter 22 pp.143-151, RNA isolation and characterization protocols, Methods in molecular biology, Volume 86, 1998, R. Rapley and D. L. Manning Eds., Human Press, or 14 in Statistical refinement of primer design parameters, Chapter 5, pp. 55-72, PCR applications: protocols for functional genomics, M.A. Innis, D.H. Gelfand and J.J. Sninsky, Eds., 1999, Academic Press). Amplifications are carried out in either isothermic conditions or using a thermal cycler (for example, a ABI 9600 or 9700 or 7700 obtained from Applied Biosystems, Foster City, CA; see Nucleic acid detection methods, pp. 1-24, in Molecular methods for virus detection, D.L. Wiedbrauk and D.H., Farkas, Eds., 1995, Academic Press). Amplified nucleic acids are detected using fluorescent-tagged detection primers (see, for example, Taqman™ PCR Reagent Kit, Protocol, part number 402823 revision A, 1996, Applied Biosystems, Foster City CA.) that are identified and synthesized from publicly known databases as described for the amplification primers. In the present case, amplified DNA is detected and quantified using the ABI Prism 7700 Sequence Detection System obtained from Applied Biosystems (Foster City, CA). Amounts of specific RNAs contained in the test sample or obtained from the indicator cell lines can be related to the relative quantity of fluorescence observed (see for example, Advances in quantitative PCR technology: 5' nuclease assays, Y.S. Lie and C.J. Petropolus, Current Opinion in Biotechnology, 1998, 9:43-48, or Rapid thermal cycling and PCR kinetics, pp. 211-229, chapter 14 in PCR applications: protocols for functional genomics, M.A. Innis, D.H. Gelfand and J.J. Sninsky, Eds., 1999, Academic Press).

As a particular implementation of the approach described here, we describe in detail a procedure for synthesis of first strand cDNA for use in PCR. This procedure can be used for both whole blood RNA and RNA extracted from cultured cells (i.e. THP-1 cells).

Materials

1. Applied Biosystems TAQMAN Reverse Transcription Reagents Kit (P/N 808-0234). Kit Components: 10X TaqMan RT Buffer, 25 mM Magnesium chloride, deoxyNTPs mixture, Random Hexamers, RNase Inhibitor, MultiScribe Reverse Transcriptase (50 U/mL) (2) RNase / DNase free water (DEPC Treated Water from Ambion (P/N 9915G), or equivalent)

Methods

1. Place RNase Inhibitor and MultiScribe Reverse Transcriptase on ice immediately. All other reagents can be thawed at room temperature and then placed on ice.

2. Remove RNA samples from -80°C freezer and thaw at room temperature and then place immediately on ice.

3. Prepare the following cocktail of Reverse Transcriptase Reagents for each 100 μL RT reaction (for multiple samples, prepare extra cocktail to allow for pipetting error):

| | 1 reaction (mL) | 11X, e.g. 10 samples (μL) |
|---|---|---|
| 10XRT Buffer | 10.0 | 110.0 |
| 25 mM MgCl$_2$ | 22.0 | 242.0 |
| dNTPs | 20.0 | 220.0 |
| Random Hexamers | 5.0 | 55.0 |
| RNAse Inhibitor | 2.0 | 22.0 |
| Reverse Transcriptase | 2.5 | 27.5 |
| Water | 18.5 | 203.5 |
| Total: | 80.0 | 880.0 (80 μL per sample) |

4. Bring each RNA sample to a total volume of 20 μL in a 1.5 mL microcentrifuge tube (for example, for THP-1 RNA, remove 10 μL RNA and dilute to 20 μL with RNase / DNase free water, for whole blood RNA use 20 μL total RNA) and add 80 μL RT reaction mix from step 5,2,3. Mix by pipetting up and down.

5. Incubate sample at room temperature for 10 minutes.

6. Incubate sample at 37°C for 1 hour.

7. Incubate sample at 90°C for 10 minutes.

8. Quick spin samples in microcentrifuge.

9. Place sample on ice if doing PCR immediately, otherwise store sample at -20°C for future use.

10. PCR QC should be run on all RT samples using 18S and b-actin.

The use of the primer probe with the first strand cDNA as described above to permit measurement of constituents of a Gene Expression Panel is as follows:

Set up of a 24-gene Human Gene Expression Panel for Inflammation.

Materials

1. 20X Primer/Probe Mix for each gene of interest.

2. 20X Primer/Probe Mix for 18S endogenous control.

3. 2X Taqman Universal PCR Master Mix.

4. cDNA transcribed from RNA extracted from cells.

5. Applied Biosystems 96-Well Optical Reaction Plates.

6. Applied Biosystems Optical Caps, or optical-clear film.

7. Applied Biosystem Prism 7700 or 7900 Sequence Detector.

Methods

1. Make stocks of each Primer/Probe mix containing the Primer/Probe for the gene of interest, Primer/Probe for 18S endogenous control, and 2X PCR Master Mix as follows. Make sufficient excess to allow for pipetting error e.g. approximately 10% excess. The following example illustrates a typical set up for one gene with quadruplicate samples testing two conditions (2 plates).

| | 1X(1 well) | 9X (2 plates worth) |
|---|---|---|
| 2X Master Mix | 12.50 | 112.50 |
| 20X 18S Primer/Probe Mix | 1.25 | 11.25 |
| 20X Gene of interest Primer/Probe Mix | 1.25 | 11.25 |
| Total | 15.00 | 135.00 |

2. Make stocks of cDNA targets by diluting 95 μL of cDNA into 2000 μL of water. The amount of cDNA is adjusted to give Ct values between 10 and 18, typically between 12 and 13.

3. Pipette 15 μL of Primer/Probe mix into the appropriate wells of an Applied Biosystems 96-Well Optical Reaction Plate.

4. Pipette 10 μL of cDNA stock solution into each well of the Applied Biosystems 96-Well Optical Reaction Plate.

5. Seal the plate with Applied Biosystems Optical Caps, or optical-clear film.

6. Analyze the plate on the AB Prism 7700 or 7900 Sequence Detector.

Methods herein may also be applied using proteins where sensitive quantitative techniques, such as an Enzyme Linked ImmunoSorbent Assay (ELISA) or mass spectroscopy, are available and well-known in the art for measuring the amount of a protein constituent. (see WO 98/24935 herein incorporated by reference).

Baseline profile data sets

The analyses of samples from single individuals and from large groups of individuals provide a library of profile data sets relating to a particular panel or series of panels. These profile data sets may be stored as records in a library for use as baseline profile data sets. As the term "baseline" suggests, the stored baseline profile data sets serve as comparators for providing a calibrated profile data set that is informative about a biological condition or agent. Baseline profile data sets may be stored in libraries and classified in a number of cross-referential ways. One form of classification may rely on the characteristics of the panels from which the data sets are derived. Another form of classification may be by particular biological condition, e.g., multiple sclerosis. The concept of biological condition encompasses any state in which a cell or population of cells may be found at any one time. This state may reflect geography of samples, sex of subjects or any other discriminator. Some of the discriminators may overlap. The libraries may also be accessed for records associated with a single subject or particular clinical trial. The classification of baseline profile data sets may further be annotated with medical information about a particular subject, a medical condition, a particular agent etc.

The choice of a baseline profile data set for creating a calibrated profile data set is related to the biological condition to be evaluated, monitored, or predicted, as well as, the intended use of the calibrated panel, e.g., as to monitor drug development, quality control or other uses. It may be desirable to access baseline profile data sets from the same subject for whom a first profile data set is obtained or from different subject at varying times, exposures to stimuli, drugs or complex compounds; or may be derived from like or dissimilar populations or sets of subjects.

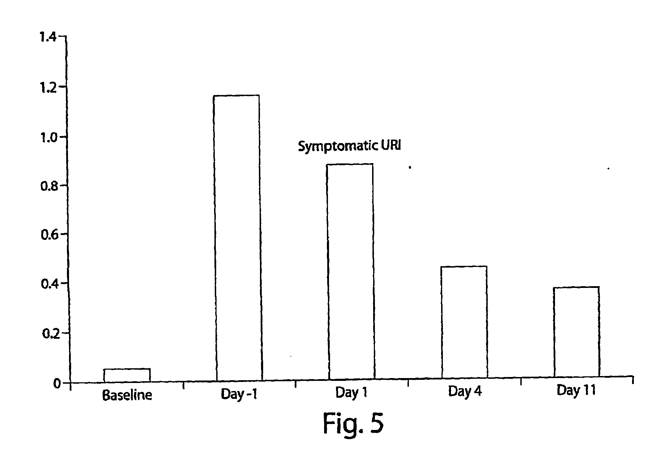

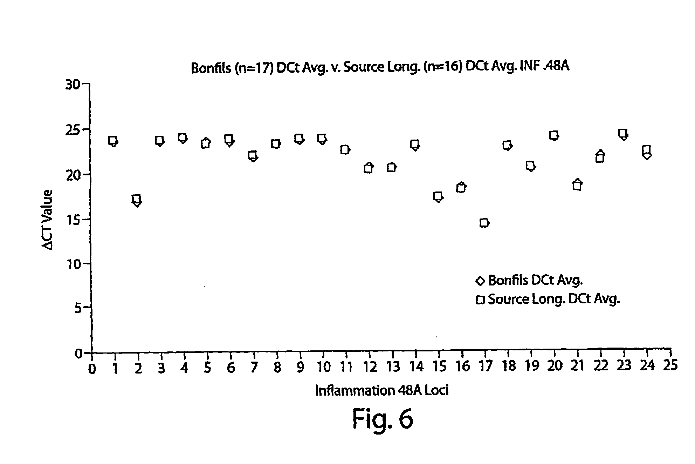

The profile data set may arise from the same subject for which the first data set is obtained, where the sample is taken at a separate or similar time, a different or similar site or in a different or similar biological condition. For example, Fig. 5 provides a protocol in which the sample is taken before stimulation or after stimulation. The profile data set obtained from the unstimulated sample may serve as a baseline profile data set for the sample taken after stimulation. The baseline data set may also be derived from a library containing profile data sets of a population or set of subjects having some defining characteristic or biological condition. The baseline profile data set may also correspond to some *ex vivo* or *in vitro* properties associated with an *in vitro* cell culture. The resultant calibrated profile data sets may then be stored as a record in a database or library (Fig. 6) along with or separate from the baseline profile data base and optionally the first profile data set although the first profile data set would normally become incorporated into a baseline profile data set under suitable classification criteria. The remarkable consistency of Gene Expression Profiles associated with a given biological condition makes it valuable to store profile data, which can be used, among other things for normative reference purposes. The normative reference can serve to indicate the degree to which a subject conforms to a given biological condition (healthy or diseased) and, alternatively or in addition, to provide a target for clinical intervention.

Selected baseline profile data sets may be also be used as a standard by which to judge manufacturing lots in terms of efficacy, toxicity, etc. Where the effect of a therapeutic agent is being measured, the baseline data set may correspond to Gene Expression Profiles taken before administration of the agent. Where quality control for a newly manufactured product is being determined, the baseline data set may correspond with a gold standard for that product. However, any suitable normalization techniques may be employed. For example, an average baseline profile data set is obtained from authentic material of a naturally grown herbal nutraceutical and compared over time and over different lots in order to demonstrate consistency, or lack of consistency, in lots of compounds prepared for release.

Calibrated data

Given the repeatability we have achieved in measurement of gene expression, described above in connection with "Gene Expression Panels" and "gene amplification", we conclude that where differences occur in measurement under such conditions, the differences are attributable to differences in biological condition. Thus is has been found that calibrated profile data sets are highly reproducible in samples taken from the same individual under the same conditions. Similarly, it has been found that calibrated profile data sets are reproducible in samples that are repeatedly tested. It has also been found repeated instances wherein calibrated profile data sets obtained when samples from a subject are exposed *ex vivo* to a compound are comparable to calibrated profile data from a sample that has been exposed to a sample *in vivo.* Importantly, it has been determined that an indicator cell line treated with an agent can in many cases provide calibrated profile data sets comparable to those obtained from *in vivo* or *ex vivo* populations of cells. Moreover, it has been determined that administering a sample from a subject onto indicator cells can provide informative calibrated profile data sets with respect to the biological condition of the subject including the health, disease states, therapeutic interventions, aging or exposure to environmental stimuli or toxins of the subject.

Calculation of calibrated profile data sets and computational aids

The calibrated profile data set may be expressed in a spreadsheet or represented graphically for example, in a bar chart or tabular form but may also be expressed in a three dimensional representation. The function relating the baseline and profile data may be a ratio expressed as a logarithm. The constituent may be itemized on the x-axis and the logarithmic scale may be on the y-axis. Members of a calibrated data set may be expressed as a positive value representing a relative enhancement of gene expression or as a negative value representing a relative reduction in gene expression with respect to the baseline.

Each member of the calibrated profile data set should be reproducible within a range with respect to similar samples taken from the subject under similar conditions. For example, the calibrated profile data sets may be reproducible within one order of magnitude with respect to similar samples taken from the subject under similar conditions. More particularly, the members may be reproducible within 50%, more particularly reproducible within 20%, and typically within 10%. In accordance with embodiments of the invention, a pattern of increasing, decreasing and no change in relative gene expression from each of a plurality of gene loci examined in the Gene Expression Panel may be used to prepare a calibrated profile set that is informative with regards to a biological condition, biological efficacy of an agent treatment conditions or for comparison to populations or sets of subjects or samples, or for comparison to populations of cells. Patterns of this nature may be used to identify likely candidates for a drug trial, used alone or in combination with other clinical indicators to be diagnostic or prognostic with respect to a biological condition or may be used to guide the development of a pharmaceutical or nutraceutical through manufacture, testing and marketing.

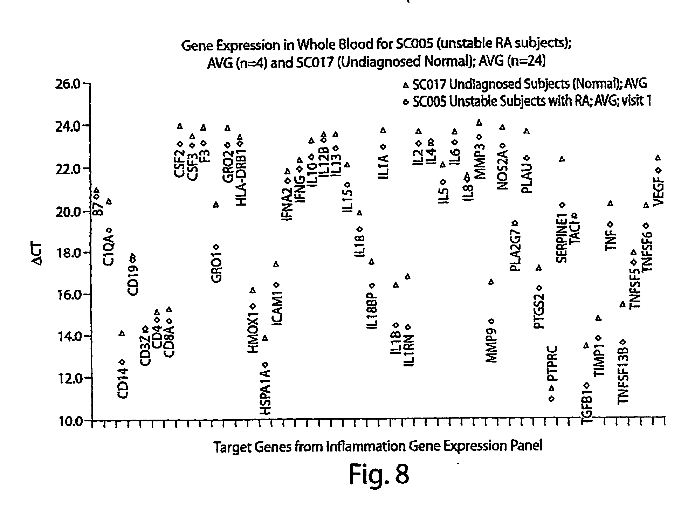

The numerical data obtained from quantitative gene expression and numerical data from calibrated gene expression relative to a baseline profile data set may be stored in databases or digital storage mediums and may retrieved for purposes including managing patient health care or for conducting clinical trials or for characterizing a drug. The data may be transferred in physical or wireless networks via the World Wide Web, email, or internet access site for example or by hard copy so as to be collected and pooled from distant geographic sites (Fig. 8).

The method also includes producing a calibrated profile data set for the panel, wherein each member of the calibrated profile data set is a function of a corresponding member of the first profile data set and a corresponding member of a baseline profile data set for the panel, and wherein the baseline profile data set is related to the multiple sclerosis or inflammatory conditions related to multiple sclerosis to be evaluated, with the calibrated profile data set being a comparison between the first profile data set and the baseline profile data set, thereby providing evaluation of the multiple sclerosis or inflammatory conditions related to multiple sclerosis of the subject.

In yet other embodiments, the function is a mathematical function and is other than a simple difference, including a second function of the ratio of the corresponding member of first profile data set to the corresponding member of the baseline profile data set, or a logarithmic function. In related embodiments, each member of the calibrated profile data set has biological significance if it has a value differing by more than an amount D, where D= F(1.1) - F(.9), and F is the second function. In such embodiments, the first sample is obtained and the first profile data set quantified at a first location, and the calibrated profile data set is produced using a network to access a database stored on a digital storage medium in a second location, wherein the database may be updated to reflect the first profile data set quantified from the sample. Additionally, using a network may include accessing a global computer network.

In an embodiment of the present invention, a descriptive record is stored in a single database or multiple databases where the stored data includes the raw gene expression data (first profile data set) prior to transformation by use of a baseline profile data set, as well as a record of the baseline profile data set used to generate the calibrated profile data set including for example, annotations regarding whether the baseline profile data set is derived from a particular Signature Panel and any other annotation that facilitates interpretation and use of the data.

Because the data is in a universal format, data handling may readily be done with a computer. The data is organized so as to provide an output optionally corresponding to a graphical representation of a calibrated data set.

For example, a distinct sample derived from a subject being at least one of RNA or protein may be denoted as $P_l$. The first profile data set derived from sample $P_l$ is denoted Mj, where $M_j$ is a quantitative measure of a distinct RNA or protein constituent of $P_l$. The record Ri is a ratio of M and P and may be annotated with additional data on the subject relating to, for example, age, diet, ethnicity, gender, geographic location, medical disorder, mental disorder, medication, physical activity, body mass and environmental exposure. Moreover, data handling may further include accessing data from a second condition database which may contain additional medical data not presently held with the calibrated profile data sets. In this context, data access may be via a computer network.

The above described data storage on a computer may provide the information in a form that can be accessed by a user. Accordingly, the user may load the information onto a second access site including downloading the information. However, access may be restricted to users having a password or other security device so as to protect the medical records contained within. A feature of this embodiment of the invention is the ability of a user to add new or annotated records to the data set so the records become part of the biological information.

The graphical representation of calibrated profile data sets pertaining to a product such as a drug provides an opportunity for standardizing a product by means of the calibrated profile, more particularly a signature profile. The profile may be used as a feature with which to demonstrate relative efficacy, differences in mechanisms of actions, etc. compared to other drugs approved for similar or different uses.

The various embodiments of the invention may be also implemented as a computer program product for use with a computer system. The product may include program code for deriving a first profile data set and for producing calibrated profiles. Such implementation may include a series of computer instructions fixed either on a tangible medium, such as a computer readable medium (for example, a diskette, CD-ROM, ROM, or fixed disk), or transmittable to a computer system via a modem or other interface device, such as a communications adapter coupled to a network. The network coupling may be for example, over optical or wired communications lines or via wireless techniques (for example, microwave, infrared or other transmission techniques) or some combination of these. The series of computer instructions preferably embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (for example, shrink wrapped software), preloaded with a computer system (for example, on system ROM or fixed disk), or distributed from a server or electronic bulletin board over a network (for example, the Internet or World Wide Web). In addition, a computer system is further provided including derivative modules for deriving a first data set and a calibration profile data set.

The calibration profile data sets in graphical or tabular form, the associated databases, and the calculated index or derived algorithm, together with information extracted from the panels, the databases, the data sets or the indices or algorithms are commodities that can be sold together or separately for a variety of purposes as described in WO 01/25473.

In other embodiments, a clinical indicator may be used to assess the multiple sclerosis or inflammatory conditions related to multiple sclerosis of the relevant set of subjects by interpreting the calibrated profile data set in the context of at least one other clinical indicator, wherein the at least one other clinical indicator is selected from the group consisting of blood chemistry, urinalysis, X-ray or other radiological or metabolic imaging technique, other chemical assays, and physical findings.

<u>Index construction</u>

In combination, (i) the remarkable consistency of Gene' Expression Profiles with respect to a biological condition across a population or set of subject or samples, or across a population of cells and (ii) the use of procedures that provide substantially reproducible measurement of constituents in a Gene Expression Panel giving rise to a Gene Expression Profile, under measurement conditions wherein specificity and efficiencies of amplification for all constituents of the panel are substantially similar, make possible the use of an index that characterizes a Gene Expression Profile, and which therefore provides a measurement of a biological condition.

An index may be constructed using an index function that maps values in a Gene Expression Profile into a single value that is pertinent to the biological condition at hand. The values in a Gene Expression Profile are the amounts of each constituent of the Gene Expression Panel that corresponds to the Gene Expression Profile. These constituent amounts form a profile data set, and the index function generates a single value—the index— from the members of the profile data set.

The index function may conveniently be constructed as a linear sum of terms, each term being what we call a "contribution function" of a member of the profile data set. For example, the contribution function may be a constant times a power of a member of the profile data set. So the index function would have the form

$$I = \sum C_i M_i{}^{P(i)},$$

where I is the index, $M_i$ is the value of the member $i$ of the profile data set, $C_i$ is a constant, and P(i) is a power to which

$M_i$ is raised, the sum being formed for all integral values of $i$ up to the number of members in the data set. We thus have a linear polynomial expression.

The values $C_i$ and P(i) may be determined in a number of ways, so that the index $I$ is informative of the pertinent biological condition. One way is to apply statistical techniques, such as latent class modeling, to the profile data sets to correlate clinical data or experimentally derived data, or other data pertinent to the biological condition. In this connection, for example, may be employed the software from Statistical Innovations, Belmont, Massachusetts, called Latent Gold®. See the web pages at statisticalinnovations.com/lg/, which are hereby incorporated herein by reference.

Alternatively, other simpler modeling techniques may be employed in a manner known in the art. The index function for inflammation may be constructed, for example, in a manner that a greater degree of inflammation (as determined by the a profile data set for the Inflammation Gene Expression Profile) correlates with a large value of the index function. In a simple embodiment, therefore, each P (i) may be +1 or- 1, depending on whether the constituent increases or decreases with increasing inflammation. As discussed in further detail below, we have constructed a meaningful inflammation index that is proportional to the expression

$$1/4\{IL1A\} + 1/4\{IL1B\} + 1/4\{TNF\} + 1/4\{INFG\} - 1/\{IL10\},$$

where the braces around a constituent designate measurement of such constituent and the constituents are a subset of the Inflammation Gene Expression Panel.

Just as a baseline profile data set, discussed above, can be used to provide an appropriate normative reference, and can even be used to create a Calibrated profile data set, as discussed above, based on the normative reference, an index that characterizes a Gene Expression Profile can also be provided with a normative value of the index function used to create the index. This normative value can be determined with respect to a relevant population or set of subjects or samples or to a relevant population of cells, so that the index may be interpreted in relation to the normative value. The relevant population or set of subjects or samples, or relevant population of cells may have in common a property that is at least one of age range, gender, ethnicity, geographic location, nutritional history, medical condition, clinical indicator, medication, physical activity, body mass, and environmental exposure.

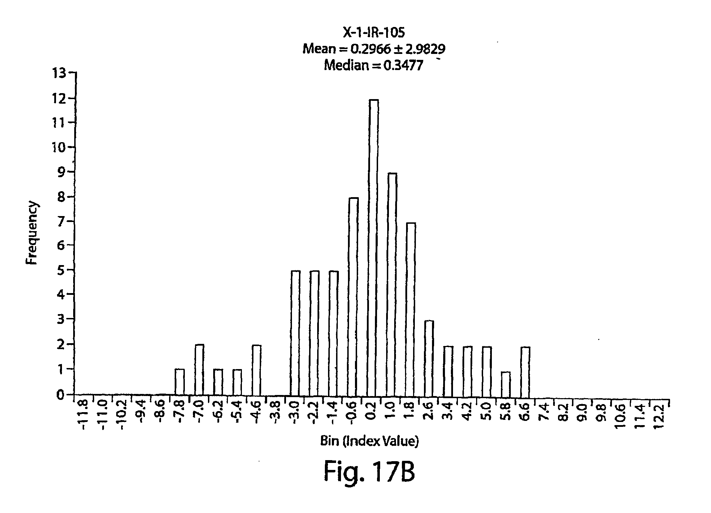

As an example, the index can be constructed, in relation to a normative Gene Expression Profile for a population or set of healthy subjects, in such a way that a reading of approximately 1 characterizes normative Gene Expression Profiles of healthy subjects. Let us further assume that the biological condition that is the subject of the index is inflammation; a reading of 1 in this example thus corresponds to a Gene Expression Profile that matches the norm for healthy subjects. A substantially higher reading then may identify a subject experiencing an inflammatory condition. The use of 1 as identifying a normative value, however, is only one possible choice; another logical choice is to use 0 as identifying the normative value. With this choice, deviations in the index from zero can be indicated in standard deviation units (so that values lying between -1 and +1 encompass 90% of a normally distributed reference population or set of subjects. Since we have found that Gene Expression Profile values (and accordingly constructed indices based on them) tend to be normally distributed, the 0-centered index constructed in this manner is highly informative. It therefore facilitates use of the index in diagnosis of disease and setting objectives for treatment. The choice of 0 for the normative value, and the use of standard deviation units, for example, are illustrated in Fig. 17B, discussed below.

Still another embodiment is a method of providing an index that is indicative of multiple sclerosis or inflammatory conditions related to multiple sclerosis of a subject based on a first sample from the subject, the first sample providing a source of RNAs, the method comprising deriving from the first sample a profile data set, the profile data set including a plurality of members, each member being a quantitative measure of the amount of a distinct RNA constituent in a panel of constituents selected so that measurement of the constituents is indicative of the presumptive signs of multiple sclerosis, the panel including at least two of the constituents of any of the Gene Expression Panels of Tables 1-9. In deriving the profile data set, such measure for each constituent is achieved under measurement conditions that are substantially repeatable, at least one measure from the profile data set is applied to an index function that provides a mapping from at least one measure of the profile data set into one measure of the presumptive signs of multiple sclerosis, so as to produce an index pertinent to the multiple sclerosis or inflammatory conditions related to multiple sclerosis of the subject.

As a further embodiment of the invention, we can employ an index function $I$ of the form

$$I = C_0 + \sum_{i=1}^{N} C_i M_i + \sum_{i=1}^{N} \sum_{j=1}^{N} C_{ij} M_i M_j,$$

where $M_i$ and $M_j$ are values respectively of the member i and member *j* of the profile data set having *N* members, and $C_i$ and $C_{ij}$ are constants,. For example, when $C_i = C_{ij} = 0$, the index function is simply the constant $C_0$. More importantly, when $C_{ij} = 0$, the index function is a linear expression, in a form used for examples herein. Similarly, when $C_{ij} = 0$ only when $i \neq j$, the index function is a simple quadratic expression without cross products Otherwise, the index function is a quadratic with cross products. As discussed in further detail below, a quadratic expression that is constructed as a meaningful identifier of rheumatoid arthritis (RA) is the following:

$$C_0 + C_1\{TLR2\} + C_2\{CD4\} + C_3\{NFKB1\} + C_4\{TLR2\}\{CD4\} +$$
$$C_5\{TLR2\}\{NFKB1\} + C_6\{NFKB1\}^2 + C_7\{TLR2\}^2 + C_8\{CD4\}^2,$$

where the constant $C_0$ serves to calibrate this expression to the biological population of interest (such as RA), that is characterized by inflammation.

In this embodiment, when the index value associated with a subject equals 0, the odds are 50: 50 of the subject's being MS vs normal. More generally, the predicted odds of being MS is $[\exp (I_i)]$, and therefore the predicted probability of being MS is $[\exp (I_i)] / [1+\exp((I_i)]$. Thus, when the index exceeds 0, the predicted probability that a subject is MS is higher than . 5, and when it falls below 0, the predicted probability is less than . 5.

The value of $C_0$ may be adjusted to reflect the prior probability of being in this population based on known exogenous risk factors for the subject. In an embodiment where $C_0$ is adjusted as a function of the subject's risk factors, where the subject has prior probability $p_i$ of being RA based on such risk factors, the adjustment is made by increasing (decreasing) the unadjusted $C_0$ value by adding to $C_0$ the natural logarithm of the ratio of the prior odds of being RA taking into account the risk factors to the overall prior odds of being RA *without* taking into account the risk factors.

It was determined that the above quadratic expression for RA may be well approximated by a linear expression of the form: $D_0 + D_1\{TLR2\} + D_2\{CD4\} + D_3\{NFKB1\}$.

Yet another embodiment provides a method of using an index for differentiating a type of pathogen within a class of pathogens of interest in a subject with multiple sclerosis or inflammatory conditions related to multiple sclerosis, based on at least one sample from the subject, the method comprising providing at least one index according to any of the above disclosed embodiments for the subject, comparing the at least one index to at least one normative value of the index, determined with respect to at least one relevant set of subjects to obtain at least one difference, and using the at least one difference between the at least one index and the at least one normative value for the index to differentiate the type of pathogen from the class of pathogen.

<u>Kits</u>

The invention also includes an MS-detection reagent, i.e., nucleic acids that specifically identify one or more multipe sclerosis or inflammatory condition related to multiple sclerosis nucleic acids (e.g., any gene listed in Tables 1-9; refered to herein as MS-associated genes) by having homologous nucleic acid sequences, such as oligonucleotide sequences, complementary to a portion of the MS-associated genes nucleic acids or antibodies to proteins encoded by the MS-associated genes nucleic acids packaged together in the form of a kit. The oligonucleotides can be fragments of the MS-associated genes genes. For example the oligonucleotides can be 200, 150, 100, 50, 25, 10 or less nucleotides in length. The kit may contain in separate containers a nucleic acid or antibody (either already bound to a solid matrix or packaged separately with reagents for binding them to the matrix), control formulations (positive and/or negative), and/or a detectable label. Instructions (i.e., written, tape, VCR, CD-ROM, etc.) for carrying out the assay may be included in the kit. The assay may for example be in the form of PCR, a Northern hybridization or a sandwich ELISA as known in the art.

For example, MS-associated genes detection reagents can be immobilized on a solid matrix such as a porous strip to form at least one MS-associated genes detection site. The measurement or detection region of the porous strip may include a plurality of sites containing a nucleic acid. A test strip may also contain sites for negative and/or positive controls. Alternatively, control sites can be located on a separate strip from the test strip. Optionally, the different detection sites may contain different amounts of immobilized nucleic acids, i.e., a higher amount in the first detection site and lesser amounts in subsequent sites. Upon the addition of test sample, the number of sites displaying a detectable signal provides a quantitative indication of the amount of MS-associated genes present in the sample. The detection sites may be configured in any suitably detectable shape and are typically in the shape of a bar or dot spanning the width of a test strip.

Alternatively, the kit contains a nucleic acid substrate array comprising one or more nucleic acid sequences. The nucleic acids on the array specifically identify one or more nucleic acid sequences represented by MS- associated genes 1- 72. In various embodiments, the expression of 2, 3, 4, 5, 6, 7, 8, 9, 10, 15, 20, 25, 40 or 50 or more of the sequences represented by MS- associated genes 1- 72 can be identified by virtue of binding to the array. The substrate array can be on, i.e., a solid substrate, i.e., a "chip" as described in U.S. Patent No. 5, 744, 305. Alternatively, the substrate array can be a solution array, i.e., Luminex, Cyvera, Vitra and Quantum Dots' Mosaic.

The skilled artisan can routinely make antibodies, nucleic acid probes, i.e., oligonucleotides, aptamers, siRNAs, antisense oligonucleotides, against any of the MS-associated genes in Tables 1-9.

**Other Embodiments**

While the invention has been described in conjunction with the detailed description thereof, the foregoing description is intended to illustrate and not limit the scope of the invention, which is defined by the scope of the appended claims.

**Examples**

Example 1: Acute Inflammatory Index to Assist in Analysis of Large, Complex Data Sets.

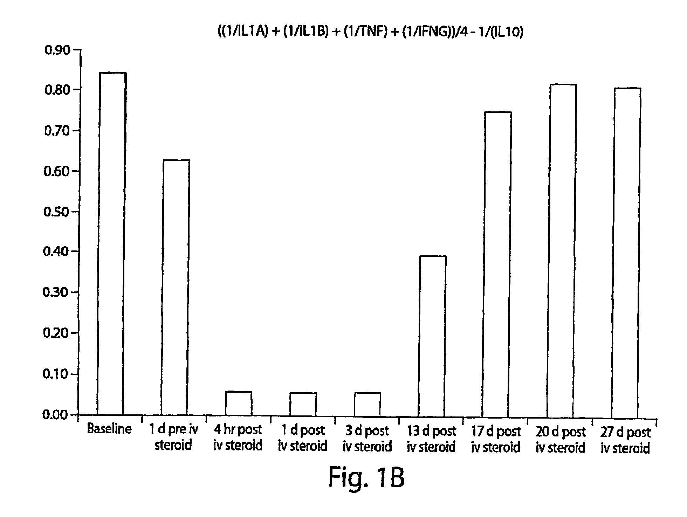

In one embodiment of the invention the index value or algorithm can be used to reduce a complex data set to a single index value that is informative with respect to the inflammatory state of a subject. This is illustrated in Figs. 1A and 1B.

Fig. 1A is entitled Source Precision Inflammation Profile Tracking of A Subject Results in a Large, Complex Data Set. The figure shows the results of assaying 24 genes from the Inflammation Gene Expression Panel on eight separate days during the course of optic neuritis in a single male subject.

Fig. 1B shows use of an Acute Inflammation Index. The data displayed in Fig. 1A above is shown in this figure after calculation using an index function proportional to the following mathematical expression: $(1/4\{IL1A\} + 1/4\{IL1B\} + 1/4\{TNF\} + 1/4\{INFG\} - 1/\{IL10\})$.

Example 2: Use of acute inflammation index or algorithm to monitor a biologicalcondition of a sample or a subject.

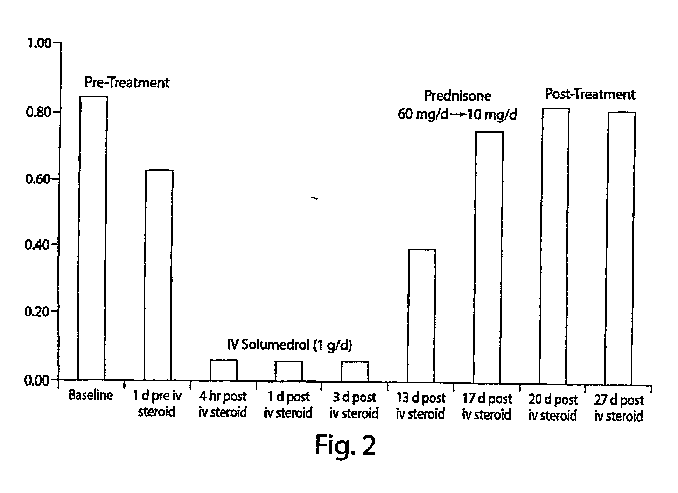

The inflammatory state of a subject reveals infonnation about the past progress of the biological condition, future progress, response to treatment, etc. The Acute Inflammation Index may be used to reveal such information about the biological condition of a subject. This is illustrated in Fig. 2.

The results of the assay for inflammatory gene expression for each day (shown for 24 genes in each row of Fig. IA) is displayed as an individual histogram after calculation. The index reveals clear trends in inflammatory status that may correlated with therapeutic intervention (Fig. 2).

Fig. 2 is a graphical illustration of the acute inflammation index calculated at 9 different, significant clinical milestones from blood obtained from a single patient treated medically with for optic neuritis. Changes in the index values for the Acute Inflammation Index correlate strongly with the expected effects of therapeutic intervention. Four clinical milestones have been identified on top of the Acute Inflammation Index in this figure including (1) prior to treatment with steroids, (2) treatment with IV solumedrol at 1 gram per day, (3) post-treatment with oral prednisone at 60 mg per day tapered to 10 mg per day and (4) post treatment. The data set is the same as for Fig. 1. The index is proportional to $1/4\{IL1A\} + 1/4\{IL1B\} + 1/4\{TNF\} + 1/4\{INFG\} - 1/\{IL10\}$. As expected, the acute inflammation index falls rapidly with treatment with IV steroid, goes up during less efficacious treatment with oral prednisone and returns to the pre-treatment level after the steroids have been discontinued and metabolized completely.

Example 3: Use of the acute inflammatory index to set dose,

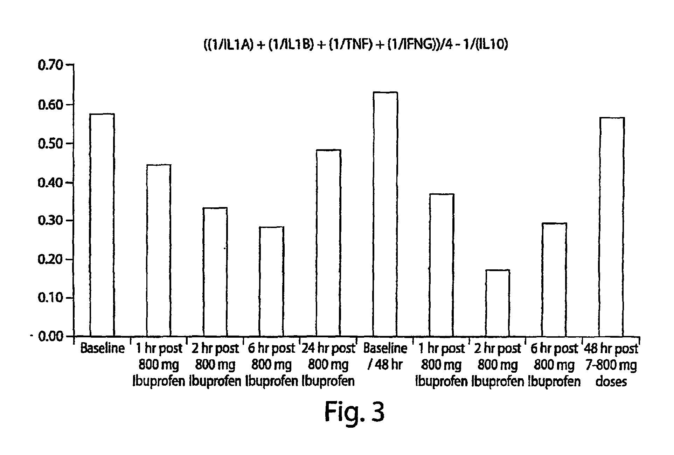

including concentrations and timing, for compounds in development or for compounds to be tested in human and non-human subjects as shown in Fig. 3. The acute inflammation index may be used as a common reference value for therapeutic compounds or interventions without common mechanisms of action. The compound that induces a gene response to a compound as indicated by the index, but fails to ameliorate a known biological conditions may be compared to a different compounds with varying effectiveness in treating the biological condition.

Fig. 3 shows the effects of single dose treatment with 800 mg of ibuprofen in a single donor as characterized by the Acute Inflammation Index. 800 mg of over-the-counter ibuprofen were taken by a single subject at Time=0 and Time=48 hr. Gene expression values for the indicated five inflammation-related gene loci were determined as described below at times=2, 4, 6, 48, 50, 56 and 96 hours. As expected the acute inflammation index falls immediately after taking the non-steroidal anti-inflammatory ibuprofen and returns to baseline after 48 hours. A second dose at T=48 follows the same kinetics at the first dose and returns to baseline at the end of the experiment at T=96.

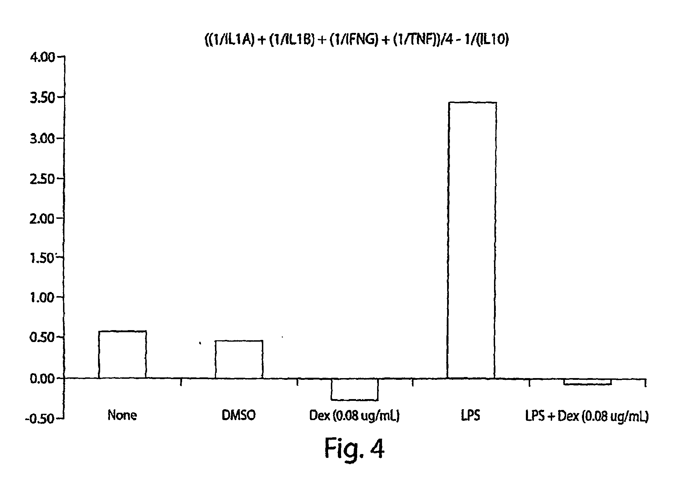

Example 4: Use of the acute inflammation index to characterize efficacy, safety, and mode ofphysiological action for an agent,

which may be in development and/or may be complex in nature. This is illustrated in Fig. 4.

Fig. 4 shows that the calculated acute inflammation index displayed graphically for five different conditions including (A) untreated whole blood; (B) whole blood treated in vitro with DMSO, an non-active carrier compound; (C) otherwise unstimulated whole blood treated in vitro with dexamethasone (0.08 ug/ml); (D) whole blood stimulated in vitro with lipopolysaccharide, a known pro-inflammatory compound, (LPS, 1 ng/ml) and (E) whole blood treated in vitro with LPS (1 ng/ml) and dexamethasone (0.08 ug/ml). Dexamethasone is used as a prescription compound that is commonly used medically as an anti-inflammatory steroid compound. The acute inflammation index is calculated from the experimentally determined gene expression levels of inflaimnation-related genes expressed in human whole blood obtained from a single patient. Results of mRNA expression are expressed as Ct's in this example, but may be expressed as, e.g., relative fluorescence units, copy number or any other quantifiable, precise and calibrated form, for the genes ILIA, IL1B, TNF, IFNG and IL10. From the gene expression values, the acute inflammation values were determined algebraically according in proportion to the expression $1/4\{IL1A\} + 1/4\{IL1B\} + 1/4\{TNF\} + 1/4\{INFG\} - 1/\{IL10\}$.

Example 5: Development and use of population nonnative values for Gene

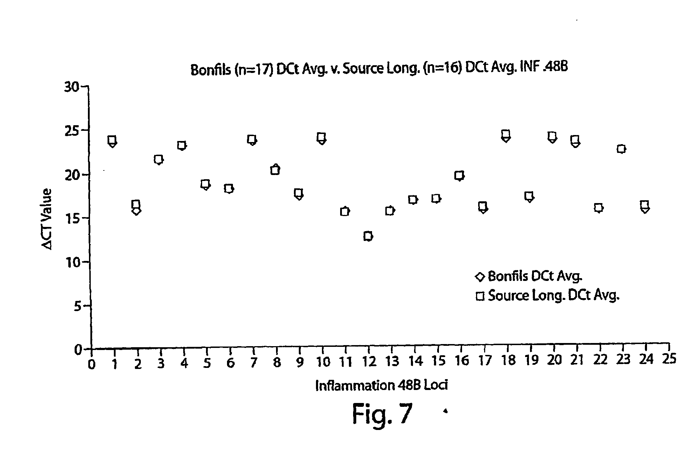

Expression Profiles. Figs. 6 and 7 show the arithmetic mean values for gene expression profiles (using the 48 loci of the Inflammation Gene Expression Panel) obtained from whole blood of two distinct patient populations (patient sets). These patient sets are both normal or undiagnosed. The first patient set, which is identified as Bonfils (the plot points for which are represented by diamonds), is composed of 17 subjects accepted as blood donors at the Bonfils Blood Center in Denver, Colorado. The second patient set is 9 donors, for which Gene Expression Profiles were obtained from assays conducted four times over a four-week period. Subjects in this second patient set (plot points for which are represented by squares) were recruited from employees of Source Precision Medicine, Inc., the assignee herein. Gene expression averages for each population were calculated for each of 48 gene loci of the Gene Expression Inflammation Panel. The results for loci 1-24 (sometimes referred to below as the Inflammation 48A loci) are shown in Fig. 6 and for loci 25-48 (sometimes referred to below as the Inflammation 48B loci) are shown in Fig. 7.

The consistency between gene expression levels of the two distinct patient sets is dramatic. Both patient sets show gene expressions for each of the 48 loci that are not significantly different from each other. This observation suggests that there is a "normal" expression pattern for human inflammatory genes, that a Gene Expression Profile, using the Inflammation Gene Expression Panel (or a subset thereof) characterizes that expression pattern, and that a population-normal expression pattern can be used, for example, to guide medical intervention for any biological condition that results in a change from the normal expression pattern.

In a similar vein, Fig. 8 shows arithmetic mean values for gene expression profiles (again using the 48 loci of the Inflammation Gene Expression Panel) also obtained from whole blood of two distinct patient populations (patient sets). One patient set, expression values for which are represented by triangular data points, is 24 normal, undiagnosed subjects (who therefore have no known inflammatory disease). The other patient set, the expression values for which are represented by diamond-shaped data points, is four patients with rheumatoid arthritis and who have failed therapy (who therefore have unstable rheumatoid arthritis).

As remarkable as the consistency of data from the two distinct normal patient sets shown in Figs. 6 and 7 is the systematic divergence of data from the normal and diseased patient sets shown in Fig. 8. In 45 of the shown 48 inflammatory gene loci, subjects with unstable rheumatoid arthritis showed, on average, increased inflammatory gene expression (lower cycle threshold values; Ct), than subjects without disease. The data thus further demonstrate that is possible to identify groups with specific biological conditions using gene expression if the precision and calibration of the underlying assay are carefully designed and controlled according to the teachings herein.

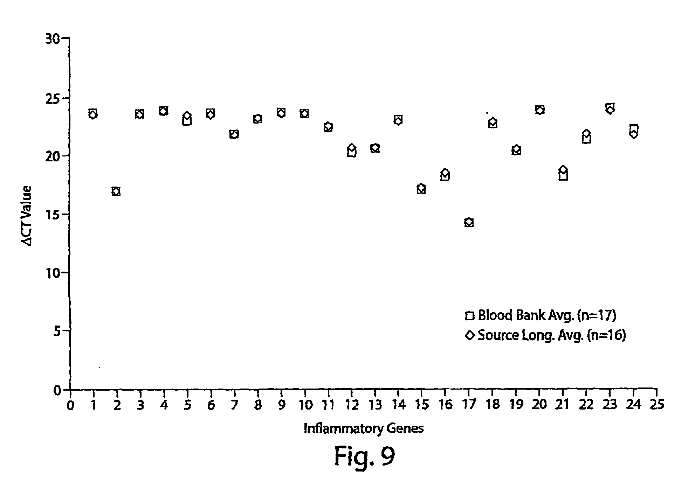

Fig. 9, in a manner analogous to Fig. 8, shows the shows arithmetic mean values for gene expression profiles using 24 loci of the Inflammation Gene Expression Panel) also obtained from whole blood of two distinct patient sets. One patient set, expression values for winch are represented by diamond-shaped data points, is 17 normal, undiagnosed subjects (who therefore have no known inflammatory disease) who are blood donors. The other patient set, the expression values for which are represented by square-shaped data points, is 16 subjects, also normal and undiagnosed, who have been monitored over six months, and the averages of these expression values are represented by the square-shaped data points. Thus the cross-sectional gene expression-value averages of a first healthy population match closely the longitudinal gene expression-value averages of a second healthy population, with approximately 7% or less variation in measured expression value on a gene-to-gene basis.

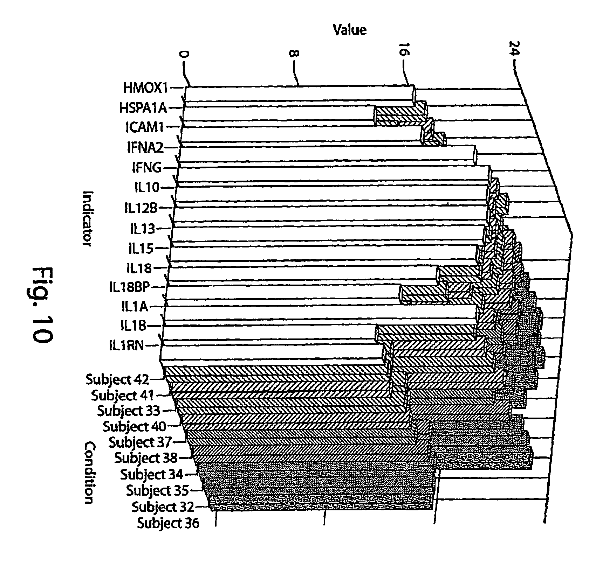

Fig. 10 shows the shows gene expression values (using 14 loci of the Inflammation Gene Expression Panel) obtained from whole blood of 44 normal undiagnosed blood donors (data for 10 subjects of which is shown). Again, the gene expression values for each member of the population (set) are closely matched to those for the entire set, represented visually by the consistent peak heights for each of the gene loci. Other subjects of the set and other gene loci than those depicted here display results that are consistent with those shown here.

In consequence of these principles, and in various embodiments of the present invention, population normative values for a Gene Expression Profile can be used in comparative assessment of individual subjects as to biological condition, including both for purposes of health and/or disease. In one embodiment the normative values for a Gene Expression Profile may be used as a baseline in computing a "calibrated profile data set" (as defined at the beginning of this section) for a subject that reveals the deviation of such subject's gene expression from population normative values. Population normative values for a Gene Expression Profile can also be used as baseline values in constructing index functions in accordance with embodiments of the present invention. As a result, for example, an index function can be constructed to reveal not only the extent of an individual's inflammation expression generally but also in relation to normative values.

Example 6: Consistency of expression values, of constituents in Gene Expression Panels, over time as reliable indicators of biological condition.

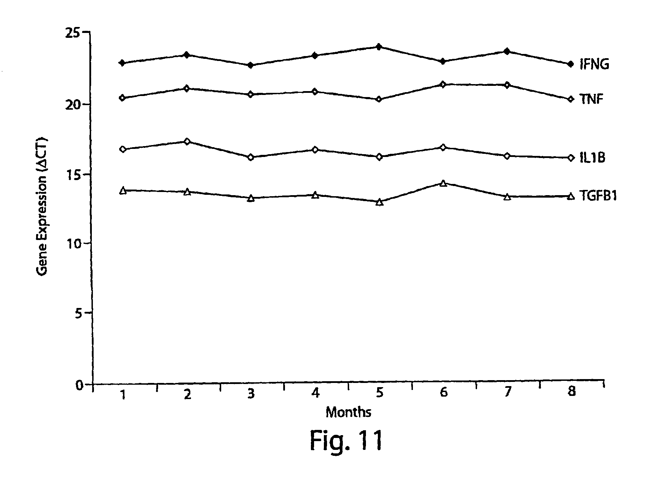

Fig. 11 shows the expression levels for each of four genes (of the Inflammation Gene Expression Panel), of a single subject, assayed monthly over a period of eight months. It can be seen that the expression levels are remarkably consistent over time.

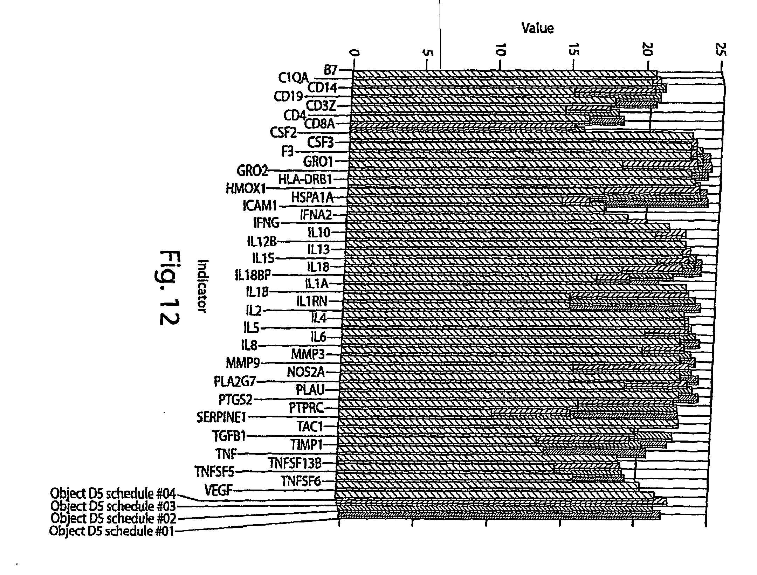

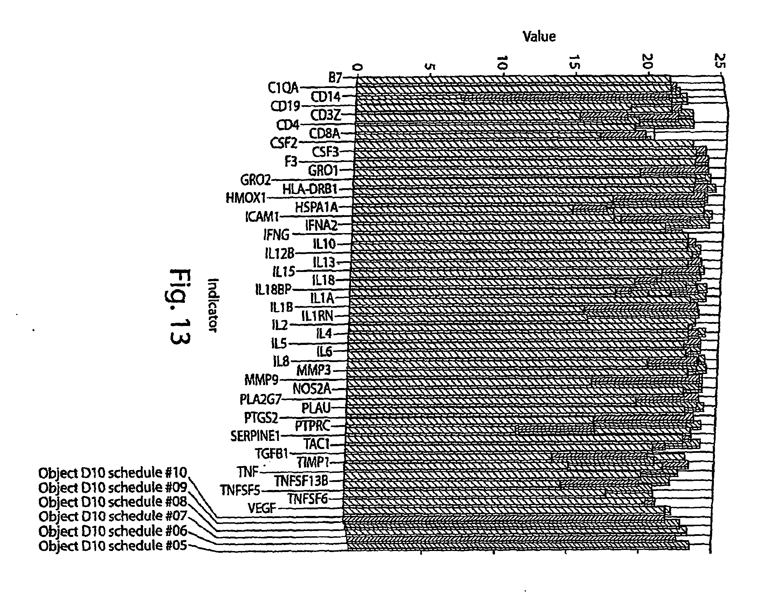

Figs. 12 and 13 similarly show in each case the expression levels for each of 48 genes (of the Inflammation Gene Expression Panel), of distinct single subjects (selected in each case on the basis of feeling well and not taking drugs), assayed, in the case of Fig. 12 weekly over a period of four weeks, and in the case of Fig. 13 monthly over a period of six months. In each case, again the expression levels are remarkably consistent over time, and also similar across individuals.

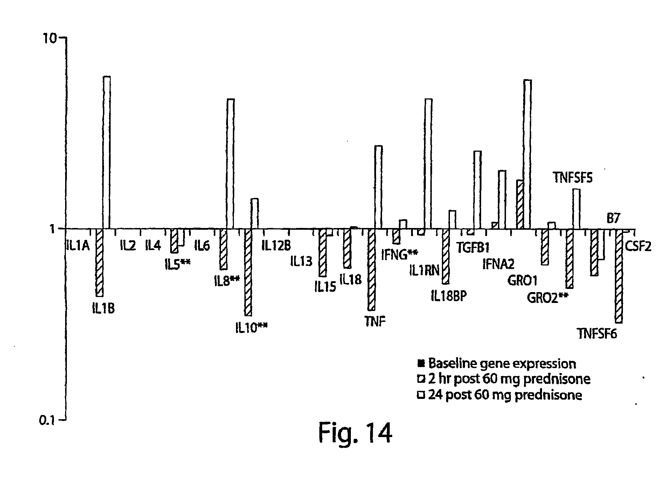

Fig. 14 also shows the effect over time, on inflammatory gene expression in a single human subject, of the administration of an anti-inflammatory steroid, as assayed using the Inflammation Gene Expression Panel. In this case, 24 of 48 loci are displayed. The subject had a baseline blood sample drawn in a PAX RNA isolation tube and then took a single 60 mg dose of prednisone, an anti-inflammatory, prescription steroid. Additional blood samples were drawn at 2 hr and 24 hr post the single oral dose. Results for gene expression are displayed for all three time points, wherein values for the baseline sample are shown as unity on the x-axis. As expected, oral treatment with prednisone resulted in the decreased expression of most of inflammation-related gene loci, as shown by the 2-hour post-administration bar graphs. However, the 24-hour post-administration bar graphs show that, for most of the gene loci having reduced gene expression at 2 hours, there were elevated gene expression levels at 24 hr.

Although the baseline in Fig. 14 is based on the gene expression values before drug intervention associated with the single individual tested, we know from the previous example, that healthy individuals tend toward population normative values in a Gene Expression Profile using the Inflammation Gene Expression Panel (or a subset of it). We conclude from Fig. 14 that in an attempt to return the inflammatory gene expression levels to those demonstrated in Figs. 6 and 7 (normal or set levels), interference with the normal expression induced a compensatory gene expression response that over-compensated for the drug-induced response, perhaps because the prednisone had been significantly metabolized to inactive forms or eliminated from the subj ect.

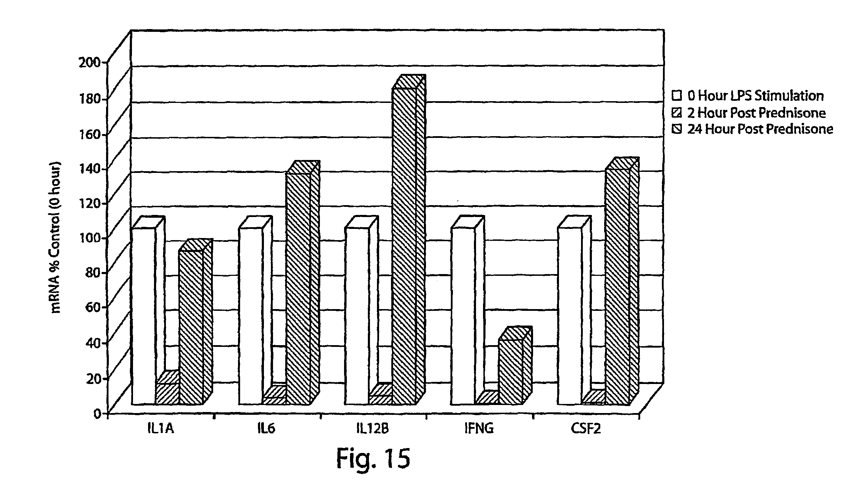

Fig. 15, in a manner analogous to Fig. 14, shows the effect over time, via whole blood samples obtained from a human subj ect, administered a single dose of prednisone, on expression of 5 genes (of the Inflammation Gene Expression Panel). The samples were taken at the time of administration (t = 0) of the prednisone, then at two and 24 hours after such administration. Each whole blood sample was challenged by the addition of 0.1 ng/ml of lipopolysaccharide (a Gram-negative endotoxin) and a gene expression profile of the sample, post-challenge, was determined. It can seen that the two-hour sample shows dramatically reduced gene expression of the 5 loci of the Inflammation Gene Expression Panel, in relation to the expression levels at the time of administration (t = 0). At 24 hours post administration, the inhibitory effect of the prednisone is no longer apparent, and at 3 of the 5 loci, gene expression is in fact higher than at t = 0, illustrating quantitatively at the molecular level the well-known rebound effect.

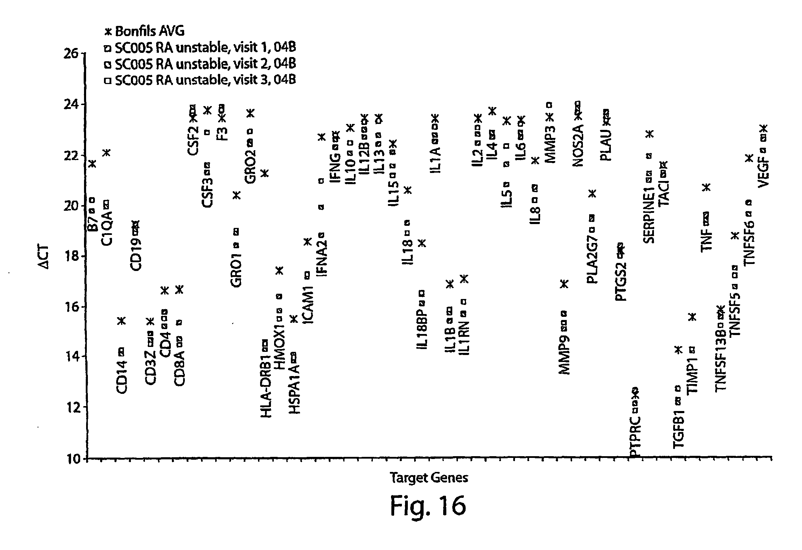

Fig. 16 also shows the effect over time, on inflammatory gene expression in a single human subject suffering from rheumatoid arthritis, of the administration of a TNF-inhibiting compound, but here the expression is shown in comparison to the cognate locus average previously determined (in connection with Figs. 6 and 7) for the normal (i.e., undiagnosed, healthy) patient set. As part of a larger international study involving patients with rheumatoid arthritis, the subject was followed over a twelve-week period. The subject was enrolled in the study because of a failure to respond to conservative drug therapy for rheumatoid arthritis and a plan to change therapy and begin immediate treatment with a TNF-inhibiting compound. Blood was drawn from the subject prior to initiation of new therapy (visit 1). After initiation of new therapy, blood was drawn at 4 weeks post change in therapy (visit 2), 8 weeks (visit 3), and 12 weeks (visit 4) following the start of new therapy. Blood was collected in PAX RNA isolation tubes, held at room temperature for two hours and then frozen at-30°C.

Frozen samples were slipped to the central laboratory at Source Precision Medicine, the assignee herein, in Boulder, Colorado for determination of expression levels of genes in the 48-gene Inflammation Gene Expression Panel. The blood samples were thawed and RNA extracted according to the manufacturer's recommended procedure. RNA was converted to cDNA and the level of expression of the 48 inflammatory genes was determined. Expression results are shown for 11 of the 48 loci in Fig. 16. When the expression results for the 11 loci are compared from visit one to a population average of normal blood donors from the United States, the subject shows considerable difference. Similarly, gene expression levels at each of the subsequent physician visits for each locus are compared to the same normal average value. Data from visits 2, 3 and 4 document the effect of the change in therapy. In each visit following the change in the therapy, the level of inflammatory gene expression for 10 of the 11 loci is closer to the cognate locus average previously determined for the normal (i.e., undiagnosed, healthy) patient set.

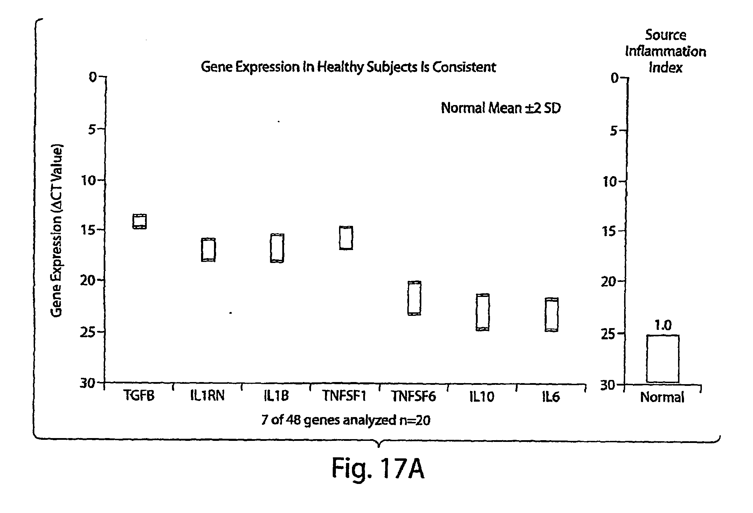

Fig. 17A further illustrates the consistency of inflammatory gene expression, illustrated here with respect to 7 loci of (of the Inflammation Gene Expression Panel), in a set of 44 normal, undiagnosed blood donors. For each individual locus is shown the range of values lying within $\pm$ 2 standard deviations of the mean expression value, which corresponds to 95% of a normally distributed population. Notwithstanding the great width of the confidence interval (95%), the measured gene expression value ($\Delta CT$)- remarkably- still lies within 10% of the mean, regardless of the expression level involved. As described in further detail below, for a given biological condition an index can be constructed to provide a measurement of the condition. This is possible as a result of the conjunction of two circumstances: (i) there is a remarkable consistency of Gene Expression Profiles with respect to a biological condition across a population and (ii) there can be employed procedures that provide substantially reproducible measurement of constituents in a Gene Expression Panel giving rise to a Gene Expression Profile, under measurement conditions wherein specificity and efficiencies of amplification for all constituents of the panel are substantially similar and which therefore provides a measurement of a biological condition. Accordingly, a function of the expression values of representative constituent loci of Fig. 17A is here used to generate an inflammation index value, which is normalized so that a reading of 1 corresponds to constituent expression values of healthy subjects, as shown in the right- hand portion of Fig. 17A.

In Fig. 17B, an inflammation index value was determined for each member of a set of 42 normal undiagnosed blood donors, and the resulting distribution of index values, shown in the figure, can be seen to approximate closely a normal distribution, notwithstanding the relatively small subject set size. The values of the index are shown relative to a 0-based median, with deviations from the median calibrated in standard deviation units. Thus 90% of the subject set lies within +1 and -1 of a 0 value. We have constructed various indices, which exhibit similar behavior.

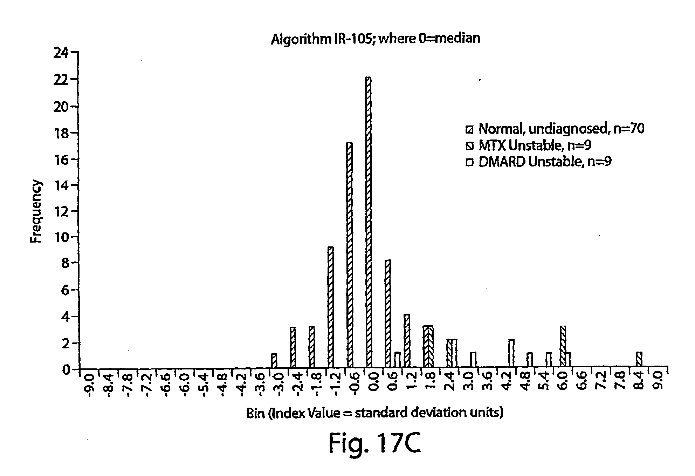

Fig. 17C illustrates the use of the same index as Fig. 17B, where the inflammation median for a normal population of subjects has been set to zero and both normal and diseased subjects are plotted in standard deviation units relative to that median. An inflammation index value was determined for each member of a normal, undiagnosed population of 70 individuals (black bars). The resulting distribution of index values, shown in Fig. 17C, can be seen to approximate closely a normal distribution. Similarly, index values were calculated for individuals from two diseased population groups, (1) rheumatoid arthritis patients treated with methotrexate (MTX) who are about to change therapy to more efficacious drugs (e.g., TNF inhibitors)(hatched bars), and (2) rheumatoid arthritis patients treated with disease modifying anti-rheumatoid drugs (DMARDS) other than MTX, who are about to change therapy to more efficacious drugs (e.g., MTX). Both populations of subjects present index values that are skewed upward (demonstrating increased inflammation) in comparison to the normal distribution. This figure thus illustrates the utility of an index to derived from Gene Expression Profile data to evaluate disease status and to provide an objective and quantifiable treatment objective. When these two populations of subjects were treated appropriately, index values from both populations returned to a more normal distribution (data not shown here).

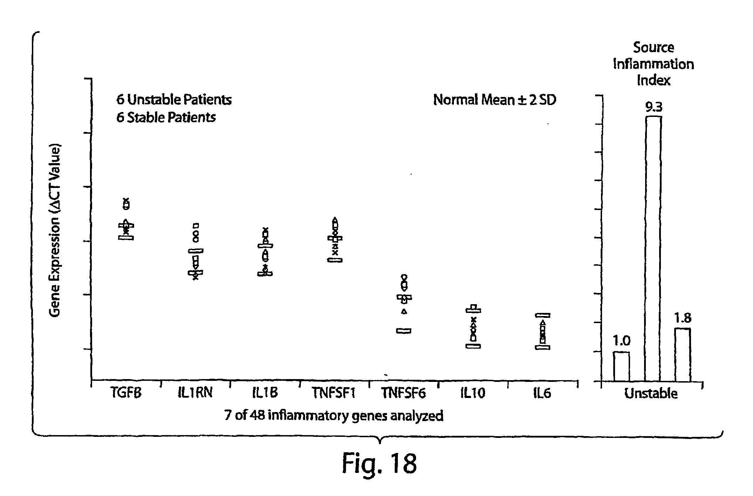

Fig. 18 plots, in a fashion similar to that of Fig. 17A, Gene Expression Profiles, for the same 7 loci as in Fig. 17A, two different 6-subject populations of rheumatoid arthritis patients. One population (called "stable" in the figure) is of patients who have responded well to treatment and the other population (called "unstable" in the figure) is of patients who have not responded well to treatment and whose therapy is scheduled for change. It can be seen that the expression values for the stable patient population, lie within the range of the 95% confidence interval, whereas the expression values for the unstable patient population for 5 of the 7 loci are outside and above this range. The right-hand portion of the figure shows an average inflammation index of 9.3 for the unstable population and an average inflammation index of 1.8 for the stable population, compared to 1 for a normal undiagnosed population of patients. The index thus provides a measure of the extent of the underlying inflammatory condition, in this case, rheumatoid arthritis. Hence the index, besides providing a measure of biological condition, can be used to measure the effectiveness of therapy as well as to provide a target for therapeutic intervention.

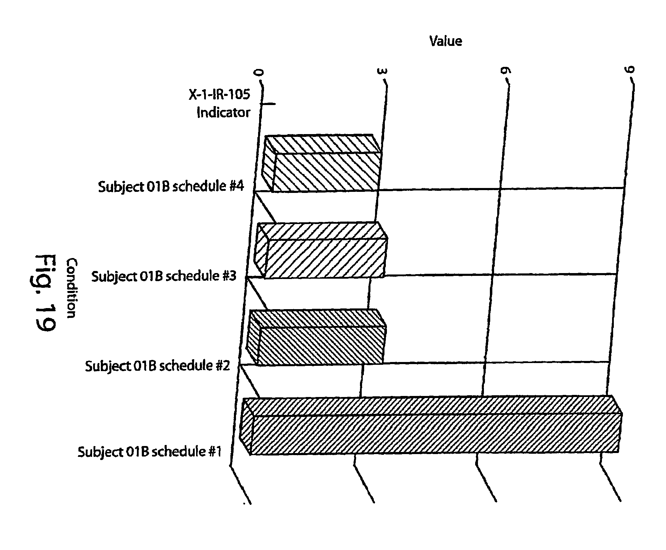

Fig. 19 thus illustrates use of the inflammation index for assessment of a single subject suffering from rheumatoid arthritis, who has not responded well to traditional therapy with methotrexate. The inflammation index for this subject is shown on the far right at start of a new therapy (a TNF inhibitor), and then, moving leftward, successively, 2 weeks, 6 weeks, and 12 weeks thereafter. The index can be seen moving towards normal, consistent with physician observation of the patient as responding to the new treatment.

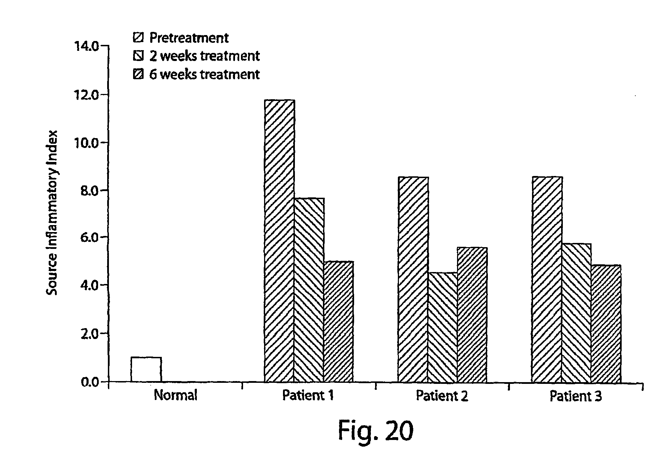

Fig. 20 similarly illustrates use of the inflammation index for assessment of three subjects suffering from rheumatoid arthritis, who have not responded well to traditional therapy with methotrexate, at the beginning of new treatment (also with a TNF inhibitor), and 2 weeks and 6 weeks thereafter. The index in each case can again be seen moving generally towards normal, consistent with physician observation of the patients as responding to the new treatment.

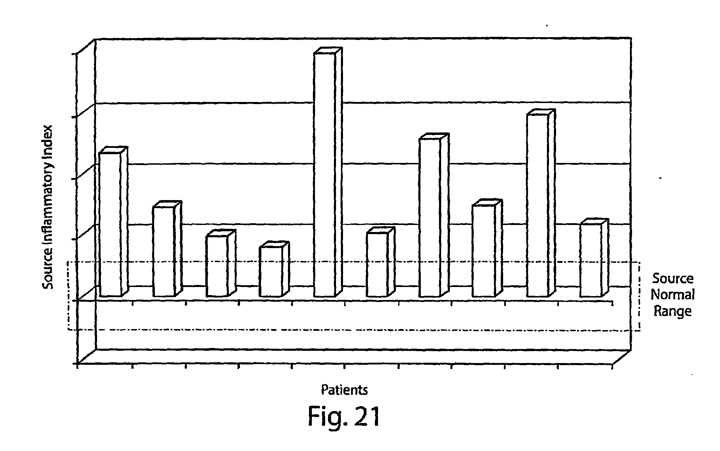

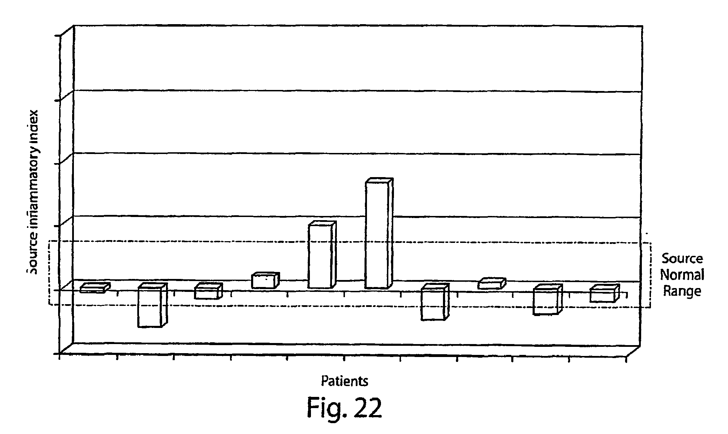

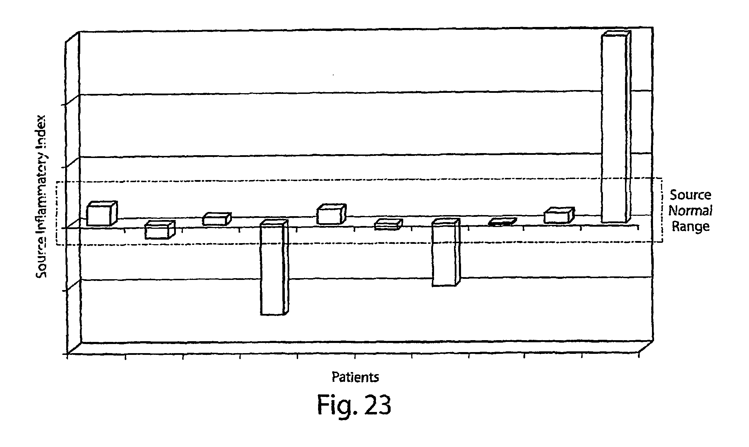

Each of Figs. 21-23 shows the inflammation index for an international group of subjects, suffering from rheumatoid arthritis, each of whom has been characterized as stable (that is, not anticipated to be subjected to a change in therapy) by the subject's treating physician. Fig. 21 shows the index for each of 10 patients in the group being treated with methotrexate, which known to alleviate symptoms without addressing the underlying disease. Fig. 22 shows the index for each of 10 patients in the group being treated with Enbrel (an TNF inhibitor), and Fig. 23 shows the index for each 10 patients being treated with Remicade (another TNF inhibitor). It can be seen that the inflammation index for each of the patients in Fig. 21 is elevated compared to normal, whereas in Fig. 22, the patients being treated with Enbrel as a class have an inflammation index that comes much closer to normal (80% in the normal range). In Fig. 23, it can be seen that, while all but one of the patients being treated with Remicade have an inflammation index at or below normal, two of the patients have an abnormally low inflammation index, suggesting an immunosuppressive response to this drug. (Indeed, studies have shown that Remicade has been associated with serious infections in some subjects, and here the immunosuppressive effect is quantified.) Also in Fig. 23, one subject has an inflammation index that is significantly above the normal range. This subject in fact was also on a regimen of an anti-inflammation steroid (prednisone) that was being tapered; within approximately one week after the inflammation index was sampled, the subject experienced a significant flare of clinical symptoms.

Remarkably, these examples show a measurement, derived from the assay of blood taken from a subject, pertinent to the subject's arthritic condition. Given that the measurement pertains to the extent of inflammation, it can be expected that other inflammation- based conditions, including, for example, cardiovascular disease, may be monitored in a similar fashion.

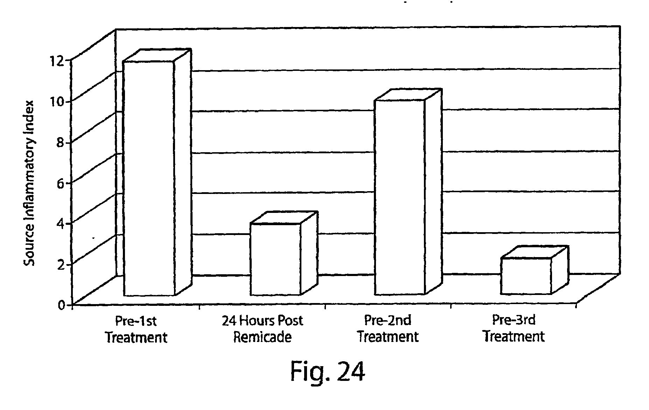

Fig. 24 illustrates use of the inflammation index for assessment of a single subject suffering from inflammatory bowel disease, for whom treatment with Remicade was initiated in three doses. The graphs show the inflammation index just prior to first treatment, and then 24 hours after the first treatment; the index has returned to the normal range. The index was elevated just prior to the second dose, but in the normal range prior to the third dose. Again, the index, besides providing a measure of biological condition, is here used to measure the effectiveness of therapy (Remicade), as well as to provide a target for therapeutic intervention in terms of both dose and schedule.

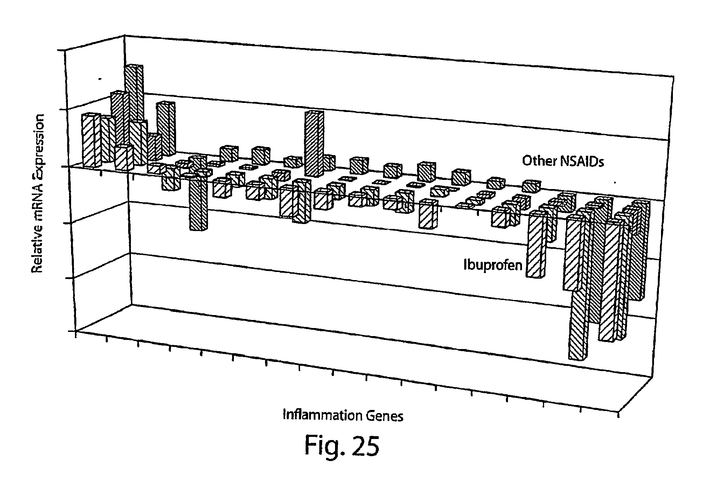

Fig. 25 shows Gene Expression Profiles with respect to 24 loci (of the Inflammation Gene Expression Panel) for whole blood treated with Ibuprofen in vitro in relation to other non-steroidal anti-inflammatory drugs (NSAIDs). The profile for Ibuprofen is in front. It can be seen that all of the NSAIDs, including Ibuprofen share a substantially similar profile, in that the patterns of gene expression across the loci are similar. Notwithstanding these similarities, each individual drug has its own distinctive signature.

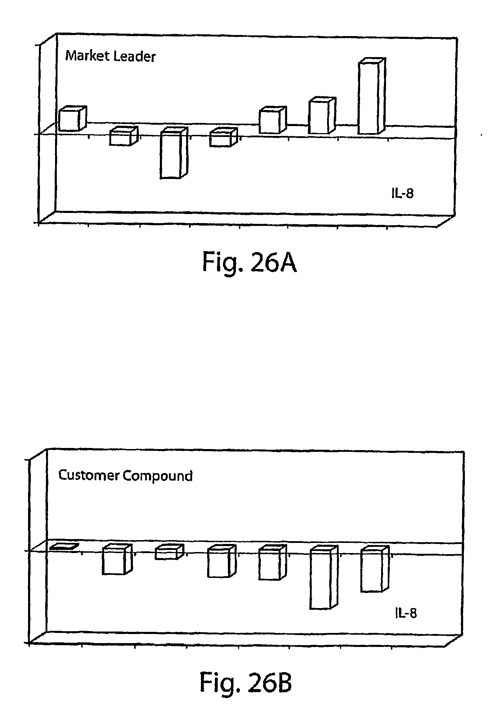

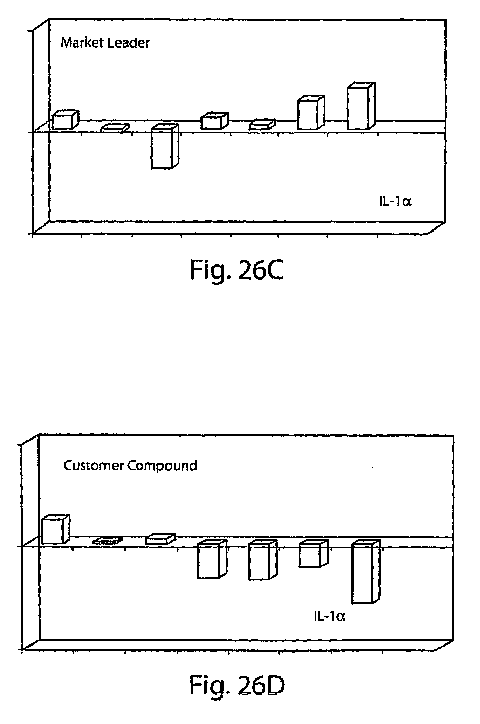

Fig. 26 illustrates how the effects of two competing anti-inflammatory compounds can be compared objectively, quantitatively, precisely, and reproducibly. In this example, expression of each of a panel of two genes (of the Inflammation Gene Expression Panel) is measured for varying doses (0.08 - 250 $\mu$g/ml) of each drug in vitro in whole blood. The market leader drug shows a complex relationship between dose and inflammatory gene response. Paradoxically, as the dose is increased, gene expression for both loci initially drops and then increases in the case the case of the market leader. For the other compound, a more consistent response results, so that as the dose is increased, the gene expression for both loci decreases more consistently.

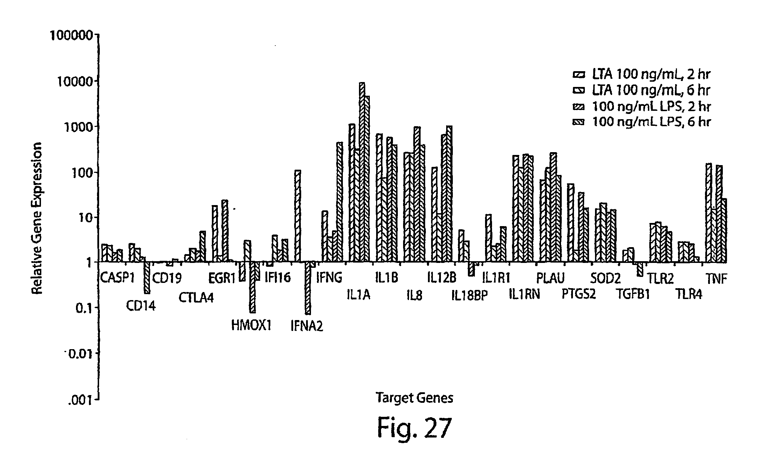

Figs. 27 through 41 illustrate the use of gene expression panels in early identification and monitoring of infectious disease. These figures plot the response, in expression products of the genes indicated, in whole blood, to the administration of various infectious agents or products associated with infectious agents. In each figure, the gene expression levels are "calibrated", as that term is defined herein, in relation to baseline expression levels determined with respect to the whole blood prior to administration of the relevant infectious agent. In this respect the figures are similar in nature to various figures of our below-referenced patent application WO 01/25473 (for example, Fig. 15 therein). The concentration change is shown ratiometrically, and the baseline level of 1 for a particular gene locus corresponds to an expression level for such locus that is the same, monitored at the relevant time after addition of the infectious agent or other stimulus, as the expression level before addition of the stimulus. Ratiometric changes in concentration are plotted on a logarithmic scale. Bars below the unity line represent decreases in concentration and bars above the unity line represent increases in concentration, the magnitude of each bar indicating the magnitude of the ratio of the change. We have shown in WO 01/25473 and other experiments that, under appropriate conditions, Gene Expression Profiles derived in vitro by exposing whole blood to a stimulus can be representative of Gene Expression Profiles derived in vivo with exposure to a corresponding stimulus.

Fig. 27 uses a novel bacterial Gene Expression Panel of 24 genes, developed to discriminate various bacterial conditions in a host biological system. Two different stimuli are employed: lipotechoic acid (LTA), a gram positive cell wall constituent, and lipopolysaccharide (LPS), a gram negative cell wall constituent. The final concentration immediately after administration of the stimulus was 100 ng/mL, and the ratiometric changes in expression, in relation to pre-administration levels, were monitored for each stimulus 2 and 6 hours after administration. It can be seen that differential expression can be observed as early as two hours after administration, for example, in the IFNA2 locus, as well as others, permitting discrimination in response between gram positive and gram negative bacteria.

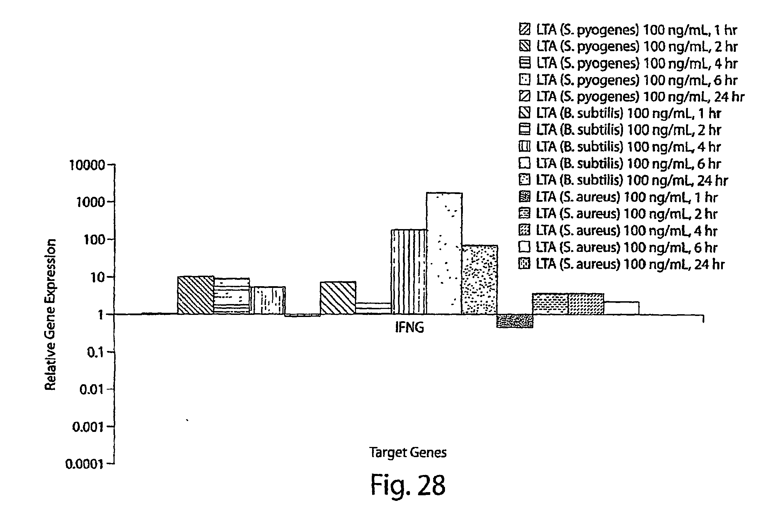

Fig. 28 shows differential expression for a single locus, IFNG, to LTA derived from three distinct sources: S. pyrogenes, B. subtilis, and S. aureus. Each stimulus was administered to achieve a concentration of 100 ng/mL, and the response was monitored at 1, 2, 4, 6, and 24 hours after administration. The results suggest that Gene Expression Profiles can be used to distinguish among different infectious agents, here different species of gram positive bacteria.

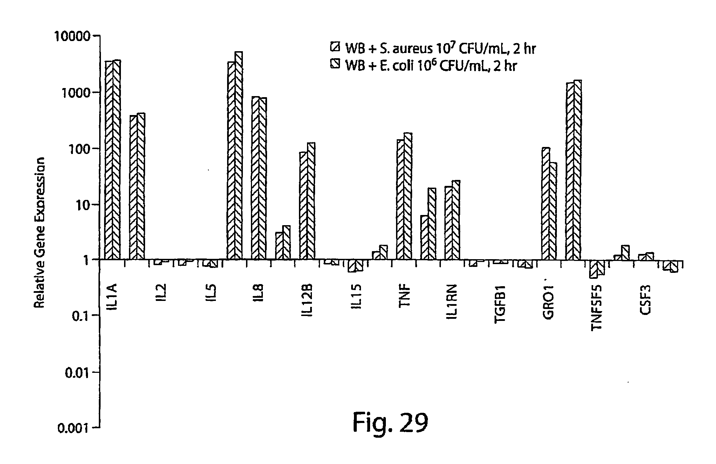

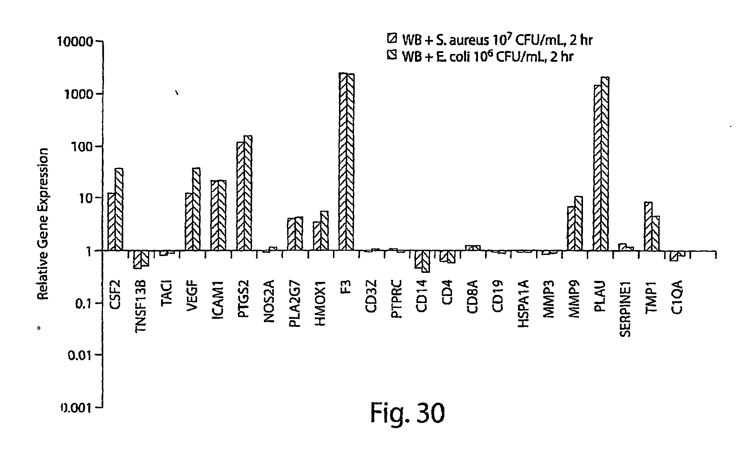

Figs. 29 and 30 show the response of the Inflammation 48A and 48B loci respectively (discussed above in connection with Figs. 6 and 7 respectively) in whole blood to administration of a stimulus of S. aureus and of a stimulus of E. coli (in the indicated concentrations, just after administration, of $10^7$ and $10^6$ CFU/mL respectively), monitored 2 hours after administration in relation to the pre-administration baseline. The figures show that many of the loci respond to the presence of the bacterial infection within two hours after infection.

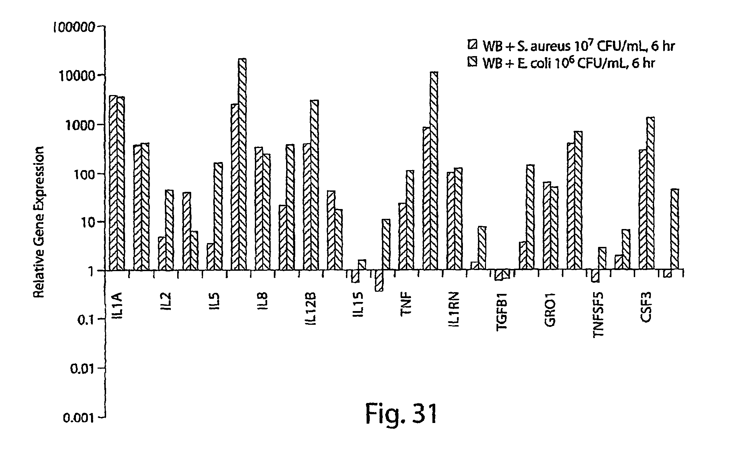

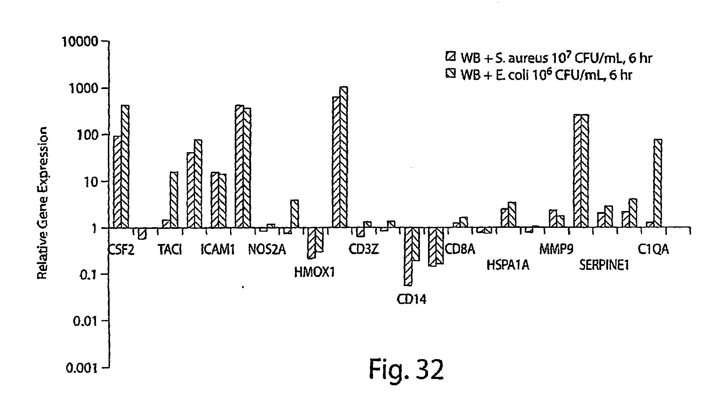

Figs. 31 and 32 correspond to Figs. 29 and 30 respectively and are similar to them, with the exception that the monitoring here occurs 6 hours after administration. More of the loci are responsive to the presence of infection. Various loci, such as IL2, show expression levels that discriminate between the two infectious agents.

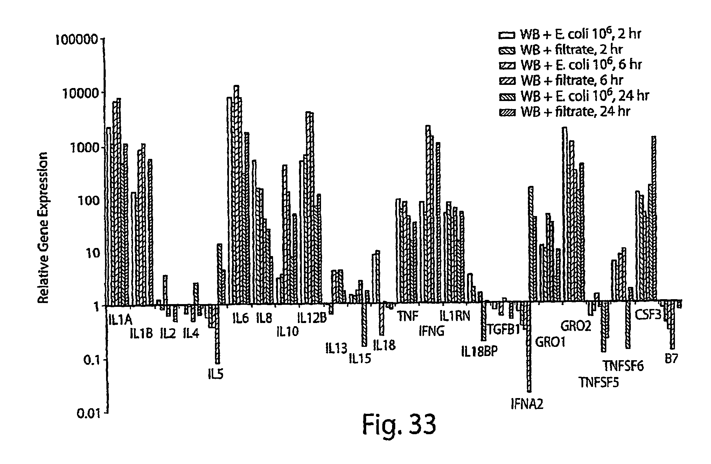

Fig. 33 shows the response of the Inflammation 48A loci to the administration of a stimulus of *E. coli* (again in the concentration just after administration of $10^6$ CFU/mL) and to the administration of a stimulus of an *E. coli* filtrate containing *E. coli* bacteria by products but lacking *E. coli* bacteria. The responses were monitored at 2, 6, and 24 hours after administration. It can be seen, for example, that the responses over time of loci IL1B, IL18 and CSF3 to *E. coli* and to *E. coli* filtrate are different.

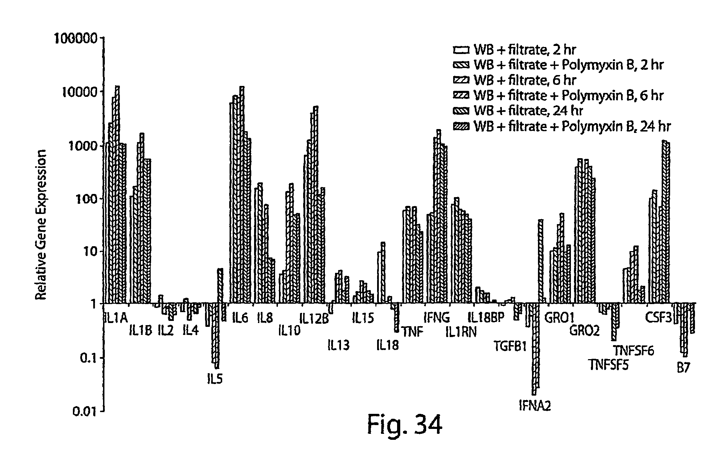

Fig. 34 is similar to Fig. 33, but here the compared responses are to stimuli from *E. coli* filtrate alone and from *E. coli* filtrate to which has been added polymyxin B, an antibiotic known to bind to lipopolysaccharide (LPS). An examination of the response of IL1B, for example, shows that presence of polymyxin B did not affect the response of the locus to *E. coli* filtrate, thereby indicating that LPS does not appear to be a factor in the response of IL1B to *E. coli* filtrate.

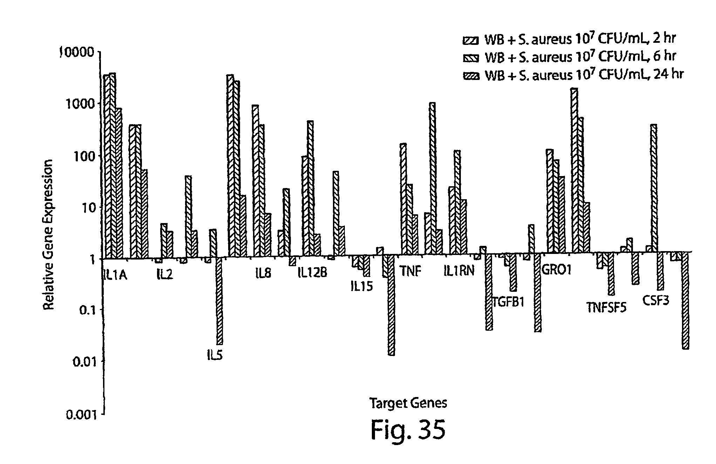

Fig. 35 illustrates the responses of the Inflammation 48A loci over time of whole blood to a stimulus of *S. aureus* (with a concentration just after administration of $10^7$ CFU/mL) monitored at 2, 6, and 24 hours after administration. It can be seen that response over time can involve both direction and magnitude of change in expression. (See for example, IL5 and IL18.)

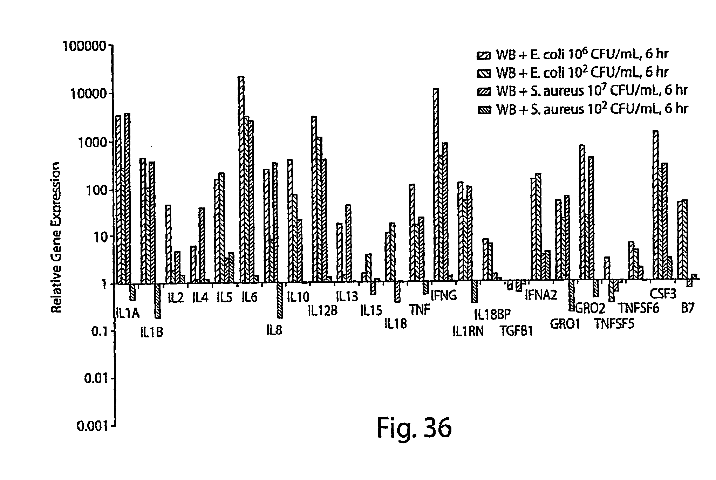

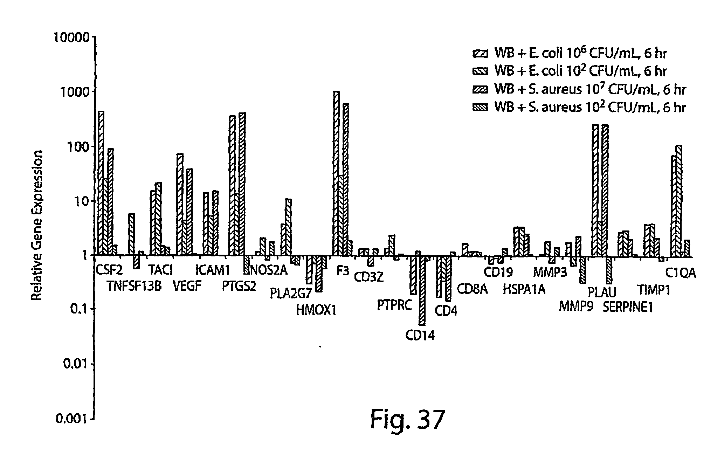

Figs. 36 and 37 show the responses, of the Inflammation 48A and 48B loci respectively, monitored at 6 hours to stimuli from *E. coli* (at concentrations of $10^6$ and $10^2$ CFU/mL immediately after administration) and from *S. aureus* (at concentrations of $10^7$ and $10^2$ CFU/mL immediately after administration). It can be seen, among other things, that in various loci, such as B7 (Fig. 36), TACI, PLA2G7, and C1QA (Fig. 37), *E. coli* produces a much more pronounced response than *S. aureus.* The data suggest strongly that Gene Expression Profiles can be used to identify with high sensitivity the presence of gram negative bacteria and to discriminate against gram positive bacteria.

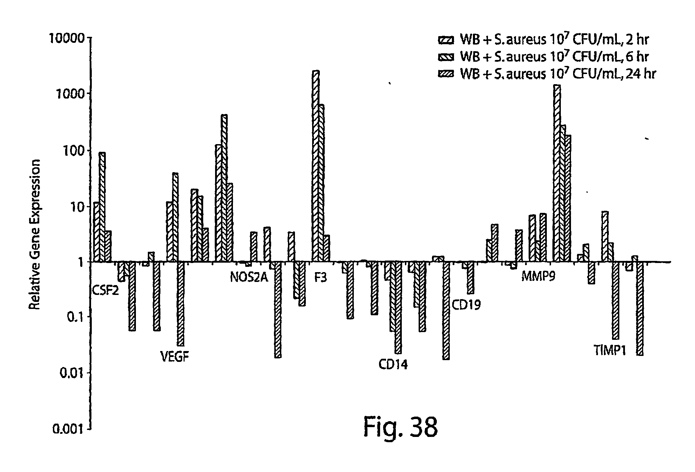

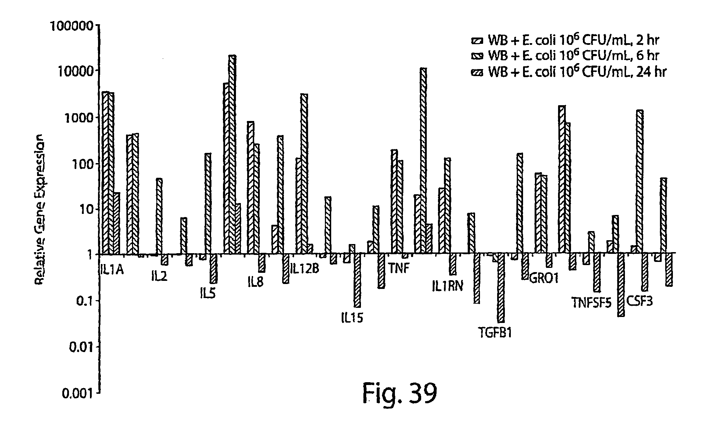

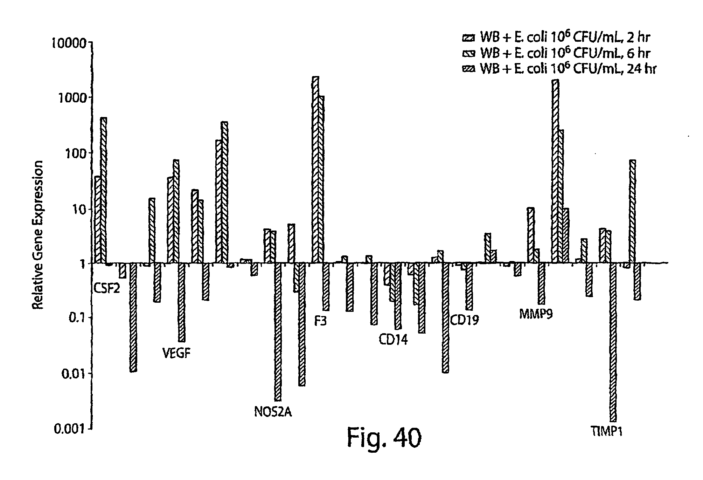

Figs. 38 and 39 show the responses, of the Inflammation 48B and 48A loci respectively, monitored 2, 6, and 24 hours after administration, to stimuli of high concentrations of *S. aureus* and *E. coli* respectively (at respective concentrations of $10^7$ and $10^6$ CFU/mL immediately after administration). The responses over time at many loci involve changes in magnitude and direction. Fig. 40 is similar to Fig. 39, but shows the responses of the Inflammation 48B loci.

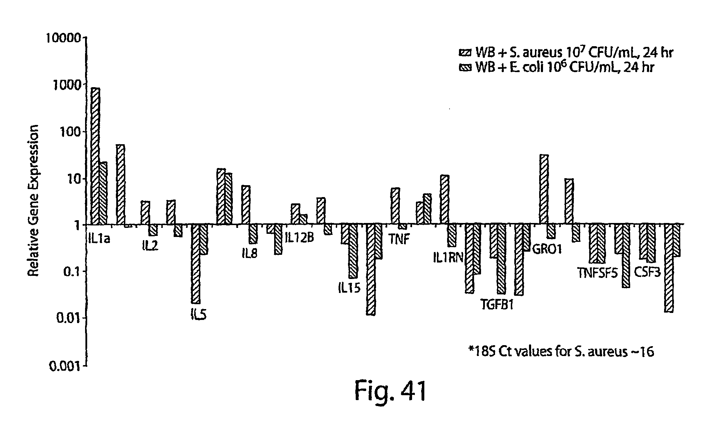

Fig. 41 similarly shows the responses of the Inflammation 48A loci monitored at 24 hours after administration to stimuli high concentrations of *S. aureus* and *E. coli* respectively (at respective concentrations of $10^7$ and $10^6$ CFU/mL immediately after administration). As in the case of Figs. 20 and 21, responses at some loci, such as GRO1 and GRO2, discriminate between type of infection.

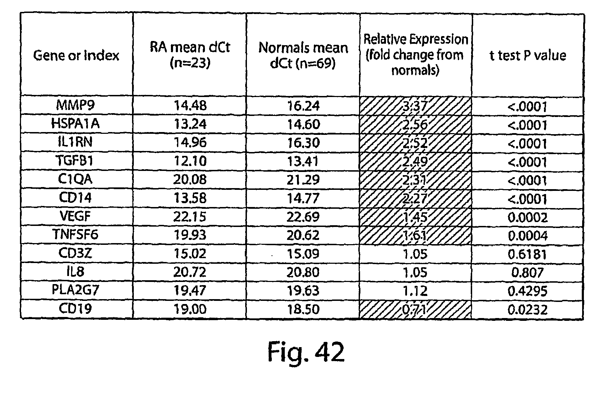

Fig. 42 illustrates application of a statistical T-test to identify potential members of a signature gene expression panel that is capable of distinguishing between normal subjects and subjects suffering from unstable rheumatoid arthritis. The grayed boxes show genes that are individually highly effective (t test P values noted in the box to the right in each case) in distinguishing between the two sets of subjects, and thus indicative of potential members of a signature gene expression panel for rheumatoid arthritis.

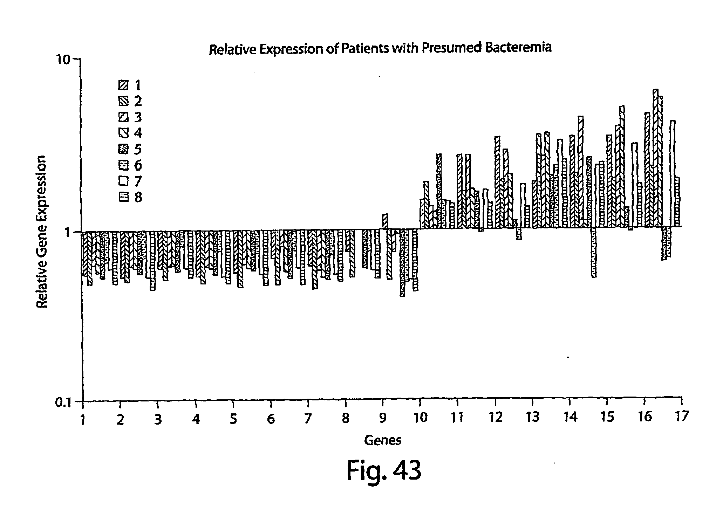

Fig. 43 illustrates, for a panel of 17 genes, the expression levels for 8 patients presumed to have bacteremia. The data are suggestive of the prospect that patients with bacteremia have a characteristic pattern of gene expression.

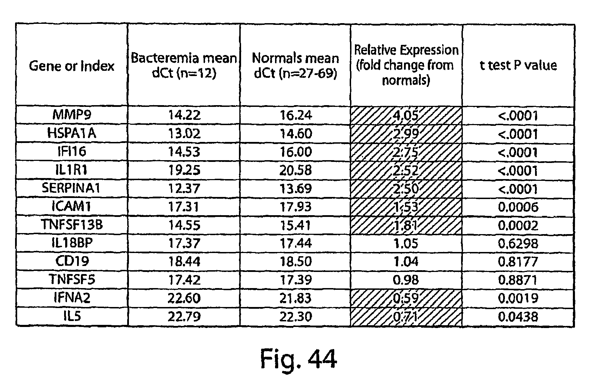

Fig. 44 illustrates application of a statistical T-test to identify potential members of a signature gene expression panel that is capable of distinguishing between normal subjects and subjects suffering from bacteremia. The grayed boxes show genes that are individually highly effective (t test P values noted in the box to the right in each case) in distinguishing between the two sets of subjects, and thus indicative of potential members of a signature gene expression panel for bacteremia.

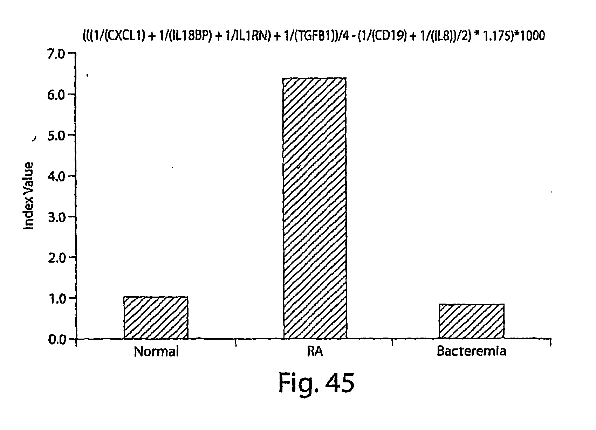

Fig. 45 illustrates application of an algorithm (shown in the figure), providing an index pertinent to rheumatoid arthritis (RA) as applied respectively to normal subjects, RA patients, and bacteremia patients. The index easily distinguishes RA subjects from both normal subjects and bacteremia subjects.

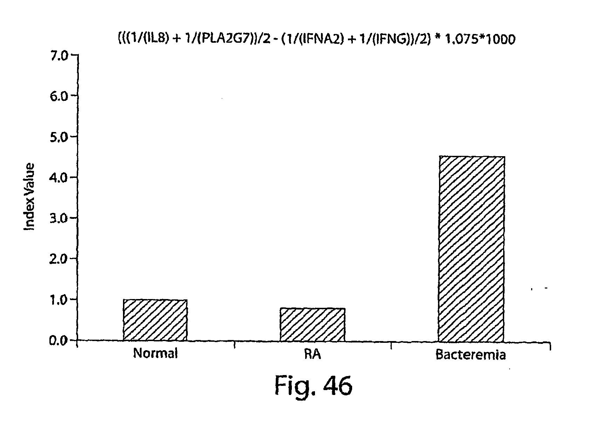

Fig. 46 illustrates application of an algorithm (shown in the figure), providing an index pertinent to bacteremia as applied respectively to normal subjects, rheumatoid arthritis patients, and bacteremia patients. The index easily distinguishes bacteremia subjects from both normal subjects and rheumatoid arthritis subjects.

Example 7 High Precision Gene Expression Analysis of an Individual with RRMS

A female subject with a long, documented history of relapsing, remitting multiple sclerosis (RRMS) sought medical attention from a neurologist for increasing lower trunk muscle weakness (Visit 1, May 22, 2002). Blood was drawn for several assays and the subject was given 5 mg prednisone at that visit. Increasing weakness and spreading of the involvement caused subject to return to the neurologist 6 days later. Blood was drawn and the subject was started on 100 mg prednisone and tapered to 5 mg over one week. The subject reported that her muscle weakness subsided rapidly. The subject was seen for a routine visit (visit 3) more than 2 months later (July 15, 2002). The patient reported no signs of illness at that visit.

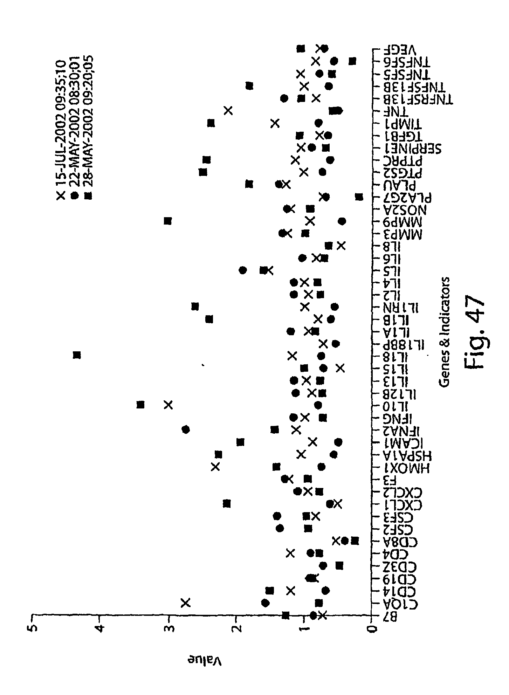

Results of high precision gene expression analysis are shown below in Fig. 47. The "y" axis reports the gene expression level in standard deviation units compared to the Source Precision Medicine Normal Reference Population Value for that gene locus at dates May 22, 2002 (before prednisone treatment), May 28, 2002 (after 5 mg treatment on May 22) and July 15, 2002 (after 100 mg prednisone treatment on May 28, tapering to 5 mg within one week). Expression Results for several genes from the 73 gene locus Multiple Sclerosis Precision Profile (selected from genes in Table 3) are shown along the "x" axis. Some gene loci, for example IL18; IL1B; MMP9; PTGS2, reflect the severity of the signs while other loci, for example IL10, show effects induced by the steroid treatment. Other loci reflect the non-relapsing TIMP1; TNF; HMOX1.

Example 8 Experimental Design for Identification and Selection of Diagnostic and Prognostic Markers for Evaluating Multiple Sclerosis (before, during, and after flare),

Samples of whole blood from patients with relapsing remitting multiple sclerosis (RRMS) were collected while their disease is clinically inactive. Additional samples were collected during a clinical exacerbation of the MS (or attack). Levels of gene expression of mediators of inflammatory processes are examined before, during, and after the episode, whether or not anti-inflammatory treatment is employed. The post-attack samples were then compared to samples obtained at baseline and those obtained during the exacerbation, prior to initiation of any anti-inflammatory medication. The results of this study were compared to a database of normal subjects to identify and select diagnostic and prognostic markers of MS activity useful in the Gene Expression Panels for characterizing and evaluating MS according to the invention. Selected markers were tested in additional trials in patients know to have MS, and those suspected of having MS. By using genes selected to be especially probative in characterizing MS and inflammation related to MS, such conditions are identified in patients using the herein-described gene expression profile techniques and methods of characterizing multiple sclerosis or inflammatory conditions related to multiple sclerosis in a subject based on a sample from the subject. These data demonstrate the ability to evaluate, diagnose and characterize MS and inflammatory conditions related to MS in a subject, or population of subjects.

In this system, RRMS subjects experiencing a clinical exacerbation showed altered inflammatory-immune response gene expression compared to RRMS patients during remission and healthy subjects. Additionally, gene expression changes are evident in patients who have exacerbations coincident with initiation and completion of treatment.

This system thus provides a gene expression assay system for monitoring MS patients that is predictive of disease progression and treatment responsiveness. In using this system, gene expression profile data sets were determined and prepared from inflammation and immune-response related genes (mRNA and protein) in whole blood samples taken from RRMS patients before, during and after clinical exacerbation. Samples taken during an exacerbation were collected prior to treatment for the attack. Gene expression results were then correlated with relevant clinical indices as described.

In addition, the observed data in the gene expression profile data sets was compared to reference profile data sets determined from samples from undiagnosed healthy subjects (normals), gene expression profiles for other chronic immune-related genes, and to profile data sets determined for the individual patients during and after the attack. If desired, a subset of the selected identified genes is coupled with appropriate predictive biomedical algorithms for use in predicting and monitoring RRMS disease activity.

A study was conducted with 14 patients. Patients were required to have an existing diagnosis of RRMS and be clinically stable for at least thirty days prior to enrollment. Some patients were using disease-modifying medication (Interferon or Glatirimer Acetate). All patients are sampled at baseline, defined as a time when the subject is not currently experiencing an attack (see inclusion criteria). Those who experience significant neurological symptoms, suggestive of a clinical exacerbation, were sampled prior to any treatment for the attack. If the patient was found to have a clinical exacerbation, then a repeat sample is obtained four weeks later, regardless of whether the patient receives steroids or other treatment for the exacerbation.

A clinical exacerbation is defined as the appearance of a new symptom or worsening/reoccurrence of an old symptom, attributed to RRMS, lasting at least 24 hours in the absence of fever, and preceded by stability or improvement for at least 30 days.

Each subject was asked to provide a complete medical history including any existing laboratory test results (i.e. MRI, EDSS scores, blood chemistry, hematology, etc) relevant to the patient's MS contained within the patient's medical records. Additional test results (ordered while the subject is enrolled in the study) relating to the treatment of the patient's MS were collected and correlated with gene expression analysis.

Subjects in the study meet all of the following criteria:

1. Male or Female subjects at least 18 years old with clinically documented active Relapsing-Remitting MS (RRMS) characterized by clearly defined acute attacks followed by full or partial recovery to the pre-existing level of disability, and by a lack of disease progression in the periods between attacks.
2. Subjects are clinically stable for a minimum of 30 days or for a time period determined at the clinician's discretion.
3. Patients are stable (at least three-months) on Interferon therapy or Glatiramer Acetate or are therapy naïve or without the above mentioned therapy for 4 weeks.
4. Subjects must be willing to give written informed consent and to comply with the requirements of the study protocol.

Subjects are excluded from the study if they meet any of the following criteria:

1. Primary progressive multiple sclerosis (PPMS).
2. Immunosuppressive therapy (such as azathioprine and MTX) within three months of study participation. Subjects having prior treatment with cyclophosphamide, total lymphoid irradiation, mitoxantrone, cladribine, or bone marrow transplantation, regardless of duration, are also excluded.
3. Corticosteroid therapy within four weeks of participation of the study.
4. Use of any investigational drug with the intent to treat MS or the symptoms of MS within six months of participation in this trial (agents for the symptomatic treatment of MS, e.g., 4-aminopyridine <4-AP>, may be allowed following discussion with Clinician).
5. Infection or risk factors for severe infections, including: excessive immunosuppression including human immunodeficiency virus (HIV) infection; severe, recurrent, or persistent infections (such as Hepatitis B or C, recurrent urinary tract infection or pneumonia); evidence of current inactive or active tuberculosis (TB) infection including recent exposure to *M. tuberculosis* (converters to a positive purified protein derivative); subjects with a positive PPD or a chest X-ray suggestive of prior TB infection; active Lyme disease; active syphilis; any significant infection requiring hospitalization or IV antibiotics in the month prior to study participation; infection requiring treatment with antibiotics in the two weeks prior to study participation.
6. Any of the following risk factors for development of malignancy: history of lymphoma or leukemia; treatment of cutaneous squamous-cell or basal cell carcinoma within 2 years of enrollment into the study; other malignancy within 5 years; disease associated with an increased risk of malignancy.
7. Other diseases (in addition to MS) that produce neurological manifestations, such as amyotrophic lateral sclerosis, Gullain-Barre syndrome, muscular dystrophy, etc.)
8. Pregnant or lactating females.

Example 9 Experimental Design for Identification and Selection of Diagnostic and Prognostic Markers for Evaluating Multiple Sclerosis (pre and post therapy).

These studies were designed to identify possible markers of disease activity in multiple sclerosis (MS) to aid in selecting genes for particular Gene Expression Panels. Similar to the previously-described example, the results of this study were compared to a database of gene expression profile data sets determined and obtained from samples from healthy subjects, and the results were used to identify possible markers of MS activity to be used in Gene Expression Panels for characterizing and evaluating MS according to described embodiments. Selected markers were then tested in additional trials to assess their predictive value.

Eleven subjects were used in this study. Initially, a smaller number of patients were evaluated, and gene expression profile data sets were determined for these patients and the expression profiles of selected inflammatory markers were assessed. Additional subjects were added to the study after preliminary evidence for particular disease activity markers is obtained so that a larger or more particular panel of genes is selected for determining profile data sets for the full number of subjects in the study.

Patients who were not receiving disease-modifying therapy such as interferon are of particular interest but inclusion of patients receiving such therapy was also useful. Patients were asked to give blood at two timepoints - first at enrollment and then again at 3-12 months after enrollment. Clinical data relating to present and history of disease activity, concomitant medications, lab and MRI results, as well as general health assessment questionnaires were also collected.

Patients meeting the following specific criteria are desirable for the study:

1. Patients having MS that meets the criteria of McDonald et al. Ann Neurol. 2001 Jul; 50 (1) : 121- 7.
2. Patients with clinically active disease as shown by ≥ 1 exacerbation in previous 12 months.
3. Patients not in acute relapse
4. Patients willing to provide up to 10 ml of blood at up to 3 time points

In addition, patients with know hepatitis or HIV infection were not eligible. The enrollment samples from suitable subjects were collected prior to the patient receiving any disease modifying therapy. The later samples are collected 3-12 months after the patients start therapy. Preliminary data suggests that gene expression can used to track drug response and that only a plurality or several genetic markers is required to identify MS in a population of samples.

Example 10 Experimental Design for Identification and Selection of Diagnostic and Prognostic Markers for Evaluating Multiple Sclerosis (Dosing, Safety and Response),

Theses studies were designed to identify biomarkers for use in a specific Gene Expression Panel for MS, wherein the genes/biomarkers are selected to evaluate dosing and safety of a new compound developed for treating MS, and to track drug response. Specifically a multi-center, randomized, double blind, placebo-controlled trial was used evaluate a new drug therapy in patients with multiple sclerosis.

Thirty subjects were enrolled in this study. Only patients who exhibit stable MS for three months prior to the study were selected for the trial. Stable disease is defined as the absence of progression and relapse. Subjects enrolled in this study had been removed from disease modifying therapy for at least 1 month. A subject's clinical status was monitored throughout the study by MRI and hematology and blood chemistries.

Throughout the study patients received all medications necessary for management of their MS, including high-dose corticosteroids for management of relapses and introduction of standard treatments for MS. Initiation of such treatments will confound assessment of the trial's endpoints. Consequently, patients who require such treatment were removed from the new drug therapy phase of the trial but will continue to be followed for safety, immune response, and gene expression.

Blood samples for gene expression analysis were collected at screening/baseline (prior to initiation of drug), several times during the treatment phase and several times during follow-up (post-treatment phase). Gene expression results werecompared within subjects, between subjects, and to Source Precision Medicine profile data sets determined to be what are termed "Normals" - i.e., a baseline profile dataset determined for a population of healthy (undiagnosed) individuals who do not have MS or other inflammatory conditions, disease, infections. The results were also evaluated to compare and contrast gene expression between different timepoints. This study was used to track individual and population response to the drug, and to correlate clinical symptoms (i.e. disease progression, disease remittance, adverse events) with gene expression.

Baseline samples from a subset of patients were analyzed. The preliminary data from the baseline samples suggest that that only a plurality of or optionally several specific genetic markers is required to identify MS across a population of samples. The study was also used to track drug response and clinical endpoints.

Example 11 Experimental Design for Identification and Selection of Diagnostic and Prognostic Markers for Evaluating Multiple Sclerosis (Testing treatment),

Theses studies were designed a study for testing a new experimental treatment for MS. The study enrolled 200 MS subjects in a Phase 2, multi-center, randomized, double-blind, parallel group, placebo-controlled, dose finding, safety, tolerability, and efficacy study. Samples for gene expression were collected at baseline and at several timepoints during the study. Samples were compared between subjects, within individual subjects, and to Source Precision Medicine profile data sets determined to be what are termed "Normals" - i.e., a baseline profile dataset determined for a population of healthy (undiagnosed) individuals who do not have MS or other inflammatory conditions, disease, infections. The gene expression profile data sets were then assessed for their ability to track individual response to therapy, for identifying a subset of genes that exhibit altered gene expression in MS and/or are affected by the drug treatment. Clinical data collected during the study include: MRIs, disease progression tests (EDSS, MSFC, ambulation tests, auditory testing, dexterity testing), medical history, concomitant medications, adverse events, physical exam, hematology and chemistry labs, urinalysis, and immunologic testing.

Subj ects enrolled in the study were asked to discontinue any MS disease modifying therapies they may be using for their disease for at least 3 months prior to dosing with the study drug or drugs.

Example 12 - Clinical Data analyzed with Latent Class Modeling

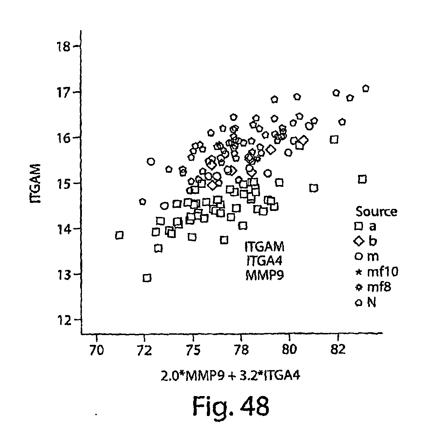

Figures 48 through 53 show various analyses of data performed using latent class modeling. From a targeted 96-gene panel, selected to be informative relative to biological state of MS patients, primers and probes were prepared for a subset of 54 genes (those with p-values of 0.05 or better) or 72 genes. Gene expression profiles were obtained using these subsets of genes, and of these individual genes, ITGAM was found to be uniquely and exquisitely informative regarding MS, yielding the best discrimination from normals of the genes examined.

In order, ranked by increasing p-values, with higher values indicating less discrimination from normals, the following genes were determined to be especially useful in discriminating MS subjects from normals (listed below from more discriminating to less discriminating).

|  | Normals vs. all MS sets | | Normals vs. 3-month washed out MS |
|---|---|---|---|
|  | p-value | | p-value |
| ITGAM | 8.4E-21 | ITGAM | 2.7E-27 |
| NFKB1 | 1.1E-18 | NFKB1 | 2.9E-18 |
| NFKBIB | 1.4E-17 | CASP9 | 3.8E-18 |
| CASP9 | 2.6E-15 | IRF5 | 3.0E-17 |
| IRF5 | 3.0E-15 | NFKBIB | 2.1E-16 |

A ranking of the top 54 genes is shown below, listed from more discriminating to less discriminating, by p-value.

**Table 1** Ranking of Genes, by P-Value, From More Discriminating to Less Discriminating

| # | Gene Symbol | p-value (MS v. N) | p-value (Washed-out v. N) | # | Gene Symbol | p-value (MS v. N) | p-value (Washed-out v. N) |
|---|---|---|---|---|---|---|---|
| 1 | ITGAM | 8.40E-21 | 2.70E-27 | 28 | IL13 | 4.60E-05 | 1.50E-06 |
| 2 | NFKB | 1.10E-18 | 2,90E-18 | 29 | ARG2 | 5.10E-05 | 7.10E-06 |
| 3 | NFKBIB | 1.40E-17 | 2.10E-16 | 30 | CCR5 | 5.80E-05 | 6.90E-05 |
| 4 | CASP9 | 2.60E-15 | 3.80E-18 | 31 | APAF1 | 7.60E-05 | 0.00016 |
| 5 | IRF5 | 3.00E-15 | 3.00E-17 | 32 | SERPINE1 | 8.30E-05 | 0.0001 |
| 6 | IL18R1 | 2.70E-12 | 1.50E-14 | 33 | MMP3 | 9.90E-05 | 4.30E-5 |
| 7 | TGFBR2 | 7.70E-12 | 1.30E-12 | 34 | PLA2G7 | 0.00014 | 0.00043 |
| 8 | NOS3 | 1.60E-10 | 1.50E-13 | 35 | NOS1 | 0.00015 | 0.00041 |
| 9 | IL1RN | 2.00E-10 | 1.00E-07 | 36 | FCGR1A | 0.00021 | 0.00041 |
| 10 | TLR2 | 5.70E-10 | 3.00E-08 | 37 | PF4 | 0.00032 | 2.70E-05 |
| 11 | CXCR3 | 1.60E-09 | 2.00E-09 | 38 | ICAM1 | 0.00056 | 0.0016 |
| 12 | FTL | 2.00E-09 | 4.00E-09 | 39 | PTX3 | 0.00071 | 0.0014 |
| 13 | CCR1 | 3.60E-09 | 9.60E-07 | 40 | MMP9 | 0.00073 | 0.0012 |
| 14 | TNFSF13B | 1.30E-08 | 2.90E-05 | 41 | LBP | 0.0011 | 6.60E-05 |
| 15 | TLR4 | 9.80E-08 | 2.10E-06 | 42 | MBL2 | 0.0014 | 0.00068 |
| 16 | LTA | 2.20E-07 | 3.10E-10 | 43 | CCL3 | 0.0039 | 0.011 |
| 17 | BCL2 | 2.50E-07 | 3.90E-08 | 44 | CXCL10 | 0.0043 | 1.00E-05 |
| 18 | TREM1 | 6.20E-07 | 1.80E-05 | 45 | PTGS2 | 0.0053 | 0.0025 |
| 19 | HMOX1 | 9.00E-07 | 2.40E-06 | 46 | CD8A | 0.0068 | 0.007 |
| 20 | CALCA | 1.00E-06 | 8.00E-05 | 47 | SFTPD | 0.0094 | 0.0089 |

(continued)

| # | Gene Symbol | p-value (MS v. N) | p-value (Washed-out v. N) | # | Gene Symbol | p-value (MS v. N) | p-value (Washed-out v. N) |
|---|---|---|---|---|---|---|---|
| 21 | PLAU | 1.00E-06 | 4.30E-07 | 48 | F3 | 0.015 | 0.0016 |
| 22 | TIMP1 | 1.10E-06 | 1.00E-06 | 49 | CD4 | 0.018 | 0.0041 |
| 23 | MIF | 1.50E-06 | 1.30E-10 | 50 | CCL2 | 0.025 | 0.36 |
| 24 | PI3 | 8.40E-06 | 2.00E-09 | 51 | IL6 | 0.027 | 0.05 |
| 25 | IL1B | 5.50E-06 | 5.50E-06 | 52 | SPP1 | 0.029 | 0.012 |
| 26 | DTR | 1.50E-05 | 0.00011 | 53 | IL12B | 0.03 | 0.011 |
| 27 | CCL5 | 2.30E-05 | 6.90E-05 | 54 | CASP1 | 0.045 | 0.26 |

**Table 2** Remaining Genes Making up the 72-gene Panel

| # | Gene Symbol | p-value (MS v. N) | p-value (Washed-out v. N) | # | Gene Symbol | p-value (MS v. N) | p-value (Washed-out v.N) |
|---|---|---|---|---|---|---|---|
| 55 | TNFSF6 | 0.06 | 0.1 | 64 | IL8 | 0.21 | 0.3 |
| 56 | ITGA4 | 0.08 | 0.23 | 65 | VEGF | 0.39 | 0.2 |
| 57 | TNFSF5 | 0.085 | 0.23 | 66 | CASP3 | 0.41 | 0.5 |
| 58 | JUN | 0.089 | 0.033 | 67 | IL10 | 0.43 | 0.37 |
| 59 | CCR3 | 0.12 | 0.019 | 68 | CSF2 | 0.48 | 0.68 |
| 60 | CD86 | 0.12 | 0.62 | 69 | CD19 | 0.56 | 0.94 |
| 61 | IFNG | 0.15 | 0.2 | 70 | IL4 | 0.79 | 0.66 |
| 62 | IL1A | 0.15 | 0.057 | 71 | CCL4 | 0.92 | 0.83 |
| 63 | IL2 | 0.19 | 0.21 | 72 | IL15 | 0.94 | 0.81 |

As shown above, ITGAM was shown to be most discriminating for MS, have the lowest p-value of all genes examined. Latent Class Modeling was then performed with several other genes in combination with ITGAM, to produce three-gene models, four-gene models, and 5-gene models for characterizing MS relative to normals data for a variety of MS subjects. These results are shown in Figures 48 through 53, discussed below.

Fig. 48 shows a three-gene model generated with Latent Class Modeling using ITGAM in combination with MMP9 and ITGA4. In this study, four different groups of MS subjects were compared to normals data for a subset of 72 genes of the 104-gene panel in Table 3. The question asked was, using only ITGAM combined with two other genes, in this case MMP9 and ITGA4, is it possible to discriminate MS subjects from normal subjects (those with no history or diagnosis of MS) The groups of MS patients included "washed-out" subjects, i.e. those diagnosed with MS but off any treatment for three months or longer, and who are represented by Xs and diamonds. Another group of subjects, represented by penagon, are MS subjects who are not washed out from treatment, but rather were on a treatment regimen at the time of this study. The subjects represented by circles are subjects from another clinical study diagnosed with MS and who were also on a treatment regimen at the time of this study. Within this group, two subjects "flared" during the study, and were put on different therapies, and thus moved towards the normal range, as indicated by data taken at that later time and represented in this figures as the star (mf10) and the flower (mf8). Normals data are represented by pentagons. As can be seen in the scatter plot depicted in Figure 48, there is only moderate discrimination with this model between normals and MS subjects, although the discrimination between normals and "washed out" subjects is better.

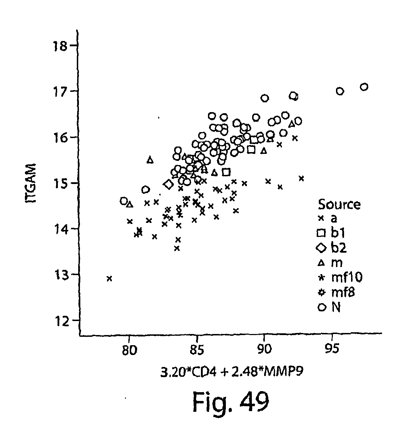

Fig. 49 shows a scatter plot for an alternative three- gene model using ITGAM combined with CD4 and MMP9. The groups of MS patients included "washed out" subjects (Xs), subjects from one clinical study on a treatment regimen (triangle), subjects from another clinical study on a treatment regimen (squares), subjects on an experimental treatment regimen (diamonds), two subjects who flared during the study (mf8 and mf10), and normal subjects (circles) . As can be seen, there is almost complete discrimination with this model between normals and "washed out" subjects. Less discrimination is observed, however, between normals and subjects from the other clinical studies who were being treated at the time these data were generated.

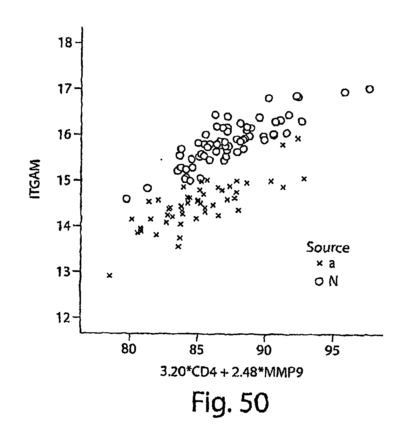

Fig. 50 shows a scatter plot of the same alternative three-gene model of Figure 49 using ITGAM with MMP9 and CD4 but now displaying only washed out subjects relative to normals. As indicated by the straight line, there is almost complete discrimination with this model between normals (circles) and "washed out" (Xs) subjects.

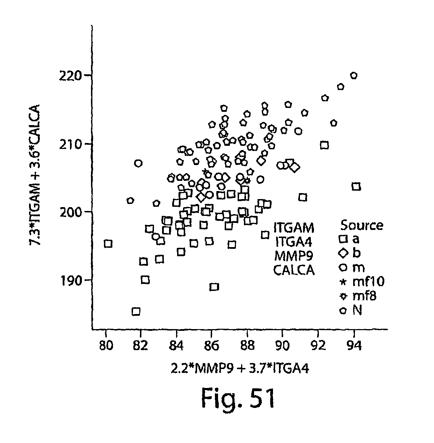

Fig. 51 shows a scatter plot of a four-gene model useful for discriminating all MS subjects, whether washed out, on treatment, or pre-diagnosis. The four-gene model was produced using Latent Class Modeling with ITGAM with ITGA4, MMP9 and CALCA. As can be seen, most MS subjects analyzed (square, diamonds, circles) were quite well-discriminated from normals (pentagon) with this model.

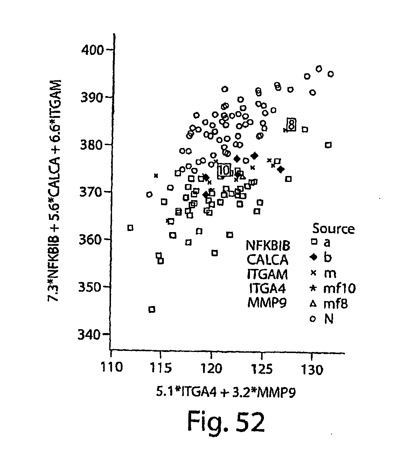

Fig. 52 shows a scatter plot of a five-gene model using ITGAM with ITGA4, NFKB1B, MMP9 and CALCA which further discriminates all MS subjects (square diamonds, Xs) from normals (circles). Note that subjects designated as mf10 and mf8 can be seen to move closer to normal upon treatment during the study from their "flared" state which occurred after enrollment.

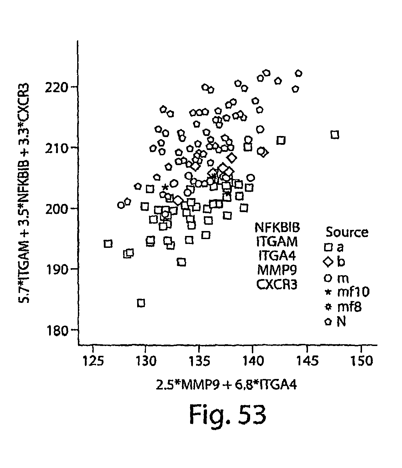

Fig. 53 shows a scatter plot of another five-gene model using ITGAM with ITGA4, NFKB1B, MMP9 and CXCR3 replacing CALCA. Because CALCA is a low expression gene in general, an alternative five-gene model was produced replacing CALCA with CXCR3. Again one can see how the two flared subjects, mf10 and mf8 move closer to normals (star and flower) after treatment. Normals (pentagon)

**Table 3: Stepwise Regression Analysis of Wash-out MS Baseline Subjects (dataset A$_1$A$_2$, n=103) vs Source MDx Normals (dataset N$_1$, n=100)**

| Gene Loci (24) | LogIT p-value | Gene Loci (24) | LogIT p-value | Gene Loci (24) | LogIT p-value | Gene Loci (24) | LogIT p-value |
|---|---|---|---|---|---|---|---|
| | **Step 1** | | **Step 2** | | **Step 3** | | **Step 4** |
| **CASP9** | **3.20E-22** | **HLADRA** | **1.70E-10** | **ITGAL** | **8.60E-07** | **TGFBR2** | **5.20E-04** |
| ITGAM | 2.40E-19 | TGFBR2 | 1.70E-06 | TGFBR2 | 9.10E-07 | IL1 R1 | 0.0025 |
| ITGAL | 5.20E-18 | ITGAL | 0.0018 | BCL2 | 0.0005 | JUN | 0.0084 |
| NFKBIB | 1.20E-16 | JUN | 0.0024 | IFI16 | 0.0065 | ICAM1 | 0.043 |
| IL18R1 | 8.30E-16 | VEGFB | 0.0054 | CD8A | 0.0071 | VEGFB | 0.044 |
| NFKB1 | 8.60E-16 | CD14 | 0.0066 | IL18R1 | 0.013 | IL18R1 | 0.048 |
| STAT3 | 7.60E-15 | BCL2 | 0.0098 | IL1R1 | 0.039 | STAT3 | 0.048 |
| BCL2 | 4.00E-14 | P13 | 0.018 | JUN | 0.058 | CD4 | 0.068 |
| IL1B | 4.70E-11 | IL18R1 | 0.02 | P13 | 0.16 | CCR3 | 0.089 |
| PI3 | 6.20E-11 | CCR3 | 0.059 | MX1 | 0.16 | P13 | 0.11 |
| HSPA1A | 5.80E-09 | IL1R1 | 0.067 | CD4 | 0.2 | CD14 | 0.11 |
| CD4 | 1.30E-07 | ICAM1 | 0.083 | STAT3 | 0.21 | HSPA1A | 0.12 |
| ICAM1 | 3.40E-07 | ITGAM | 0.094 | IL1B | 0.29 | IF116 | 0.21 |
| TGFBR2 | 5.40E-07 | IFI16 | 0.13 | VEGFB | 0.3 | BCL2 | 0.28 |
| IFI16 | 5.60E-07 | CD4 | 0.26 | NFKBIB | 0.3 | NFKB1 | 0.31 |
| HLADRA | 1.20E-05 | CD8A | 0.29 | CCR3 | 0.32 | CD8A | 0.33 |
| IL1R1 | 5.70E-05 | IL1B | 0.42 | BPI | 0.53 | ITGAM | 0.47 |
| CD8A | 6.30E-05 | STAT3 | 0.5 | HSPA1A | 0.7 | NFKBIB | 0.59 |
| CD14 | 0.00018 | HSPA1A | 0.55 | ICAM1 | 0.79 | IL1B | 0.77 |
| BPI | 0.00085 | NFKB1 | 0.9 | CD14 | 0.98 | MX1 | 0.83 |
| CCR3 | 0.0014 | NFKBIB | 0.91 | ITGAM | 0.99 | BPI | 0.94 |
| MX1 | 0.017 | MX1 | 0.96 | NFKB1 | 0.99 | ITGAL | included |
| JUN | 0.017 | BPI | 1 | HLADRA | included | HLADRA | included |

(continued)

| Gene Loci (24) | LogIT p-value | Gene Loci (24) | LogIT p-value | Gene Loci (24) | LogIT p-value | Gene Loci (24) | LogIT p-value |
|---|---|---|---|---|---|---|---|
| | **Step 1** | | **Step 2** | | **Step 3** | | **Step 4** |
| VEGFB | 0.36 | CASP9 | included | CASP9 | included | CASP9 | included |
| | | | | | | | |
| R-squared = | 0.397 | R-squared | 0.544 | R-squared | 0.628 | R-squared | 0.669 |
| | | | | | | | |
| itgam + hladra | $R^2=0.434$ | | | | | | |
| itgal + hladra | $R^2=0.55$ | | | | | | |
| | | | | | | | |
| in this 3-gene model, hladra is most significant, itgal & casp9 are comparable | | | | | | | |

These data support illustrate that Gene Expression Profiles with sufficient precision and calibration as described herein (1) can determine subsets of individuals with a known biological condition, particularly individuals with multiple sclerosis or individuals with inflammatory conditions related to multiple sclerosis; (2) may be used to monitor the response of patients to therapy; (3) may be used to assess the efficacy and safety of therapy; and (4) may used to guide the medical management of a patient by adjusting therapy to bring one or more relevant Gene Expression Profiles closer to a target set of values, which may be normative values or other desired or achievable values. It has been shown that Gene Expression Profiles may provide meaningful information even when derived from ex vivo treatment of blood or other tissue. It has been shown that Gene Expression Profiles derived from peripheral whole blood are informative of a wide range of conditions neither directly nor typically associated with blood.

Gene Expression Profiles is used for characterization and monitoring of treatment efficacy of individuals with multiple sclerosis, or individuals with inflammatory conditions related to multiple sclerosis.

Additionally Gene Expression Profiles is also used for characterization and early identification (including pre-symptomatic states) of infectious disease. This characterization includes discriminating between infected and uninfected individuals, bacterial and viral infections, specific subtypes of pathogenic agents, stages of the natural history of infection (e.g., early or late), and prognosis. Use of the algorithmic and statistical approaches discussed above to achieve such identification and to discriminate in such fashion is within the scope of various embodiments herein.

**Table 4**

| Multiple Sclerosis or Inflammatory Conditions Related to Multiple Sclerosis Gene Expression Panel | | | |
|---|---|---|---|
| Symbol | Name | Classification | Description |
| APAF1 | Apoptotic Protease Activating Factor 1 | Protease activating peptide | Cytochrome c binds to APAF1, triggering activation of CASP3, leading to apoptosis. May also facilitate procaspase 9 auto activation. |
| ARG2 | Arginase II | Enzyme/redox | Catalyzes the hydrolysis of arginine to ornithine and urea; may play a role in down regulation of nitric oxide synthesis |
| BCL2 | B-cell CLL / lymphoma 2 | Apoptosis Inhibitor - cell cycle control -oncogenesis | Blocks apoptosis by interfering with the activation of caspases |

(continued)

| Multiple Sclerosis or Inflammatory Conditions Related to Multiple Sclerosis Gene Expression Panel | | | |
|---|---|---|---|
| Symbol | Name | Classification | Description |
| BPI | Bactericidal/permeabi lity-increasing protein | Membrane- bound protease | LPS binding protein; cytotoxic for many gram negative organisms; found in myeloid cells |
| C1QA | Complement component 1, q subcomponent, alpha polypeptide | Proteinase/ proteinase inhibitor | Serum complement system; forms C1 complex with the proenzymes c1r and c1s |
| CALCA | Calcitonin/calcitonin- related polypeptide, alpha | cell-signaling and activation | AKA CALC1; Promotes rapid incorporation of calcium into bone |
| CASP1 | Caspase 1 | Proteinase | Activates IL1B; stimulates apoptosis |
| CASP3 | Caspase 3 | Proteinase / Proteinase Inhibitor | Involved in activation cascade of caspases responsible for apoptosis - cleaves CASP6, CASP7, CASP9 |
| CASP9 | Caspase 9 | Proteinase | Binds with APAF1 to become activated; cleaves and activates CASP3 |
| CCL1 | Chemokine (C-C Motif) ligand 1 | Cytokines- chemokines- growth factors | Secreted by activated T cells; chemotactic for monocytes, but not neutrophils; binds to CCR8 |
| CCL2 | Chemokine (C-C Motif) ligand 2 | Cytokines-chemokines- growth factors | CCR2 chemokine; Recruits monocytes to areas of injury and infection; Upregulated in liver inflammation; Stimulates IL-4 production; Implicated in diseases involving monocyte, basophil infiltration of tissue (e.g. psoriasis, rheumatoid arthritis, atherosclerosis) |
| CCL3 | Chemokine (C-C motif) ligand 3 | Cytokines-chemokines- growth factors | AKA: MIP1-alpha; monokine that binds to CCR1, CCR4 and CCR5; major HIV-suppressive factor produced by CD8 cells. |
| CCL4 | Chemokine (C-C Motif) ligand 4 | Cytokines-chemokines- growth factors | Inflammatory and chemotactic monokine; binds to CCR5 and CCR8 |

(continued)

| Multiple Sclerosis or Inflammatory Conditions Related to Multiple Sclerosis Gene Expression Panel | | | |
|---|---|---|---|
| Symbol | Name | Classification | Description |
| CCL5 | Chemokine (C-C Motif) ligand 5 | Cytokines-chemokines-growth factors | Binds to CCR1, CCR3, and CCR5 and is a chemoattractant for blood monocytes, memory T-helper cells and eosinophils; A major HIV-suppressive factor produced by CD8-positive T-cells |
| CCR1 | chemokine (C-C motif) receptor 1 | chemokine receptor | A member of the beta chemokine receptor family (seven transmembrane protein). Binds SCYA3/MIP-1a, SCYA5/RANTES, MCP-3, HCC-1, 2, and 4, and MPIF-1. Plays role in dendritic cell migration to inflammation sites and recruitment of monocytes. |
| CCR3 | Chemokine (C-C motif) receptor 3 | Chemokine receptor | C-C type chemokine receptor (Eotaxin receptor) binds to Eotaxin, Eotaxin-3, MCP-3, MCP-4, SCYA5/RANTES and mip-1 delta thereby mediating intracellular calcium flux. Alternative co-receptor with CD4 for HIV-1 infection. Involved in recruitment of eosinophils. Primarily a Th2 cell chemokine receptor. |
| CCR5 | chemokine (C-C motif) receptor 5 | chemokine receptor | Binds to CCL3/MIP-1a and CCL5/RANTES. An important co-receptor for macrophage-tropic virus, including HTV, to enter cells. |
| CD14 | CD14 antigen | Cell Marker | LPS receptor used as marker for monocytes |
| CD19 | CD19 antigen | Cell Marker | AKA Leu 12; B cell growth factor |
| CD3Z | CD3 antigen, zeta polypeptide | Cell Marker | T-cell surface glycoprotein |
| CD4 | CD4 antigen (p55) | Cell Marker | Helper T-cell marker |
| CD86 | CD 86 Antigen (cD 28 antigen ligand) | Cell signaling and activation | AKA B7-2; membrane protein found in B lymphocytes and monocytes; co-stimulatory signal necessary for T lymphocyte proliferation through IL2 production. |

(continued)

| Multiple Sclerosis or Inflammatory Conditions Related to Multiple Sclerosis Gene Expression Panel | | | |
|---|---|---|---|
| Symbol | Name | Classification | Description |
| CD8A | CD8 antigen, alpha polypeptide | Cell Marker | Suppressor T cell marker |
| CKS2 | CDC28 protein kinase regulatory subunit 2 | Cell signaling and activation | Essential for function of cyclin-dependent kinases |
| CRP | C-reactive protein | acute phase protein | the function of CRP relates to its ability to recognize specifically foreign pathogens and damaged cells of the host and to initiate their elimination by interacting with humoral and cellular effector systems in the blood |
| CSF2 | Granulocyte-monocyte colony stimulating factor | Cytokines-chemokines-growth factors | AKA GM-CSF; Hematopoietic growth factor; stimulates growth and differentiation of hematopoietic precursor cells from various lineages, including granulocytes, macrophages, eosinophils, and erythrocytes |
| CSF3 | Colony stimulating factor 3 (granulocyte) | Cytokines-chemokines-growth factors | AKA GCSF controls production differentiation and function of granulocytes. |
| CXCL3 | Chemokine (C-X-C- motif) ligand 3 | Cytokines-chemokines-growth factors | Chemotactic pro-inflammatory activation-inducible cytokine, acting primarily upon hemopoietic cells in immunoregulatory processes, may also play a role in inflammation and exert its effects on endothelial cells in an autocrine fashion. |
| CXCL10 | Chemokine (C-X-C motif) ligand 10 | Cytokines-chemokines-growth factors | AKA: Gamma IP10; interferon inducible cytokine IP10; SCYB10; Ligand for CXCR3; binding causes stimulation of monocytes, NK cells; induces T cell migration |
| CXCR3 | chemokine (C-X-C motif) receptor 3 | cytokines-chemokines-growth factors | Binds to SCYB10/ IP-10, SCYB9/ MIG, SCYB11/ I-TAC. Binding of chemokines to CXCR3 results in integrin activation, cytoskeletal changes and chemotactic migration. |

(continued)

| Multiple Sclerosis or Inflammatory Conditions Related to Multiple Sclerosis Gene Expression Panel | | | |
|---|---|---|---|
| **Symbol** | **Name** | **Classification** | **Description** |
| DPP4 | Dipeptidyl-peptidase 4 | Membrane protein; exopeptidase | Removes dipeptides from unmodified, n-terminus prolines; has role in T cell activation |
| DTR | Diphtheria toxin receptor (heparin-binding epidermal growth factor-like growth factor) | cell signaling, mitogen | Thought to be involved in macrophage-mediated cellular proliferation. DTR is a potent mitogen and chemotactic factor for fibroblasts and smooth muscle cells, but not endothelial cells. |
| ELA2 | Elastase 2, neutrophil | Protease | Modifies the functions of NK cells, monocytes and granulocytes |
| F3 | F3 | enzyme / redox | AKA thromboplastin, Coagulation Factor 3; cell surface glycoprotein responsible for coagulation catalysis |
| FCGR1A | Fc fragment of IgG, high affinity receptor IA | Membrane protein | Membrane receptor for CD64; found in monocytes, macrophages and neutrophils |
| FTL | Ferritin, light polypeptide | iron chelator | Intracellular, iron storage protein |
| GZMB | Granzyme B | proteinase | AKA CTLA1; Necessary for target cell lysis in cell-mediated immune responses. Crucial for the rapid induction of target cell apoptosis by cytotoxic T cells. Inhibition of the GZMB-IGF2R (receptor for GZMB) interaction prevented GZMB cell surface binding, uptake, and the induction of apoptosis. |
| HLA-DRA | Major Histocompatability Complex; class II, DR alpha | Membrane protein | Anchored heterodimeric molecule; cell-surface antigen presenting complex |
| HMOX1 | Heme oxygenase (decycling) 1 | Enzyme / Redox | Endotoxin inducible |
| HSPA1A | Heat shock protein 70 | Cell Signaling and activation | heat shock protein 70 kDa; Molecular chaperone, stabilizes AU rich mRNA |

(continued)

| Multiple Sclerosis or Inflammatory Conditions Related to Multiple Sclerosis Gene Expression Panel | | | |
|---|---|---|---|
| Symbol | Name | Classification | Description |
| HIST1H1C | Histo 1, Hic | Basic nuclear protein | responsible for the nucleosome structure within the chromosomal fiber in eukaryotes; may attribute to modification of nitrotyrosine-containing proteins and their immunoreactivity to antibodies against nitrotyrosine. |
| ICAM1 | Intercellular adhesion molecule 1 | Cell Adhesion / Matrix Protein | Endothelial cell surface molecule; regulates cell adhesion and trafficking, unregulated during cytokine stimulation |
| IFI16 | Gamma interferon inducible protein 16 | Cell signaling and activation | Transcriptional repressor |
| IFNA2 | Interferon, alpha 2 | Cytokines-chemokines-growth factors | interferon produced by macrophages with antiviral effects |
| IFNG | Interferon, Gamma | Cytokines / Chemokines / Growth Factors | Pro- and anti-inflammatory activity; TH1 cytokine; nonspecific inflammatory mediator; produced by activated T-cells. |
| IL10 | Interleukin 10 | Cytokines-chemokines-growth factors | Anti-inflammatory; TH2; suppresses production of proinflammatory cytokines |
| IL12B | Interleukin 12 p40 | Cytokines-chemokines-growth factors | Proinflammatory; mediator of innate immunity, TH1 cytokine, requires co-stimulation with IL-18 to induce IFN-g |
| IL13 | Interleukin 13 | Cytokines / Chemokines / Growth Factors | Inhibits inflammatory cytokine production |
| IL18 | Interleukin 18 | Cytokines-chemokines-growth factors | Proinflammatory, TH1, innate and acquired immunity, promotes apoptosis, requires co-stimulation with IL-1 or IL-2 to induce TH1 cytokines in T- and NK-cells |
| IL18R1 | Interleukin 18 receptor 1 | Membrane protein | Receptor for interleukin 18; binding the agonist leads to activation of NFKB-B; belongs to IL1 family but does not bind IL1A or IL1B. |

| Multiple Sclerosis or Inflammatory Conditions Related to Multiple Sclerosis Gene Expression Panel | | | |
|---|---|---|---|
| **Symbol** | **Name** | **Classification** | **Description** |
| IL1A | Interleukin 1, alpha | Cytokines-chemokines-growth factors | Proinflammatory; constitutively and inducibly expressed in variety of cells. Generally cytosolic and released only during severe inflammatory disease |
| IL1B | Interleukin 1, beta | Cytokines-chemokines-growth factors | Proinflammatory; constitutively and inducibly expressed by many cell types, secreted |
| IL1R1 | Interleukin 1 receptor, type I | Cell signaling and activation | AKA: CD12 or IL1R1RA; Binds all three forms ofinterleukin-1 (IL1A, IL1B and IL1RA). Binding of agonist leads to NFKB activation |
| IL1RN | Interleukin 1 Receptor Antagonist | Cytokines / Chemokines / Growth Factors | IL1 receptor antagonist; Anti-inflammatory; inhibits binding of IL-1 to IL-1 receptor by binding to receptor without stimulating IL-1-like activity |
| IL2 | Interleukin 2 | Cytokines Chemokines / Growth Factors | / T-cell growth factor, expressed by activated T-cells, regulates lymphocyte activation and differentiation; inhibits apoptosis, TH1 cytokine |
| IL4 | Interleukin 4 | Cytokines / Chemokines / Growth Factors | Anti-inflammatory; TH2; suppresses proinflammatory cytokines, increases expression of IL-1RN, regulates lymphocyte activation |
| IL5 | Interleukin 5 | Cytokines / Chemokines / Growth Factors | Eosinophil stimulatory factor; stimulates late B cell differentiation to secretion of Ig |
| IL6 | Interleukin 6 (interferon, beta 2) | Cytokines- chemokines-growth factors | Pro- and anti-inflammatory activity, TH2 cytokine, regulates hematopoietic system and activation of innate response |
| IL8 | Interleukin 8 | Cytokines- chemokines-growth factors | Proinflammatory, major secondary inflammatory mediator, cell adhesion, signal transduction, cell-cell signaling, angiogenesis, synthesized by a wide variety of cell types |

(continued)

| Multiple Sclerosis or Inflammatory Conditions Related to Multiple Sclerosis Gene Expression Panel | | | |
|---|---|---|---|
| Symbol | Name | Classification | Description |
| IL15 | Interleukin 15 | cytokines-chemokines-growth factors | Proinflammatory, mediates T-cell activation, inhibits apoptosis, synergizes with IL-2 to induce IFN-g and TNF-a |
| IRF5 | interferon regulatory factor 5 | Transcription factor | possess a novel helix-turn-helix DNA-binding motif and mediate virus- and interferon (IFN)-induced signaling pathways. |
| IRF7 | Interferon regulatory factor 7 | Transcription Factor | Regulates transcription of interferon genes through DNA sequence-specific binding. Diverse roles include virus-mediated activation of interferon, and modulation of cell growth, differentiation, apoptosis, and immune system activity. |
| ITGA-4 | integrin alpha 4 | integrin | receptor for fibronectin and VCAM1; triggers homotypic aggregation for VLA4 positive leukocytes; participates in cytolytic T-cell interactions with target cells. |
| ITGAM | Integrin, alpha M; complement receptor | integrin | AKA: Complement receptor, type 3, alpha subunit; neutrophil adherence receptor; role in adherence of neutrophils and monocytes to activate endothelium |
| LBP | Lipopolysaccharide binding protein | membrane protein | Acute phase protein; membrane protein that binds to Lipid a moiety of bacterial LPS |
| LTA | LTA (lymphotoxin alpha) | Cytokine | Cytokine secreted by lymphocytes and cytotoxic for a range of tumor cells; active in vitro and in vivo |
| LTB | Lymphotoxin beta (TNFSF3) | Cytokine | Inducer of inflammatory response and normal lymphoid tissue development |
| JUN | v-jun avian sarcoma virus 17 oncogene homolog | Transcription factor-DNA binding | Proto-oncoprotein; component of transcription factor AP-1 that interacts directly with target DNA sequences to regulate gene expression |
| MBL2 | Mannose-binding protein | lectin | AKA: MBP1; mannose binding protein C precursor |

(continued)

| Multiple Sclerosis or Inflammatory Conditions Related to Multiple Sclerosis Gene Expression Panel | | | |
|---|---|---|---|
| Symbol | Name | Classification | Description |
| MIF | Macrophage migration inhibitory factor | Cell signaling and growth factor | AKA; GIF; lymphokine, regulators macrophage functions through suppression of anti-inflammatory effects of glucocorticoids |
| MMP9 | Matrix metalloproteinase 9 | proteinase | AKA gelatinase B; degrades extracellular matrix molecules, secreted by IL-8-stimulated neutrophils |
| MMP3 | Matrix metalloproteinase 3 | proteinase | capable of degrading proteoglycan, fibronectin, laminin, and type IV collagen, but not interstitial type I collagen. |
| MX1 | Myxovirus resistance 1; interferon inducible protein p78 | peptide | Cytoplasmic protein induced by influenza; associated with MS |
| N33 | Putative prostate cancer tumor suppressor | Tumor Suppressor | Integral membrane protein. Associated with homozygous deletion in metastatic prostate cancer. |
| NFKB1 | Nuclear factor of kappa light polypeptide gene enhancer in B-cells 1 (p105) | Transcription Factor | p105 is the precursor of the p50 subunit of the nuclear factor NFKB, which binds to the kappa-b consensus sequence located in the enhancer region of genes involved in immune response and acute phase reactions; the precursor does not bind DNA itself |
| NFKBIB | Nuclear factor of kappa light polypeptide gene enhancer in B-cells inhibitor, beta | Transcription Regulator | Inhibits/regulates NFKB complex activity by trapping NFKB in the cytoplasm. Phosphorylated serine residues mark the NFKBIB protein for destruction thereby allowing activation of the NFKB complex. |
| NOS1 | nitric oxide synthase 1 (neuronal) | enzyme / redox | synthesizes nitric oxide from L-arginine and molecular oxygen, regulates skeletal muscle vasoconstriction, body fluid homeostasis, neuroendocrine physiology, smooth muscle motility, and sexual function |

(continued)

| Multiple Sclerosis or Inflammatory Conditions Related to Multiple Sclerosis Gene Expression Panel | | | |
|---|---|---|---|
| Symbol | Name | Classification | Description |
| NOS3 | Nitric oxide synthase 3 | enzyme / redox | enzyme found in endothelial cells mediating smooth muscle relation; promotes clotting through the activation of platelets |
| PAFAH1B1 | Platelet activating factor acetylhydrolase, isoform !b, alpha subunit; 45 kDa | Enzyme | Inactivates platelet activating factor by removing the acetyl group |
| PF4 | Platelet Factor 4 (SCYB4) | Chemokine | PF4 is released during platelet aggregation and is chemotactic for neutrophils and monocytes. PF4's major physiologic role appears to be neutralization of heparin-like molecules on the endothelial surface of blood vessels, thereby inhibiting local antithrombin III activity and promoting coagulation. |
| PI3 | Proteinase inhibitor 3 skin derived | Proteinase inhibitor- protein binding- diseases; extracellular matrix | aka SKALP; Proteinase inhibitor found in epidermis of several inflammatory skin it's expression can be used as a marker of skin irritancy |
| PLA2G7 | Phospholipase A2, group VII (platelet activating factor acetylhydrolase, plasma) | Enzyme / Redox | Platelet activating factor |
| PLAU | Plasminogen activator, urokinase | proteinase | AKA, uPA; cleaves plasminogen to plasmin (a protease responsible for nonspecific extracellular matrix degradation; UPA stimulates cell migration via a UPA receptor |
| PLAUR | plasminogen activator, urokinase receptor | Membrane protein; receptor | key molecule in the regulation of cell-surface plasminogen activation; also involved in cell signaling. |
| PTGS2 | Prostaglandin- endoperoxide synthase 2 | Enzyme | Key enzyme in prostaglandin biosynthesis and induction of inflammation |
| PTX3 | Pentaxin-related gene, rapidly induced by IL-1 beta | Acute Phase Protein | AKA TSG-14; Pentaxin 3; Similar to the pentaxin subclass of inflammatory acute-phase proteins; novel marker of inflammatory reactions |

(continued)

| Multiple Sclerosis or Inflammatory Conditions Related to Multiple Sclerosis Gene Expression Panel | | | |
|---|---|---|---|
| Symbol | Name | Classification | Description |
| RAD52 | RAD52 (S. cerevisiae) homolog | DNA binding proteins or | Involved in DNA double-stranded break repair and meiotic / mitotic recombination |
| SERPINE1 | Serine (or cysteine) protease inhibitor, class B (ovalbumin), member 1 | Proteinase / Proteinase Inhibitor | Plasminogen activator inhibitor-1 I PAI-1 |
| SFTPD | Surfactant, pulmonary associated protein D | extracellular lipoprotein | AKA: PSPD; mannose-binding protein; suggested role in innate immunity and surfactant metabolism |
| SLC7A1 | Solute carrier family 7, member 1 | Membrane protein; permease | High affinity, low capacity permease involved in the transport of positively charged amino acids |
| SPP1 | secreted phosphoprotein 1 (osteopontin) | cell signaling and activation | binds vitronectin; protein ligand of CD44, cytokine for type 1 responses mediated by macrophages |
| STAT3 | Signal transduction and activator of transcription 3 | Transcription factor | AKA APRF: Transcription factor for acute phase response genes; rapidly activated in response to certain cytokines and growth factors; binds to IL6 response elements |
| TGFBR2 | Transforming growth factor, beta receptor | Membrane II protein | AKA: TGFR2; membrane protein involved in cell signaling and activation, ser/thr protease; binds to DAXX. |
| TIMP1 | Tissue inhibitor of metalloproteinase 1 | Proteinase / Proteinase Inhibitor | Irreversibly binds and inhibits metalloproteinases, such as collagenase |
| TLR2 | toll-like receptor 2 | cell signaling and activation | mediator of peptidoglycan and lipotechoic acid induced signaling |
| TLR4 | Toll-like receptor 4 | Cell signaling and activation | mediator of LPS induced signaling |

(continued)

| Multiple Sclerosis or Inflammatory Conditions Related to Multiple Sclerosis Gene Expression Panel | | | |
|---|---|---|---|
| Symbol | Name | Classification | Description |
| TNF | Tumor necrosis factor | Cytokine/tumor necrosis factor receptor ligand | Negative regulation of insulin action. Produced in excess by adipose tissue of obese individuals - increases IRS-1 phosphorylation and decreases insulin receptor kinase activity. Pro-inflammatory; $TH_1$ cytokine; Mediates host response to bacterial stimulus; Regulates cell growth & differentiation |
| TNFRSF7 | Tumor necrosis factor receptor superfamily, member 7 | Membrane protein; receptor | Receptor for CD27L; may play a role in activation of T cells |
| TNFSF13B | Tumor necrosis factor (ligand) superfamily, member 13b | Cytokines-chemokines-growth factors | B cell activating factor, TNF family |
| TNFRSF13B | Tumor necrosis factor receptor superfamily, member 13, subunit beta | Cytokines-chemokines-growth factors | B cell activating factor, TNF family |
| TNFSF5 | Tumor necrosis factor (ligand) superfamily, member 5 / | Cytokines-chemokines-growth factors | Ligand for CD40; expressed on the surface of T cells. It regulates B cell function by engaging CD40 on the B cell surface. |
| TNFSF6 | Tumor necrosis factor (ligand) superfamily, member 6 | Cytokines-chemokines-growth factors | AKA FasL; Ligand for FAS antigen; transduces apoptotic signals into cells |
| TREM1 | Triggering receptor expressed on myeloid cells 1 | cell signaling and activation | Member of the Ig superfamily; receptor exclusively expressed on myeloid cells. TREM1 mediates activation of neutrophils and monocytes and may have a predominant role in inflammatory responses |
| VEGF | vascular endothelial growth factor | cytokines-chemokines-growth factors | VPF; Induces vascular permeability, endothelial cell proliferation, angiogenesis. Produced by monocytes |

Example 13 - Clinical Data analyzed with Latent Class Modeling together with Substantive Criteria

Using a targeted 96-gene panel, selected to be informative relative to biological state of MS patients, primers and probes were prepared for a subset of 24 genes identified in the Stepwise Regression Analysis shown in Table 3 above.

Gene expression profiles were obtained using these subsets of genes. Actual correct classification rate for the MS patients and the normal subjects was computed. Multi-gene models were constructed which were capable of correctly classifying MS and normal subjects with at least 75% accuracy. These results are shown in Tables 5-9 below. As demonstrated in Tables 6-9, a few as two genes allows discrimination between individuals with MS and normals at an accuracy of at least 75%.

*One Gene Model*

All 24 genes were evaluated for significance (i.e., p-value) regarding their ability to discriminate between MS and Normals, and ranked in the order of significance (see, Table 5). The optimal cutoff on the delta ct value for each gene was chosen that maximized the overall correct classification rate. The actual correct classification rate for the MS and Normal subjects was computed based on this cutoff and determined as to whether both reached the 75% criteria. None of these 1-gene models satisfied the 75%/75% criteria.

Table 5

| gene | p-value |
|------|---------|
| CASP9 | 1.80E-19 |
| ITGAL | 3.00E-19 |
| ITGAM | 3.40E-16 |
| STAT3 | 2.10E-15 |
| NFKB1 | 2.90E-15 |
| NFKBIB | 5.60E-14 |
| HLADRA | 1.00E-11 |
| BCL2 | 5.40E-11 |
| IL1B | 2.30E-10 |
| PI3 | 3.10E-10 |
| IFI16 | 3.30E-10 |
| IL18R1 | 7.80E-10 |
| HSPA1A | 2.00E-08 |
| ICAM1 | 1.90E-07 |
| TGFBR2 | 4.80E-06 |
| CD4 | 3.30E-05 |
| BPI | 6.20E-05 |
| IL1R1 | 0.0001 |
| CD14 | 0.00082 |
| CD8A | 0.0012 |
| MX1 | 0.0076 |
| JUN | 0.027 |
| CCR3 | 0.13 |
| VEGFB | 0.58 |

*Two Gene Model*

The top 8 genes (lowest p-value discriminating between MS and Normals) were subject to further analysis in a two-gene model. Each of the top 8 genes, one at a time, was used as the first gene in a 2-gene model, where all 23 remaining genes were evaluated as the second gene in this 2-gene model. (See Table 6). Column four illustrates the evaluated correct classification rates for these models (Data for those combinations of genes that fell below the 75%/ 75% cutoff, not all shown). The p-values in the 2-gene models assess the fit of the null hypothesis that the 2-gene model yields predictions of class memberships (MS vs. Normal) that are no different from chance predictions. The p-values were obtained from the SEARCH stepwise logistic procedure in the GOLDMineR program.

Also included in Table 6 is the $R^2$ statistic provided by the GOLDMineR program, The $R^2$ statistic is a less formal statistical measure of goodness of prediction, which varies between 0 (predicted probability of being in MS is constant regardless of delta-ct values on the 2 genes) to 1 (predicted probability of being MS = 1 for each MS subject, and = 0 for each Normal subject).

The right-most column of Table 6 indicates whether the 2-gene model was further used in illustrate the development of 3-gene models. For this use, 7 models with the lowest p-values (most significant), plus a few others were included as indicated.

Table 6

| gene1 | gene2 | p-value | Correct Classification | | $R^2$ | used to illustrate 3-gene models? |
|---|---|---|---|---|---|---|
| | | | %MS | %normals | | |
| ITGAL | HLADRA | 1.6E-39 | 85.4% | 82.9% | 0.531 | YES |
| CASP9 | HLADRA | 1.9E-35 | 78.5% | 84.2% | 0.478 | YES |
| NFKBIB | HLADRA | 1.9E-31 | 80.0% | 80.9% | 0.429 | YES |
| STAT3 | HLADRA | 2.9E-31 | 77.7% | 86.2% | 0.428 | YES |
| NFKB1 | HLADRA | 3.0E-29 | 82.3% | 80.3% | 0.401 | YES |
| ITGAM | HLADRA | 1.6E-28 | 80.0% | 80.9% | 0.405 | YES |
| ITGAL | VEGFB | 7.3E-28 | 77.7% | 80.9% | 0.383 | YES |
| HLADRA | BCL2 | 5.3E-27 | 76.2% | 82.9% | 0.374 | |
| HLADRA | CD4 | 8.3E-26 | 83.1% | 75.0% | 0.357 | |
| HLADRA | IL1B | 1.1E-24 | 74.6% | 79.6% | 0.342 | |
| HLADRA | HSPA1A | 1.3E-24 | 76.9% | 77.6% | 0.340 | |
| HLADRA | ICAM1 | 9.9E-24 | 76.2% | 77.0% | 0.331 | |
| CASP9 | VEGFB | 1.4E-22 | 75.4% | 77.0% | 0.317 | |
| HLADRA | IL18R1 | 1.4E-22 | 76.2% | 79.6% | 0.316 | |
| CASP9 | TGFBR2 | 5.0E-22 | 75.4% | 73.7% | 0.319 | YES |
| HLADRA | CD14 | 1.9E-21 | 75.4% | 73.7% | 0.300 | |
| CASP9 | ITGAL | 2.0E-21 | 73.8% | 70.4% | 0.303 | |
| ITGAL | PI3 | 2.8E-21 | 80.0% | 75.7% | 0.302 | |
| HLADRA | EFI16 | 3.4E-21 | 75.4% | 75.0% | 0.296 | |
| CASP9 | CCR3 | 3.9E-21 | 72.3% | 75.0% | 0.296 | |
| ITGAL | CD4 | 7.8E-21 | 76.2% | 71.1% | 0.293 | |
| CASP9 | IFI16 | 8.4E-21 | 75.4% | 74.3% | 0.292 | YES |
| ITGAL | ITGAM | 1.4E-20 | 76.2% | 75.7% | 0.303 | |
| STAT3 | CD14 | 2.1E-20 | 74.6% | 75.0% | 0.286 | |
| CASP9 | CD14 | 2.6E-20 | 74.6% | 75.7% | 0.286 | |
| CASP9 | PI3 | 2.7E-20 | 70.8% | 77.0% | 0.287 | |
| ITGAL | CD14 | 4.6E-20 | 76.2% | 71.7% | 0.284 | |
| ITGAL | IFI16 | 5.5E-20 | 77.7% | 71.1% | 0.283 | |
| ITGAL | CCR3 | 9.6E-20 | | | 0.280 | |
| CASP9 | JUN | 1.2E-19 | 76.2% | 76.3% | 0.290 | |
| BCL2 | VEGFB | 1.8E-19 | 76.2% | 73.0% | 0.274 | |
| CASP9 | CD4 | 2.1E-19 | 74.6% | 67.1% | 0.274 | |
| ITGAL | NFKB1 | 2.2E-19 | 75.4% | 71.7% | 0.276 | |

(continued)

| gene1 | gene2 | p-value | Correct Classification | | R² | used to illustrate |
| | | | %MS | %normals | | 3-gene models? |
|---|---|---|---|---|---|---|
| ITGAL | IL1B | 2.9E-19 | 75.4% | 72.4% | 0.273 | |
| ITGAL | NFKBIB | 3.9E-19 | 70.8% | 75.7% | 0.273 | |
| CASP9 | BCL2 | 4.7E-19 | 72.3% | 73.0% | 0.270 | |
| ITGAL | JUN | 4.7E-19 | | | 0.281 | |
| ITGAL | IL18R1 | 6.6E-19 | 75.4% | 69.1% | 0.269 | |
| CASP9 | STAT3 | 6.7E-19 | 76.2% | 71.7% | 0.267 | |
| CASP9 | IL1R1 | 7.9E-19 | 72.3% | 73.7% | 0.266 | |
| HLADRA | PI3 | 1.0E-18 | 74.6% | 73.0% | 0.261 | |
| CASP9 | IL1B | 1.1E-18 | 77.7% | 69.1% | 0.265 | |
| ITGAL | STAT3 | 1.1E-18 | 70.0% | 74.3% | 0.266 | |
| ITGAL | CD8A | 1.1E-18 | 70.0% | 76.3% | 0.266 | |
| ITGAM | IFI16 | 1.3E-18 | 75.4% | 76.3% | 0.275 | |
| **CASP9** | **ICAM1** | 1.4E-18 | 74.6% | 74.3% | 0.263 | |
| **CASP9** | **BPI** | 1.4E-18 | 76.2% | 71.1% | 0.264 | |
| **NFKB1** | **VEGFB** | 1.5E-18 | 76.9% | 69.1% | 0.263 | |
| **CASP9** | **CD8A** | 1.7E-18 | 73.8% | 74.3% | 0.262 | |
| **CASP9** | **NFKB1** | 1.8E-18 | 75.4% | 72.4% | 0.262 | |
| **ITGAL** | **BCL2** | 1.8E-18 | | | 0.264 | |
| **CASP9** | **NFKBIB** | 1.9E-18 | 77.7% | 69.7% | 0.261 | |
| **CASP9** | **IL18R1** | 2.0E-18 | 70.8% | 75.0% | 0.261 | |
| **CASP9** | **HSPA1A** | 2.0E-18 | 72.3% | 73.7% | 0.261 | |
| **ITGAL** | **ICAM1** | 2.2E-18 | 73.1% | 71.7% | 0.262 | |
| **ITGAL** | **BPI** | 2.2E-18 | 72.3% | 73.7% | 0.262 | |
| **ITGAL** | **IL1R1** | 2.7E-18 | 70.8% | 77.0% | 0.261 | |
| **HLADRA** | **TGFBR2** | 2.8E-18 | 74.6% | 75.0% | 0.269 | |
| **CASP9** | **ITGAM** | 2.9E-18 | 75.4% | 73.0% | 0.271 | |
| **ITGAL** | **HSPA1A** | 3.4E-18 | 75.4% | 69.7% | 0.260 | |
| **ITGAL** | **TGFBR2** | 3.8E-18 | 75.4% | 71.7% | 0.270 | |
| **CASP9** | **MX1** | 4.0E-18 | 75.4% | 71.1% | 0.268 | |
| **ITGAL** | **MX1** | 9.0E-18 | 73.8% | 73.0% | 0.265 | |
| **HLADRA** | **CD8A** | 1.1E-17 | 74.6% | 67.1% | 0.248 | |
| **ITGAM** | **BCL2** | 5.2E-17 | 69.2% | 78.9% | 0.254 | |
| **ITGAM** | **CD14** | 3.5E-16 | 68.5% | 76.3% | 0.243 | |
| **ITGAM** | **TGFBR2** | 5.5E-16 | 75.4% | 76.3% | 0.240 | |
| **NFKBIB** | **TGFBR2** | 9.6E-14 | 73.8% | 74.3% | 0.222 | |

*Three Gene Model*

For each of the selected 2-gene models (including the 7 most significant), each of the remaining 22 genes was evaluated as being included as a third gene in the model. Table 7 lists these along with the incremental p-value associated with the 3$^{rd}$ gene. Only models
where the incremental p-value < .05 are listed. The others were excluded because the additional MS vs. Normal discrimination associated with the 3$^{rd}$ gene was not significant at the .05 level. Each of these 3-gene models was evaluated further to determine whether incremental p-values associated with the other 2 genes was also significant. If the incremental p-value of any one of the 3 was found to be less than .05, it was excluded  because it did not make a significant improvement over one of the 2-gene sub-models. An example of a 3-gene model that failed this secondary test was the model containing NFKBIB, HLADRA and CASP9. Here, the incremental p-value for NFKBIB was found to be only .13 and therefore did not provide a significant improvement over the 2-gene model containing HLADRA and CASP9. The ESTIMATE procedure in GOLDMineR was used to compute all of the incremental p-values, which are shown in Table 7.

Table 7

| | | incremental p-value | | | | | | incremental p-value | | incremental p-value | |
|---|---|---|---|---|---|---|---|---|---|---|---|
| | | gene | p-value | p-value | R-squared | %MS | %normals | gene | p-value | gene | p-value |
| ITGAL | HLADRA | CASP9 | 0.00024 | 2.10E-41 | 0.563 | 85.4% | 86.8% | | | | |
| ITGAL | HLADRA | NFKBIB | 0.003 | 2.20E-40 | 0.553 | 81.5% | 88.2% | | | | |
| ITGAL | HLADRA | ILIB | 0.0061 | 4.10E-40 | 0.549 | 85.4% | 84.9% | | | | |
| ITGAL | HLADRA | ITGAM | 0.02 | 2.20E-39 | 0.552 | 86.2% | 84.9% | | | | |
| ITGAL | HLADRA | VEGFB | 0.021 | 1.20E-39 | 0.544 | 83.1% | 86.2% | | | | |
| ITGAL | HLADRA | P13 | 0.03 | 1.70E-39 | 0.543 | 83.8% | 84.9% | | | | |
| CASP9 | HLADRA | ITGAL | 1.40E-08 | 2.10E-41 | 0.563 | 85.4% | 86.8% | | | | |
| CASP9 | HLADRA | TGFBR2 | 0.00048 | 2.60E-36 | 0.515 | 83.8% | 82.2% | | | | |
| CASP9 | HLADRA | BCL2 | 0.00056 | 5.20E-37 | 0.509 | 85.4% | 81.6% | | | | |
| CASP9 | HLADRA | IFII6 | 0.0016 | 1.30E-36 | 0.506 | 83.1% | 84.9% | | | | |
| CASP9 | HLADRA | CD8A | 0.0043 | 3.30E-36 | 0.499 | 83.8% | 80.9% | | | | |
| CASP9 | HLADRA | STAT3 | 0.022 | 1.40E.35 | 0.493 | 82.3% | 82.2% | | | | |
| CASP9 | HLADRA | CCR3 | 0.03 | 1.80E-35 | 0.489 | 81.5% | 80.9% | | | | |
| CASP9 | HLADRA | MXI | 0.034 | 4.40E-35 | 0.497 | 83.1% | 80.3% | | | | |
| NFKBIB | HLADRA | ITGAL | 1.20E-11 | 2.20E-40 | 0.553 | 81.5% | 88.2% | | | | |
| NFKBIB | HLADRA | BCL2 | 1.10E-06 | 1.40E-35 | 0.492 | 80.0% | 83.6% | | | | |
| NFKBIB | HLADRA | STAT3 | 5.20E-06 | 6.10E-35 | 0.484 | 80.8% | 81.6% | | | | |
| NFKBIB | HLADRA | CASP9 | 5.40E-06 | 6.30E-35 | 0.483 | 77.7% | 81.6% | nfkbib | 0.13 | hladra | 2.80E-19 |
| NFKBIB | HLADRA | ILIB | 0.00028 | 2.60E-33 | 0.464 | 79.2% | 84.2% | | | | |
| NFKBIB | HLADRA | IFII6 | 0.00039 | 3.50E-33 | 0.464 | 77.7% | 84.9% | | | | |
| NFKBIB | HLADRA | HSPAIA | 0.0004 | 3.60E-33 | 0.461 | 79.2% | 80.9% | nfkbib | 3.40E-11 | | |
| NFKBIB | HLADRA | CD4 | 0.00043 | 3.90E-33 | 0.462 | 79.2% | 80.9% | | | | |
| NFKBIB | HLADRA | BPI | 0.0043 | 3.20E-32 | 0.449 | 79.2% | 82.9% | nfkbib | 3.70E-18 | | |
| NFKBIB | HLADRA | MX1 | 0.0045 | 5.80E-32 | 0.458 | 80.0% | 83.6% | nfkbib | 2.20E-20 | | |
| NFKBIB | HLADRA | IL18R1 | 0.0046 | 3.40E-32 | 0.45 | 77.7% | 82.9% | | | | |
| NFKBIB | HLADRA | ITGAM | 0.0053 | 2.10E-31 | 0.45 | 80.0% | 82.9% | | | | |
| NFKBIB | HLADRA | CD8A | 0.0068 | 4.80E-32 | 0.449 | 78.5% | 83.6% | nfkbib | 4.10E-17 | | |
| NFKBIB | HLADRA | ICAM1 | 0.015 | 9.70E-32 | 0.445 | 77.7% | 81.6% | | | | |
| NFKBIB | HLADRA | TGFBR2 | 0.019 | 6.20E-31 | 0.445 | 77.7% | 81.6% | nfkbib | 2.20E-15 | | |
| NFKBIB | HLADRA | NFKB1 | 0.021 | 1.30E-31 | 0.443 | 77.7% | 83.6% | nfkbib | 8.40E-05 | | |

| | | incremental p-value | | | | | | incremental p-value | | incremental p-value | |
|---|---|---|---|---|---|---|---|---|---|---|---|
| | | gene | p-value | p-value | R-squared | %MS | %normals | gene | p-value | gene | p-value |
| NFKBIB | HLADRA | CD14 | 0.036 | 2.10E-31 | 0.441 | 77.7% | 82.2% | | | | |
| NFKBIB | HLADRA | PI3 | 0.049 | 2.70E-31 | 0.438 | 76.9% | 83.6% | | | | |
| STAT3 | HLADRA | ITGAL | 2.70E-10 | 6.70E-39 | 0.535 | 82.3% | 86.2% | | | | |
| STAT3 | HLADRA | BCL2 | 4.30E-07 | 8.40E-36 | 0.495 | 83.1% | 87.5% | STAT3 | 1.80E-11 | | |
| STAT3 | HLADRA | CASP9 | 7.40E-07 | 1.40E-35 | 0.493 | 80.0% | 84.2% | | | | |
| STAT3 | HLADRA | NFKB1B | 3.40E-06 | 6.10E-35 | 0.484 | 79.2% | 83.6% | STAT3 | 5.20E-06 | | |
| STAT3 | HLADRA | ILIR1 | 1.80E-05 | 3.00E-34 | 0.473 | 79.2% | 81.6% | STAT3 | 4.00E-21 | | |
| STAT3 | HLADRA | CD8A | 0.00012 | 1.80E-33 | 0.466 | 79.2% | 80.3% | STAT3 | 1.40E-18 | | |
| STAT3 | HLADRA | NFKB1 | 0.00057 | 7.60E-33 | 0.46 | 80.8% | 84.2% | STAT3 | 4.30E-06 | | |
| STAT3 | HLADRA | ITGAM | 0.0062 | 4.10E-31 | 0.45 | 81.5% | 84.2% | | | | |
| STAT3 | HLADRA | IFH16 | 0.0062 | 6.70E-32 | 0.449 | 80.0% | 83.6% | | | | |
| STAT3 | HLADRA | CD4 | 0.0097 | 1.00E-31 | 0.446 | 81.5% | 83.6% | | | | |
| STAT3 | HLADRA | PI3 | 0.012 | 1.20E-31 | 0.445 | 80.0% | 82.9% | | | | |
| STAT3 | HLADRA | ILI8R1 | 0.021 | 2.00E-31 | 0.442 | 80.8% | 84.2% | | | | |
| NFKB1 | HLADRA | ITGAL | 2.00E-12 | 5.90E-39 | 0.537 | 83.8% | 86.2% | | | | |
| NFKB1 | HLADRA | CASP9 | 7.90E-08 | 1.70E-34 | 0.479 | 79.2% | 84.2% | | | | |
| NFKB1 | HLADRA | STAT3 | 4.30E-06 | 7.60E-33 | 0.46 | 80.8% | 84.2% | | | | |
| NFKB1 | HLADRA | NFKBIB | 8.40E-05 | 1.30E-31 | 0.443 | 77.7% | 83.6% | NFKB1 | 0.021 | | |
| NFKB1 | HLADRA | BCL2 | 0.00022 | 3.20E-31 | 0.439 | 76.9% | 82.9% | NFKB1 | 9.80E-07 | | |
| NFKB1 | HLADRA | HSPA1A | 0.00042 | 5.70E-31 | 0.435 | 78.5% | 82.9% | NFKB1 | 6.20E-09 | | |
| NFKB1 | HLADRA | ILIB | 0.00051 | 6.80E-31 | 0.435 | 78.5% | 81.6% | | | | |
| NFKB1 | HLADRA | IFI16 | 0.0009 | 1.20E-30 | 0.43 | 81.5% | 85.5% | | | | |
| NFKB1 | HLADRA | ITGAM | 0.0018 | 1.10E-29 | 0.43 | 78.5% | 82.9% | | | | |
| NFKB1 | HLADRA | ICAM1 | 0.0028 | 3.30E-30 | 0.426 | 78.5% | 82.9% | | | | |
| NFKB1 | HLADRA | CD8A | 0.0049 | 5.40E-30 | 0.424 | 77.7% | 83.6% | NFKB1 | 5.10E-15 | | |
| NFKB1 | HLADRA | BP1 | 0.0079 | 8.30E-30 | 0.419 | 77.7% | 84.9% | NFKB1 | 1.10E-15 | | |
| NFKB1 | HLADRA | CD4 | 0.011 | 1.10E-29 | 0.419 | 78.5% | 83.6% | | | | |
| NFKB1 | HLADRA | MX1 | 0.016 | 2.50E-29 | 0.425 | 80.0% | 82.9% | NFKB1 | 1.10E-17 | | |
| NFKB1 | HLADRA | PI3 | 0.018 | 1.70E-29 | 0.416 | 78.5% | 84.2% | | | | |
| NFKB1 | HLADRA | ILI8R1 | 0.025 | 2.30E-29 | 0.415 | 79.2% | 80.3% | | | | |
| ITGAM | HLADRA | ITGAL | 1.40E-13 | 2.20E-39 | 0.552 | 86.2% | 84.9% | | | | |

(continued)

| gene | gene | incremental p-value gene | p-value | p-value | R-squared | %MS | %normals | incremental p-value gene | p-value | incremental p-value gene | p-value |
|------|------|------|---------|---------|-----------|-----|----------|------|---------|------|---------|
| ITGAL | HLADRA | CASP9 | 8.90E-08 | 8.80E-34 | 0.481 | 78.5% | 82.2% | | | | |
| ITGAL | HLADRA | BCL2 | 2.50E-07 | 2.40E-33 | 0.476 | 78.5% | 83.6% | ITGAM | 1.00E-09 | | |
| ITGAL | HLADRA | IFI16 | 1.70E-05 | 1.30E-31 | 0.456 | 82.3% | 82.9% | | | | |
| ITGAL | HLADRA | NFKBIB | 2.80E-05 | 2.10E-31 | 0.45 | 80.0% | 82.9% | ITGAM | 0.0053 | | |
| ITGAL | HLADRA | STAT3 | 5.80E-05 | 4.10E-31 | 0.45 | 81.5% | 84.2% | | | | |
| ITGAL | HLADRA | CD8A | 0.00028 | 1.80E-30 | 0.441 | 80.0% | 82.9% | ITGAM | 3.60E-16 | | |
| ITGAL | HLADRA | CD4 | 0.00078 | 4.50E-30 | 0.437 | 79.2% | 83.6% | | | | |
| ITGAL | HLADRA | NFKB1 | 0.0021 | 1.10E-29 | 0.43 | 78.5% | 82.9% | | | | |
| ITGAL | HLADRA | ILIB | 0.0046 | 2.30E-29 | 0.427 | 77.7% | 82.2% | | | | |
| ITGAL | HLADRA | MX1 | 0.0054 | 2.90E-29 | 0.435 | 80.8% | 83.6% | ITGAM | 2.90E-18 | | |
| ITGAL | HLADRA | PI3 | 0.031 | 1.20E-28 | 0.417 | 77.7% | 82.2% | | | | |
| ITGAL | HLADRA | VEGFB | 0.031 | 1.20E-28 | 0.417 | 78.5% | 83.6% | ITGAM | 5.60E-18 | | |
| ITGAL | HLADRA | BP1 | 0.032 | 1.20E-28 | 0.417 | 79.2% | 82.9% | ITGAM | 3-00E-15 | | |
| ITGAM | VEGFB | HLADRA | 1.60E-14 | 1.20E-39 | 0.544 | 83.1% | 86.2% | ITGAL | 2.00E-28 | | |
| ITGAM | VEGFB | BCL2 | 4.70E-07 | 2.20E-32 | 0.452 | 80.0% | 82.2% | ITGAL | 1.20E-15 | | |
| ITGAM | VEGFB | CASP9 | 5.80E-05 | 2.20E-30 | 0.427 | 80.0% | 80.3% | ITGAL | 2.00E-10 | | |
| ITGAM | VEGFB | NFKB1 | 0.0021 | 6.10E-29 | 0.41 | 76.9% | 80.9% | ITGAL | 4.90E-13 | | |
| ITGAM | VEGFB | IFI16 | 0.0077 | 1.90E-28 | 0.402 | 76.9% | 81.6% | ITGAL | 3.70E-21 | | |
| ITGAM | VEGFB | CD14 | 0.014 | 3-30E-28 | 0.4 | 76.2% | 81.6% | ITGAL | 5.30E-27 | | |
| ITGAM | VEGFB | NFKBIB | 0.026 | 5.70E-28 | 0.397 | 79.2% | 80.3% | ITGAL | 8.80E-15 | | |
| ITGAM | VEGFB | CCR3 | 0.026 | 5.70E-28 | 0.397 | 77.7% | 80.9% | ITGAL | 2.50E-29 | | |
| ITGAM | VEGFB | PI3 | 0.041 | 8.20E-28 | 0.396 | 76.9% | 81.6% | ITGAL | 3.10E-21 | | |
| ITGAM | VEGFB | ITGAM | 0.043 | 4.00E-28 | 0.409 | 78.5% | 81.6% | ITGAL | 3.70E-15 | | |
| CASP9 | TGFBR2 | HLADRA | 4.60E-17 | 2.60E-36 | 0.515 | 83.8% | 82.2% | | | | |
| CASP9 | TGFBR2 | CCR3 | 0.00031 | 5.40E-24 | 0.354 | 80.0% | 78.9% | | | | |
| CASP9 | TGFBR2 | IFI16 | 0.0014 | 2.10E-23 | 0.347 | 78.5% | 78.9% | | | | |
| CASP9 | TGFBR2 | ITGAL | 0.002 | 3.00E-23 | 0.348 | 74.6% | 82.9% | | | | |
| CASP9 | TGFBR2 | JUN | 0.0087 | 1.10E-22 | 0.339 | 76.2% | 79.6% | | | | |
| CASP9 | TGFBR2 | CD4 | 0.018 | 2.10E-22 | 0.334 | 76.2% | 78.3% | | | | |
| CASP9 | IFI16 | HLADRA | 1.40E-18 | 1.30E-36 | 0.506 | 83.1% | 84.9% | | | | |
| CASP9 | IFI16 | CD14 | 0.00011 | 4.00E-23 | 0.335 | 75.4% | 77.6% | | | | |
| CASP9 | IFI16 | CCR3 | 0.0009 | 2.80E-22 | 0.323 | 74.6% | 73.7% | | | | |

| | | incremental p-value | | | | | | incremental p-value | | incremental p-value | |
|---|---|---|---|---|---|---|---|---|---|---|---|
| | | gene | p-value | p-value | R-squared | %MS | %normals | gene | p-value | gene | p-value |
| CASP9 | IFI16 | JUN | 0.0024 | 1.20E-21 | 0.326 | 76.9% | 77.6% | | | | |
| CASP9 | IFI16 | ITGAL | 0.0027 | 7.50E-22 | 0.319 | 74.6% | 75.0% | | | | |
| CASP9 | IFI16 | PI3 | 0.0075 | 1.90E-21 | 0.314 | 75.4% | 72.4% | | | | |
| CASP9 | IFI16 | CD4 | 0.025 | 5.50E-21 | 0.307 | 74.6% | 73.0% | | | | |

*Four and Five Gene Models*

The procedure for models containing 4 and five genes is similar to the one for three genes. Table 8 and 9 show the results associated with the use of most significant 3-gene model to obtain 4-gene and 5-gene models. The incremental p-values associated with each gene in the 4-gene and 5-gene models are shown, along with the percent classified correctly. As demonstrated by Tables 8 and 9 the addition of more genes in the model did not significantly alter the ability of the models to correctly classify MS patients and normals.

*Four and Five Gene Models*

Table 8

| gene 1 | gene 2 | gene 3 | incremental p-value | | %MS | %normals | incremental p-value | | incremental p-value | | incremental p-value | |
|--------|--------|--------|---------|---------|------|----------|-------|---------|---------|----------|-------|--------|
| | | | gene 4 | p-value | | | gene | p-value | gene | p-value | gene | p-vale |
| CASP9 | HLADRA | ITGAL | CCR3 | 0.006 | 85.4% | 83.6% | CASP9 | 9.00E-06 | HLADRA | 9.40E-21 | ITGAL | 3.00E-09 |

Table 9

| gene 1 | gene 2 | gene 3 | gene 4 | gene 5 | incremental p-value | | %MS | %normals | incremental p-value | | incremental p-value | | incremental p-value | | incremental p-value | |
|---|---|---|---|---|---|---|---|---|---|---|---|---|---|---|---|---|
| | | | | | p-value | | | | gene | p-value | gene | p-value | gene | p-value | gene | p-value |
| CASP9 | HLADRA | ITGAL | CCR3 | TGFBR2 | 0.0015 | | 86.9% | 84.2% | CASP9 | 6.20E-08 | HLADRA | 5.90E-18 | ITGAL | 1.60E-07 | CCR3 | 0.0023 |

**Claims**

1. A method of characterizing multiple sclerosis in a subject, based on a sample from the subject, the sample providing a source of RNAs, the method comprising:

   assessing a profile data set containing a quantitative measure of each constituent of a panel of at least two constituents as distinct RNA constituents in the subject sample, wherein at least one of the constituents is HLADRA, and a second constituent is selected from ITGAL, CASP9, NFKBIB, STAT3, NFKB1, ITGAM, BCL2, CD4, IL1B, HSPA1A, ICAM1, IL18R1, CD14, IFI16, PI3, TGFBR2, or CD8A, and wherein such measure is obtained using amplification under measurement conditions that are substantially repeatable and the constituents are selected so that measurement of the constituents distinguish between a normal subject and an MS-diagnosed subject with at least 75% accuracy.

2. A method for determining a profile data set for characterizing a subject with multiple sclerosis based on a sample from a subject known to have multiple sclerosis, the sample providing a source of RNAs, the method comprising:

   a) using amplification for determining the quantitative measure of the amount of RNA in a panel of constituents including at least 2 constituents, wherein at least one of the constituents is HLADRA, and a second constituent is selected from ITGAL, CASP9, NFKBIB, STAT3, NFKB1, ITGAM, BCL2, CD4, IL1B, HSPA1A, ICAM1, IL18R1, CD14, IFI16, PI3, TGFBR2, or CD8A, and,
   b) arriving at a measure of each constituent,

   wherein the profile data set comprises the measure of each constituent of the panel and wherein amplification is performed under measurement conditions that are substantially repeatable.

3. The method of claim 1 or 2, wherein the method comprises determining a quantitative measure of at least four constituents including HLADRA, CASP9, ITGAL and CCR3, and optionally further comprises determining a quantitative measure of a fifth constituent TGFBR2.

4. A method of characterizing multiple sclerosis according to any of claims 1-3, wherein the constituents are selected so as to distinguish from a normal and a MS-diagnosed subject washed out from therapy.

5. The method of claim 4, wherein said MS-diagnosed subject is washed out from therapy for three or more months.

6. A method according to any of claims 1-3, wherein the subject has a presumptive sign of multiple sclerosis selected from the group consisting of altered sensory, motor, visual or proprioceptive system with at least one of numbness or weakness in one or more limbs, often occurring on one side of the body at a time or the lower half of the body, partial or complete loss of vision, frequently in one eye at a time and often with pain during eye movement, double vision or blurring of vision, tingling or pain in numb areas of the body, electric-shock sensations that occur with certain head movements, tremor, lack of coordination or unsteady gait, fatigue, dizziness, muscle stiffness or spasticity, slurred speech, paralysis, problems with bladder, bowel or sexual function, and mental changes such as forgetfulness or difficulties with concentration, relative to medical standards.

7. The method according to any of claims 1-3, wherein the multiple sclerosis is from an autoimmune condition, an environmental condition, a viral infection, a bacterial infection, a eukaryotic parasitic infection, or a fungal infection.

8. The method according to any of claims 1-3, wherein the sample is selected from the group consisting of blood, a blood fraction, body fluid, a population of cells and tissue from the subject.

9. The method of claim 1, wherein assessing further comprises:

   comparing the profile data set to a baseline profile data set for the panel, wherein the baseline profile data set is related to the multiple sclerosis.

**Patentansprüche**

1. Verfahren zum Charakterisieren von multipler Sklerose an einem Probanden auf der Grundlage einer Probe des Probanden, wobei die Probe eine Quelle von RNAs bereitstellt, wobei das Verfahren Folgendes umfasst:

Bewerten eines Profildatensatzes, der ein quantitatives Maß jedes Bestandteils eines Panels von mindestens zwei Bestandteilen als unterschiedliche RNA-Bestandteile in der Probe des Probanden enthält, wobei mindestens einer der Bestandteile HLADRA ist und ein zweiter Bestandteil ausgewählt ist aus ITGAL, CASP9, NFKBIB, STAT3, NFKB1, ITGAM, BCL2, CD4, IL1B, HSPA1A, ICAM1, IL18R1, CD14, IFI16, PI3, TGFBR2 und CD8A, und wobei ein solches Maß durch die Amplifikation unter Messbedingungen erhalten wird, die im Wesentlichen wiederholbar sind, und die Bestandteile derart ausgewählt sind, dass die Messung der Bestandteile zwischen einem normalen Probanden und einem Probanden, bei dem MS diagnostiziert wurde, mit einer Genauigkeit von mindestens 75% unterscheidet.

2. Verfahren zum Bestimmen eines Profildatensatzes zum Charakterisieren eines Probanden mit multipler Sklerose auf der Grundlage einer Probe eines Probanden, von dem bekannt ist, dass er multiple Sklerose hat, wobei die Probe eine Quelle von RNAs bereitstellt, wobei das Verfahren Folgendes umfasst:

a) Nutzen von Amplifikation zum Bestimmen des quantitativen Maßes der Menge von RNA in einem Panel von Bestandteilen, das mindestens zwei Bestandteile enthält, wobei mindestens einer der Bestandteile HLADRA ist und ein zweiter Bestandteil ausgewählt ist aus ITGAL, CASP9, NFKBIB, STAT3, NFKB1, ITGAM, BCL2, CD4, IL1B, HSPA1A, ICAM1, IL18R1, CD14, IFI16, PI3, TGFBR2 und CD8A, und
b) Erreichen eines Maßes für jeden Bestandteil,

wobei der Profildatensatz das Maß jedes Bestandteils des Panels aufweist und wobei die Amplifikation unter Messbedingungen durchgeführt wird, die im Wesentlichen wiederholbar sind.

3. Verfahren nach Anspruch 1 oder 2, wobei das Verfahren das Bestimmen eines quantitativen Maßes von mindestens vier Bestandteilen einschließlich HLADRA, CASP9, ITGAL und CCR3 umfasst und optional des Weiteren das Bestimmen eines fünften Bestandteils TGFBR2 umfasst.

4. Verfahren zum Charakterisieren von multipler Sklerose nach einem der Ansprüche 1 bis 3, wobei die Bestandteile derart ausgewählt sind, dass zwischen einem normalen Probanden und einem Probanden, bei dem MS diagnostiziert wurde und der sich in einer Wash-out-Periode der Therapie befindet, unterschieden wird.

5. Verfahren nach Anspruch 4, wobei sich der Proband, bei dem MS diagnostiziert wurde, seit drei oder mehr Monaten in einer Wash-out-Periode der Therapie befindet.

6. Verfahren nach einem der Ansprüche 1 bis 3, wobei der Proband ein vermutliches Anzeichen von multipler Sklerose hat, das aus der Gruppe ausgewählt ist, die besteht aus verändertem Sinnes-, Bewegungs-, visuellen oder Propriozeptivsystem mit mindestens einem von Taubheit oder Schwäche in einem oder mehreren Gliedern, die oft an jeweils einer Seite des Körpers oder in der unteren Körperhälfte auftreten, teilweiser oder vollständiger Verlust der Sicht, häufig in jeweils einem Auge und oft mit Schmerzen bei der Augenbewegung, doppelte Sicht oder Verschwimmen der Sicht, Kribbeln oder Schmerzen in tauben Bereichen des Körpers, bei bestimmten Kopfbewegungen auftretende Elektroschock-Empfindungen, Tremor, mangelnde Koordination oder unsteter Gang, Müdigkeit, Schwindel, Muskelsteifigkeit oder Spastik, undeutliche Sprache, Lähmung, Probleme mit Blase, Darm oder sexueller Funktion und mentale Veränderungen wie Vergesslichkeit oder Konzentrationsschwierigkeiten, bezogen auf medizinischen Standards.

7. Verfahren nach einem der Ansprüche 1 bis 3, wobei die multiple Sklerose von einer Autoimmunerkrankung, einer Umgebungsbedingung, einer Virusinfektion, einer Bakterieninfektion, einer Infektion durch eukaryotische Parasiten oder einer Pilzinfektion stammt.

8. Verfahren nach einem der Ansprüche 1 bis 3, wobei die Probe aus der Gruppe ausgewählt ist, die aus Blut, einer Blutfraktion, Körperflüssigkeiten, einer Zellpopulation und Gewebe des Probanden besteht.

9. Verfahren nach Anspruch 1, wobei das Bewerten des Weiteren Folgendes aufweist:

Vergleichen des Profildatensatzes mit einem Grundlinien-Profildatensatz für das Panel, wobei der Grundlinien-Profildatensatz mit der multiplen Sklerose im Zusammenhang steht.

**Revendications**

**1.** Procédé de caractérisation de la sclérose en plaques chez un sujet, sur la base d'un échantillon provenant du sujet, l'échantillon fournissant une source d'ARN, le procédé comprenant :

l'évaluation d'un ensemble de données de profil contenant une mesure quantitative de chaque constituant d'un panel d'au moins deux constituants en tant que constituants d'ARN distincts dans l'échantillon du sujet, où au moins l'un des constituants est HLADRA et un second constituant est choisi parmi ITGAL, CASP9, NFKBIB, STAT3, NFKB1, ITGAM, BCL2, CD4, IL1B, HSPA1A, ICAM1, IL18R1, CD14, IFI16, PI3, TGFBR2 ou CD8A et où une telle mesure est obtenue à l'aide d'une amplification dans des conditions de mesure qui peuvent sensiblement être répétées et les constituants sont choisis de telle sorte que la mesure des constituants permet la distinction entre un sujet normal et un sujet diagnostiqué pour la sclérose en plaques (MS) avec une précision d'au moins 75 %.

**2.** Procédé de détermination d'un ensemble de données de profil pour la caractérisation d'un sujet atteint d'une sclérose en plaques sur la base d'un échantillon provenant d'un sujet connu comme ayant une sclérose en plaques, l'échantillon fournissant une source d'ARN, le procédé comprenant :

a) utiliser une amplification pour déterminer la mesure quantitative de la quantité d'ARN dans un panel de constituants comprenant au moins 2 constituants, où au moins l'un des constituants est HLADRA et un second constituant est choisi parmi ITGAL, CASP9, NFKBIB, STAT3, NFKB1, ITGAM, BCL2, CD4, IL1B, HSPA1A, ICAM1, IL18R1, CD14, IFI16, PI3, TGFBR2 ou CD8A, et,
b) arriver à une mesure de chaque constituant,

où l'ensemble des données de profil comprend la mesure de chaque constituant du panel et où l'amplification est réalisée dans des conditions de mesure qui peuvent sensiblement être répétées.

**3.** Procédé selon l'une des revendications 1 ou 2, dans lequel le procédé comprend la détermination d'une mesure quantitative d'au moins quatre constituants comprenant HLADRA, CASP9, ITGAL et CCR3, et comprend facultativement en outre la détermination d'une mesure quantitative d'un cinquième constituant TGFBR2.

**4.** Procédé de caractérisation de la sclérose en plaques selon l'une quelconque des revendications 1-3, dans lequel les constituants sont choisis de sorte qu'ils permettent la distinction entre un sujet normal et un sujet diagnostiqué pour MS en sevrage thérapeutique.

**5.** Procédé selon la revendication 4, dans lequel ledit sujet diagnostiqué pour MS est en sevrage thérapeutique depuis au moins trois mois.

**6.** Procédé selon l'une quelconque des revendications 1-3, dans lequel le sujet présente un signe présumé de la sclérose en plaques choisi dans le groupe consistant en un système sensoriel, moteur, visuel ou proprioceptif modifié avec au moins l'un parmi un engourdissement ou une faiblesse dans au moins un membre, ayant lieu souvent d'un côté du corps à la fois ou dans la moitié inférieure du corps, une perte partielle ou complète de la vision, fréquemment dans un oeil à un moment et souvent avec une douleur au cours du mouvement de l'oeil, une vision double ou une vision trouble, un picotement ou une douleur dans les régions engourdies du corps, des sensations d'électrochoc qui ont lieu avec certains mouvements de la tête, un tremblement, un défaut de coordination ou une démarche instable, la fatigue, le vertige, la raideur musculaire ou la spasticité musculaire, un trouble de l'élocution, une paralysie, des problèmes avec la fonction de la vessie, de l'intestin ou sexuelle, et des modifications mentales telles que l'oubli ou des difficultés de concentration, par rapport à des normes médicales.

**7.** Procédé selon l'une quelconque des revendications 1-3, dans lequel la sclérose en plaques provient d'un état auto-immun, d'un état environnemental, d'une infection virale, d'une infection bactérienne, d'une infection parasitaire eucaryote ou d'une infection fongique.

**8.** Procédé selon l'une quelconque des revendications 1-3, dans lequel l'échantillon est choisi dans le groupe consistant en le sang, une fraction sanguine, un liquide organique, une population de cellules et un tissu provenant du sujet.

**9.** Procédé selon la revendication 1, dans lequel l'évaluation comprend en outre :

la comparaison de l'ensemble de données de profil à un ensemble de données de profil de ligne de base pour le panel, l'ensemble de données de profil de ligne de base étant corrélé à la sclérose en plaques.

Fig. 1A

$((1/IL1A) + (1/IL1B) + (1/TNF) + (1/IFNG))/4 - 1/(IL10)$

Fig. 1B

EP 2 062 981 B1

Fig. 2

EP 2 062 981 B1

$$((1/IL1A) + (1/IL1B) + (1/TNF) + (1/IFNG))/4 - 1/(IL10)$$

Fig. 3

Fig. 4

Fig. 5

Bonfils (n=17) DCt Avg. v. Source Long. (n=16) DCt Avg. INF .48A

Fig. 6

EP 2 062 981 B1

Fig. 7

Gene Expression in Whole Blood for SC005 (unstable RA subjects);
AVG (n=4) and SC017 (Undiagnosed Normal); AVG (n=24)

Fig. 8

Target Genes from Inflammation Gene Expression Panel

Fig. 9

□ Blood Bank Avg. (n=17)
◇ Source Long. Avg. (n=16)

Inflammatory Genes

ΔCT Value

Fig. 10

Fig. 11

Fig. 12

Fig. 13

Fig. 14

■ Baseline gene expression
◻ 2 hr post 60 mg prednisone
◻ 24 post 60 mg prednisone

Fig. 15

Fig. 16

Fig. 17A

Fig. 17B

Fig. 17C

Fig. 18

Fig. 19

Fig. 20

EP 2 062 981 B1

Fig. 21

Patients

Source Inflammatory Index

Source Normal Range

81

Fig. 22

Fig. 23

Fig. 24

EP 2 062 981 B1

Fig. 25

85

Fig. 26A

Fig. 26B

Fig. 26C

Fig. 26D

Fig. 27

EP 2 062 981 B1

☑ LTA (S. pyogenes) 100 ng/mL, 1 hr
◪ LTA (S. pyogenes) 100 ng/mL, 2 hr
▤ LTA (S. pyogenes) 100 ng/mL, 4 hr
⊡ LTA (S. pyogenes) 100 ng/mL, 6 hr
☑ LTA (S. pyogenes) 100 ng/mL, 24 hr
◪ LTA (B. subtilis) 100 ng/mL, 1 hr
▤ LTA (B. subtilis) 100 ng/mL, 2 hr
▥ LTA (B. subtilis) 100 ng/mL, 4 hr
☐ LTA (B. subtilis) 100 ng/mL, 6 hr
⊡ LTA (B. subtilis) 100 ng/mL, 24 hr
▦ LTA (S. aureus) 100 ng/mL, 1 hr
▩ LTA (S. aureus) 100 ng/mL, 2 hr
▨ LTA (S. aureus) 100 ng/mL, 4 hr
☐ LTA (S. aureus) 100 ng/mL, 6 hr
▦ LTA (S. aureus) 100 ng/mL, 24 hr

Relative Gene Expression

10000
1000
100
10
1
0.1
0.01
0.001
0.0001

IFNG

Target Genes

Fig. 28

Fig. 29

Fig. 30

Fig. 31

Fig. 32

Fig. 33

Fig. 34

Fig. 35

Fig. 36

Fig. 37

Fig. 38

Fig. 39

EP 2 062 981 B1

Fig. 40

Fig. 41

| Gene or Index | RA mean dCt (n=23) | Normals mean dCt (n=69) | Relative Expression (fold change from normals) | t test P value |
|---|---|---|---|---|
| MMP9 | 14.48 | 16.24 | 3.37 | <.0001 |
| HSPA1A | 13.24 | 14.60 | 2.56 | <.0001 |
| IL1RN | 14.96 | 16.30 | 2.52 | <.0001 |
| TGFB1 | 12.10 | 13.41 | 2.49 | <.0001 |
| C1QA | 20.08 | 21.29 | 2.31 | <.0001 |
| CD14 | 13.58 | 14.77 | 2.27 | <.0001 |
| VEGF | 22.15 | 22.69 | 1.45 | 0.0002 |
| TNFSF6 | 19.93 | 20.62 | 1.61 | 0.0004 |
| CD3Z | 15.02 | 15.09 | 1.05 | 0.6181 |
| IL8 | 20.72 | 20.80 | 1.05 | 0.807 |
| PLA2G7 | 19.47 | 19.63 | 1.12 | 0.4295 |
| CD19 | 19.00 | 18.50 | 0.71 | 0.0232 |

## Fig. 42

Fig. 43

| Gene or Index | Bacteremia mean dCt (n=12) | Normals mean dCt (n=27-69) | Relative Expression (fold change from normals) | t test P value |
|---|---|---|---|---|
| MMP9 | 14.22 | 16.24 | 4.05 | <.0001 |
| HSPA1A | 13.02 | 14.60 | 2.99 | <.0001 |
| IFI16 | 14.53 | 16.00 | 2.75 | <.0001 |
| IL1R1 | 19.25 | 20.58 | 2.52 | <.0001 |
| SERPINA1 | 12.37 | 13.69 | 2.50 | <.0001 |
| ICAM1 | 17.31 | 17.93 | 1.53 | 0.0006 |
| TNFSF13B | 14.55 | 15.41 | 1.81 | 0.0002 |
| IL18BP | 17.37 | 17.44 | 1.05 | 0.6298 |
| CD19 | 18.44 | 18.50 | 1.04 | 0.8177 |
| TNFSF5 | 17.42 | 17.39 | 0.98 | 0.8871 |
| IFNA2 | 22.60 | 21.83 | 0.59 | 0.0019 |
| IL5 | 22.79 | 22.30 | 0.71 | 0.0438 |

Fig. 44

$((((1/(CXCL1) + 1/(IL18BP) + 1/IL1RN) + 1/(TGFB1))/4 - (1/(CD19) + 1/(IL8))/2) * 1.175)*1000$

Fig. 45

$((( 1/(IL8) + 1/(PLA2G7))/2 - (1/(IFNA2) + 1/(IFNG))/2) * 1.075*1000$

Fig. 46

Fig. 47

Fig. 48

Fig. 49

Fig. 50

Fig. 51

Fig. 52

Fig. 53

**4-Gene MS Discriminating Model -**
**ITGAL / CASP9 / HLADRA / TGFBR2**

MS Baseline Subjects (dataset $A_1A_2$, n=103) vs
Source MDx Normals (dataset $N_1$, n=100)

BASED ON ORIGINAL MODEL CONTAINING 103 MS AND 100 NORMALS

| PREDICTED PROBABILITY OF NORMAL vs MS PER 4 GENE MODEL - CASP9, ITGAL, TGFBR2 AND HLADRA | | | | |
|---|---|---|---|---|
| | TOTAL | NORMAL | MS | CLASSIFICATION PER MODEL |
| PROB > .55 - ASSIGNED TO NORMAL CLASSIFICATION | 96 | 86 | 10 | 89.6% |
| PROB BETWEEN .45 AND .55 - ASSIGNED 50% NORMAL AND 50% MS | 6 | 3 | 3 | 50.0% |
| PROB < .45 - ASSIGNED TO MS CLASSIFICATION | 101 | 11 | 90 | 89.1% |
| | 203 | 100 | 103 | |
| CORRECTED CLASSIFICATION OF MS SUBJECTS | | 88.8% | | |
| R-SQUARED | | 0.67 | | |

Fig. 54

**2-Gene MS Discriminating Model -
CASP9 / HLADRA**

MS Baseline Subjects (dataset $A_1A_2$, n=103) vs
Source MDx Normals (dataset $N_1$, n=100)

BASED ON ORIGINAL MODEL CONTAINING 103 MS AND 100 NORMALS

| PREDICTED PROBABILITY OF NORMAL vs MS PER 2 GENE MODEL - CASP9 AND HLADRA | | | | |
|---|---|---|---|---|
| | TOTAL | NORMAL | MS | CLASSIFICATION PER MODEL |
| PROB > .55 - ASSIGNED TO NORMAL CLASSIFICATION | 91 | 76 | 15 | 83.5% |
| PROB BETWEEN .45 AND .55 - ASSIGNED 50% NORMAL AND 50% MS | 12 | 8 | 4 | 66.7% |
| PROB < .45 - ASSIGNED TO MS CLASSIFICATION | 100 | 16 | 84 | 84.0% |
| | 203 | 100 | 103 | |

CORRECTED CLASSIFICATION OF MS SUBJECTS    83.5%

R-SQUARED    0.54

## Fig. 55

Fig. 56

**3-Gene MS Discriminating Model -
ITGAL / CASP9 / HLADRA**

MS Baseline Subjects (dataset $A_1A_2$, n=103) vs
Source MDx Normals (dataset $N_1$, n=100)

Group
× MS
o Normals

BASED ON ORIGINAL MODEL CONTAINING 103 MS AND 100 NORMALS

PREDICTED PROBABILITY OF NORMAL vs MS PER 3 GENE MODEL - CASP9, ITGAL AND HLADRA

|  | TOTAL | NORMAL | MS | CLASSIFICATION PER MODEL |
|---|---|---|---|---|
| PROB > .55 - ASSIGNED TO NORMAL CLASSIFICATION | 91 | 82 | 9 | 90.1% |
| PROB BETWEEN .45 AND .55 - ASSIGNED 50% NORMAL AND 50% MS | 11 | 6 | 5 | 54.5% |
| PROB < .45 - ASSIGNED TO MS CLASSIFICATION | 101 | 12 | 89 | 88.1% |
|  | 203 | 100 | 103 |  |

CORRECTED CLASSIFICATION OF MS SUBJECTS   88.8%
R-SQUARED   0.63

## Fig. 57

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03023056 A
- US 6692916 B
- WO 0125473 A
- WO 9824935 A
- US 5744305 A

### Non-patent literature cited in the description

- **HIRAYAMA et al.** *Blood,* 1998, vol. 92, 46-52
- RNA Isolation Strategies. RNA Methodologies, A laboratory guide for isolation and characterization. Academic Press, 1998, 55-104
- RNA Methodologies, A laboratory guide for isolation and characterization. Academic Press, 1998
- RNA isolation and characterization protocols, Methods in molecular biology. Human Press, 1998, vol. 86, 143-151
- Statistical refinement of primer design parameters. PCR applications: protocols for functional genomics. Academic Press, 1999, 55-72
- Nucleic acid detection methods. Molecular methods for virus detection. Academic Press, 1995, 1-24
- **Y.S. LIE ; C.J. PETROPOLUS.** *Current Opinion in Biotechnology,* 1998, vol. 9, 43-48
- PCR kinetics. PCR applications: protocols for functional genomics. Academic Press, 1999, 211-229
- **MCDONALD et al.** *Ann Neurol.,* July 2001, vol. 50 (1), 121-7